(19) Europäisches Patentamt / European Patent Office / Office européen des brevets

(11) EP 4 545 099 A1

(12) **EUROPEAN PATENT APPLICATION**
published in accordance with Art. 153(4) EPC

(43) Date of publication:
**30.04.2025 Bulletin 2025/18**

(21) Application number: **23830219.4**

(22) Date of filing: **27.06.2023**

(51) International Patent Classification (IPC):
***A61K 47/68*** *(2017.01)* ***A61K 47/65*** *(2017.01)*
***A61K 31/4745*** *(2006.01)* ***C07K 16/00*** *(2006.01)*
***A61P 35/00*** *(2006.01)*

(52) Cooperative Patent Classification (CPC):
**A61K 31/4745; A61K 47/65; A61K 47/68; A61P 35/00; C07K 16/00**

(86) International application number:
**PCT/CN2023/102590**

(87) International publication number:
**WO 2024/002042 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.06.2022 CN 202210740621**

(71) Applicant: **Bio-Thera Solutions, Ltd.**
**Guangzhou, Guangdong 510530 (CN)**

(72) Inventors:
- **MEI, Xingxing**
  **Guangzhou, Guangdong 510530 (CN)**
- **LI, Xiao**
  **Guangzhou, Guangdong 510530 (CN)**
- **SONG, Shuqiang**
  **Guangzhou, Guangdong 510530 (CN)**
- **TANG, Weijia**
  **Guangzhou, Guangdong 510530 (CN)**
- **YU, Jin-Chen**
  **Guangzhou, Guangdong 510530 (CN)**
- **LI, Shengfeng**
  **Guangzhou, Guangdong 510530 (CN)**

(74) Representative: **Engelhard, Markus**
**Boehmert & Boehmert Anwaltspartnerschaft mbB**
**Pettenkoferstrasse 22**
**80336 München (DE)**

Remarks:
The complete document including Reference Table(s) and the Sequence Listing(s) can be downloaded from the EPO website

(54) **METHOD FOR TREATING SOLID TUMOR**

(57) The present invention provides a method for treating a solid tumor, comprising administering to a patient with a solid tumor an effective amount of a TROP2-targeted antibody-drug conjugate.

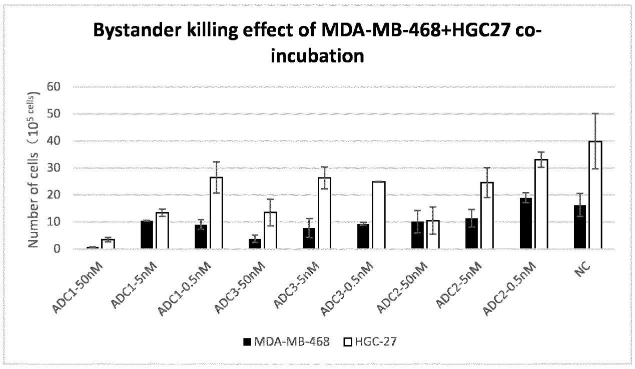


FIG. 1



Processed by Luminess, 75001 PARIS (FR)

## Description

## TECHNICAL FIELD

The present invention relates to the field of pharmaceutical therapeutic methods, specifically to a method for treating solid tumors with antibody drug conjugate.

## BACKGROUND

The targeted treatment of cancer, immunodeficiency, infectious diseases, etc. is currently the main focus of precision medicine. Over the years, there have been numerous reports of using cell surface receptor binding molecules as drug delivery vehicles to form conjugates with cytotoxin molecules for targeted delivery of cytotoxin molecules to attack various pathogenic cells (Allen, T.M. and Cullis, P.R., 2004 Science, 303(5665), 1818-22; Hu, Q.Y., et al. (2016), Chem Soc Rev 45(6): 1691-1719).

An antibody-drug conjugate consists of three parts: an antibody, a cytotoxin molecule, and a linker in between for linking the two (Thomas, A., et al. (2016), Lancet Oncol 17(6): e254-e262). The three components each serve unique functions: the antibody should be able to specifically bind to tumor cells, the cytotoxin molecule should be sufficiently active and have a broad spectrum for the tumor cells, and the linker should be uniquely functional, stable in the blood circulation and effective in releasing the cytotoxin molecule upon reaching the tumor cells (Chari, R.V. (2008), Acc Chem Res 41(1): 98-107). Good clinical results can be produced only when the three components are reasonably constructed (Singh, S.K., et al. (2015), Pharm Res 32(11): 3541-3571; Hamilton, G.S. (2015), Biologicals 43(5): 318-332).

TROP2 (Trophoblast cell surface antigen 2), also known as EGP-1, M1S1, or GA733-1, is a member of the TACSTD family together with the epithelial cell adhesion molecule (EpCAM) and can regulate downstream signal transduction through phosphorylation. TROP2 can directly regulate the expression of PARP1 enzyme and impair the DNA repair function of normal cells. It also induces EMT, leading to loss of polarity in cancer cells and reducing the adhesion between adjacent epithelia, which ultimately leads to the metastasis and spread of cancer cells. TROP2 is overexpressed in cells of various cancers, such as gastric cancer, colon cancer, papillary thyroid carcinoma, urothelial carcinoma, prostate cancer, lung adenocarcinoma, lung squamous cell carcinoma, pancreatic cancer, breast cancer, oral squamous cell carcinoma, ovarian epithelial cancer, cervical cancer, etc.

## SUMMARY

The present invention provides a method for treating a solid tumor comprising administering to a patient with the solid tumor an effective amount of an antibody-drug conjugate; use of an antibody-drug conjugate in preparing a pharmaceutical composition for treating a solid tumor; or use of an antibody-drug conjugate in treating a solid tumor. The effective amount of the antibody-drug conjugate is administered about once every 1-8 weeks at about 0.1-30 mg/kg per dose, and the antibody-drug conjugate has a structure of Formula I, or a stereoisomer thereof, or a pharmaceutically acceptable salt or solvate thereof:

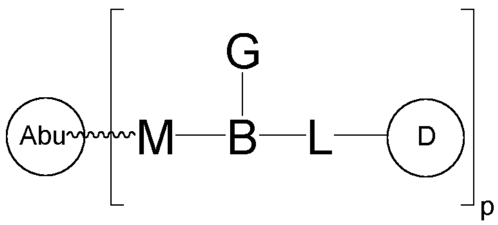

Formula I

wherein,

Abu is an antibody or an antigen-binding unit thereof that binds to TROP2;

D is drug,

M is

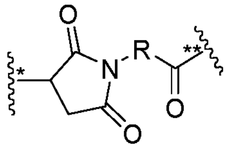

,

wherein * links to Abu, ** links to B, and R is selected from: $-(CH_2)_r-$, $-(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8 carbocyclyl)-, -(C3-C8 carbocyclyl)-$(CH_2)_r-$, C3-C8 heterocyclyl, $-(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10;

B is

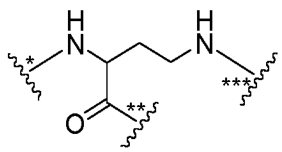

,

or is

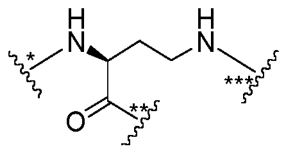

;

wherein * links to M, ** links to L, and *** links to G;

L is-$(AA)_i-(FF)_f-$, wherein AA is an amino acid or polypeptide, and i is 1, 2, 3, 4, 5, 6, 7, 8, 9, 10, 11, 12, 13, 14, 15, 16, 17, 18, 19 or 20;

each FF is independently

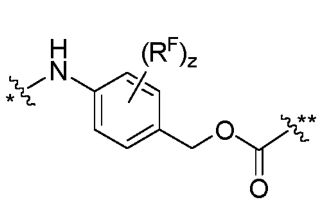

,

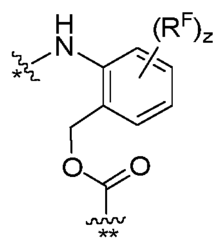

or

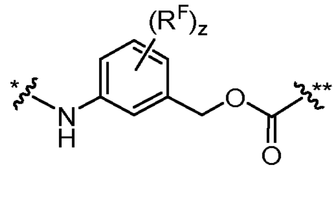

,

wherein each $R^F$ is independently C1-C6 alkyl, C1-C6 alkoxy, $-NO_2$ or halogen; z is 0, 1, 2, 3 or 4; wherein * links to AA, and ** links to D;

G is

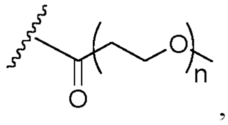

wherein n is an integer from 1-24;

p is 1-10.

In one or more embodiments, the drug is an anti-cancer drug, a cytotoxic drug, a cell differentiation factor, a stem cell trophic factor, a steroid drug, a drug for treating autoimmune diseases, an antiinflammatory drug or a drug for treating infectious diseases.

In one or more embodiments, the drug is a tubulin inhibitor, a DNA damaging agent, or a DNA topoisomerase inhibitor.

In one or more embodiments, the tubulin inhibitor is selected from dolastatin, auristatins and maytansinoids (maytansine).

In one or more embodiments, the drug is an auristatin, selected from monomethyl auristatin E (MMAE), monomethyl auristatin F (MMAF), or auristatin F (AF).

In one or more embodiments, the drug is a DNA damaging agent, e.g., a calicheamicin, a duocarmycin, or the anthramycin derivative PBD (pyrrolobenzodiazepine).

In one or more embodiments, the drug is a DNA topoisomerase inhibitor or a salt thereof, e.g., irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, the drug is

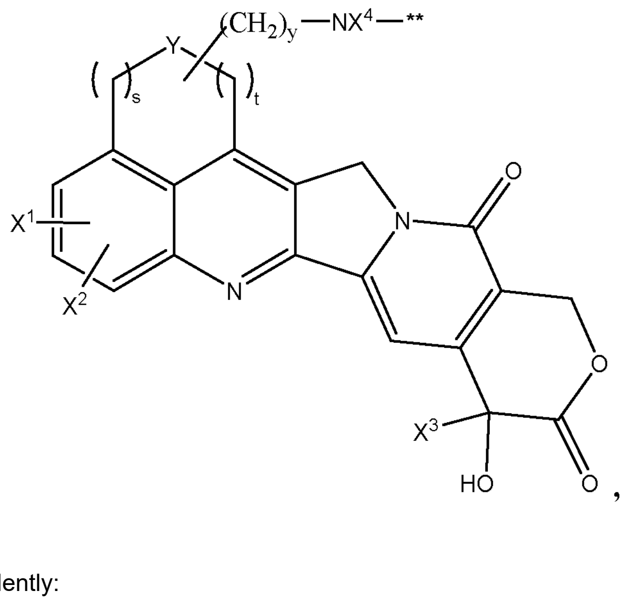

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl,

carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-aryle ne-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, $-(CH_2)_q$ $C(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)$ $NR^n(CH_2CH_2O)_q-CH_2-CH_3$ or $-(CH_2CH_2O)_qC(O)NR^n(CH_2)_q-CH_3$; wherein each $R^n$ is independently H , C1-C6 alkyl, C3-C8 carbocyclyl, phenyl or benzyl, and each q is independently 1, 2, 3, 4, 5, 6, 7, 8, 9 or 10;

** links to L;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, t-butoxycarbonyl, benzyl, or*p*-methoxybenzyloxycarbonyl.

In one or more embodiments, the drug is

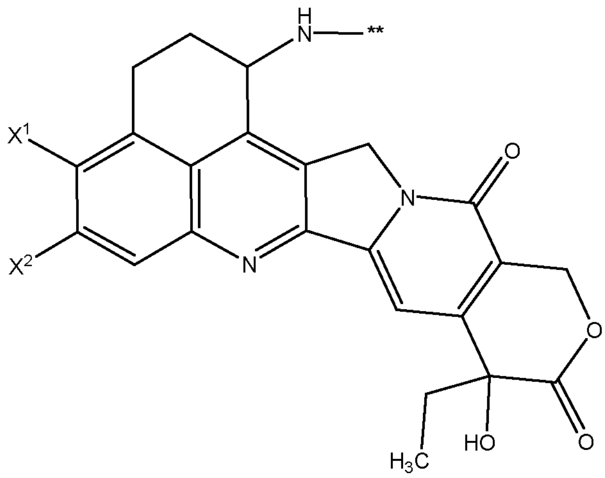

,

wherein $X^1$ and $X^2$ are each independently C1-C6 alkyl, halogen, or -OH; ** links to L.

In one or more embodiments, the drug is

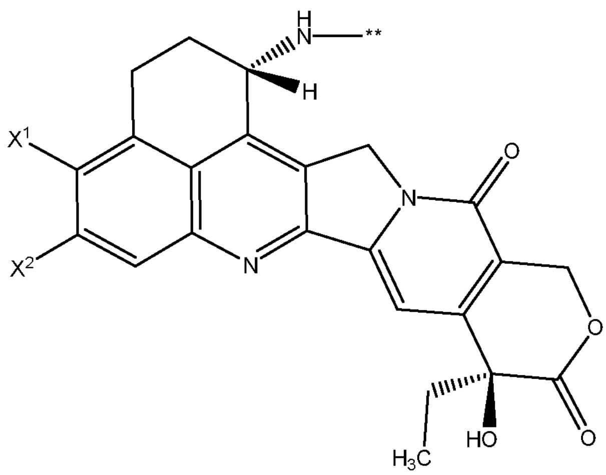

,

wherein $X^1$ and $X^2$ are each independently C1-C6 alkyl, halogen, or -OH; ** links to L.

In one or more embodiments, $X^1$ and $X^2$ are each $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently $-CH_3$, F, or -OH.

In one or more embodiments, $X^1$ and $X^2$ are each independently F or $-CH_3$.

In one or more embodiments, $X^1$ is $-CH_3$ and $X^2$ is F.

In one or more embodiments, R is -(CH2)r-.

In one or more embodiments, R is $-(CH_2)_r-$, wherein r is 1 or 5.

In one or more embodiments, R is $-(CH_2)-$.

In one or more embodiments, each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, i is 1.

In one or more embodiments, AA is Val-Cit, i is 1.

In one or more embodiments, each FF is independently

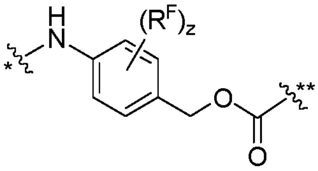 , 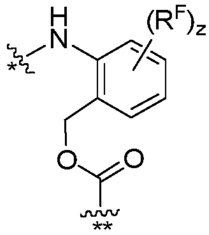

or

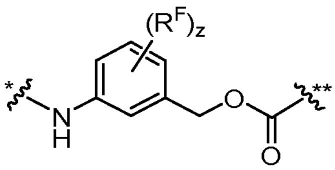 ,

wherein each $R^F$ is independently C1-C6 alkyl, C1-C6 alkoxy, $-NO_2$, or halogen, wherein * links to AA, and ** links to D.

In one or more embodiments, the halogen is F.

In one or more embodiments, each $R^F$ is independently $-CH_3$, F, $-NO_2$ or $-OCH_3$.

In one or more embodiments, z is 0.

In one or more embodiments, z is 1 or 2.

In one or more embodiments, f is 1.

In one or more embodiments, each FF is independently

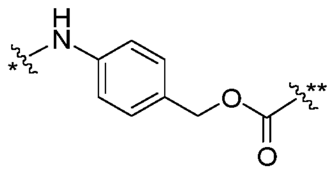 ,

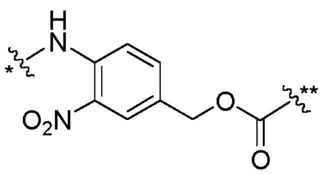 , 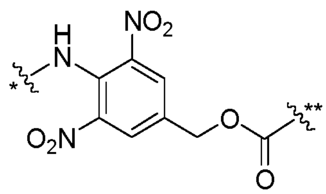 , 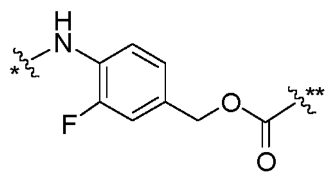 ,

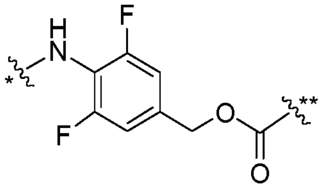 , 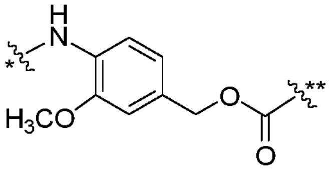 , 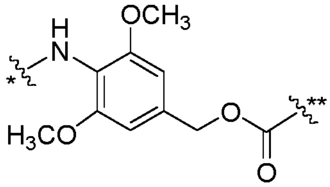 ,

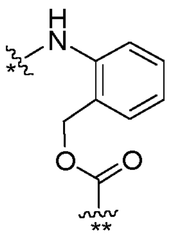, 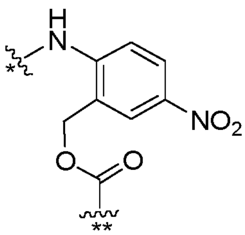, 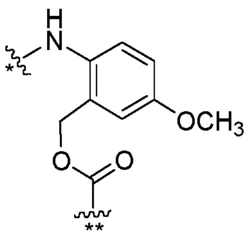, 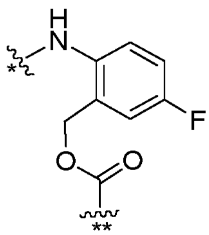, 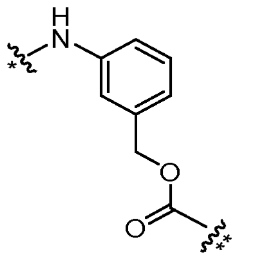,

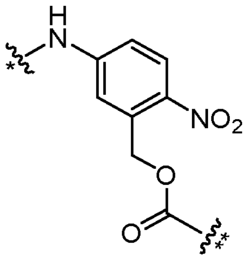, 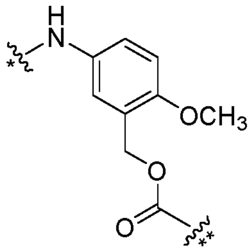 or 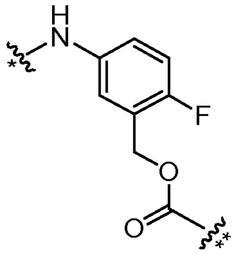;

wherein * links to AA, and ** links to D.

In one or more embodiments, f is 1.

In one or more embodiments, FF is

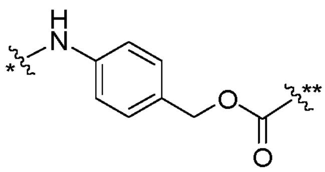,

and f is 1, wherein * links to AA, and ** links to D.

In one or more embodiments, L is

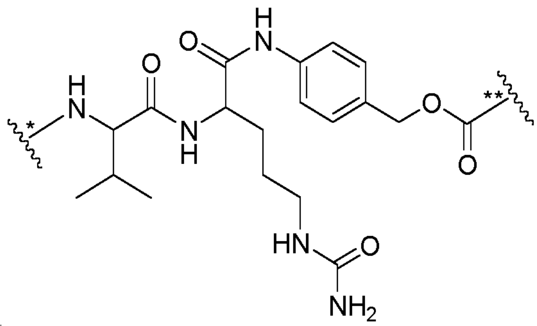,

wherein * links to B, and ** links to D

In one or more embodiments, L is

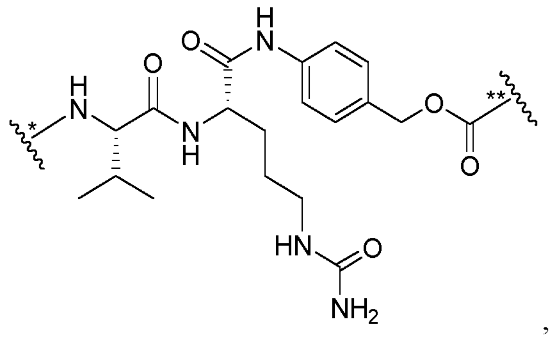

wherein * links to B, and ** links to D.

In one or more embodiments, G is

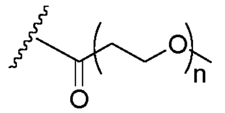

and n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 4-5.

In one or more embodiments, p is 6-8.

In one or more embodiments, p is 7-8.

In one or more embodiments, the Abu is an hRS9 antibody.

In one or more embodiments, the antibody-drug conjugate has a structure shown as Formula I-1, I-2, I-3, I-4, I-5, I-6, I-7, I-8, I-9, I-10, or I-11, or a stereoisomer thereof, or a pharmaceutically acceptable salt or solvate thereof:

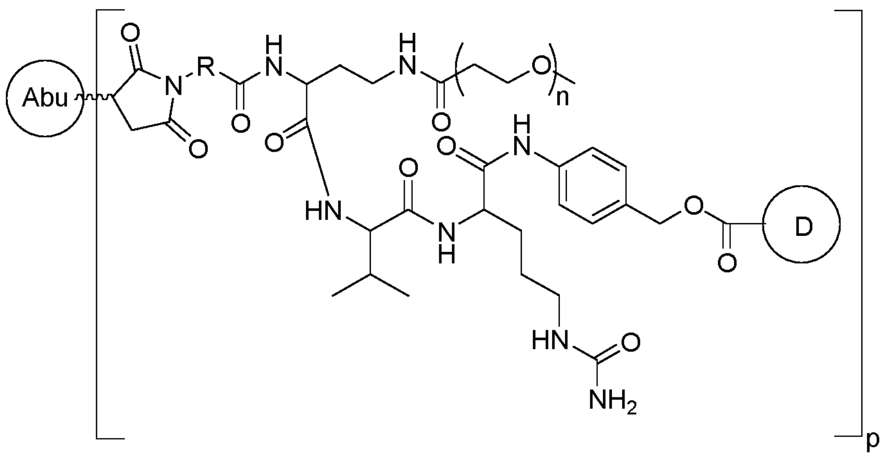

Formula I-1

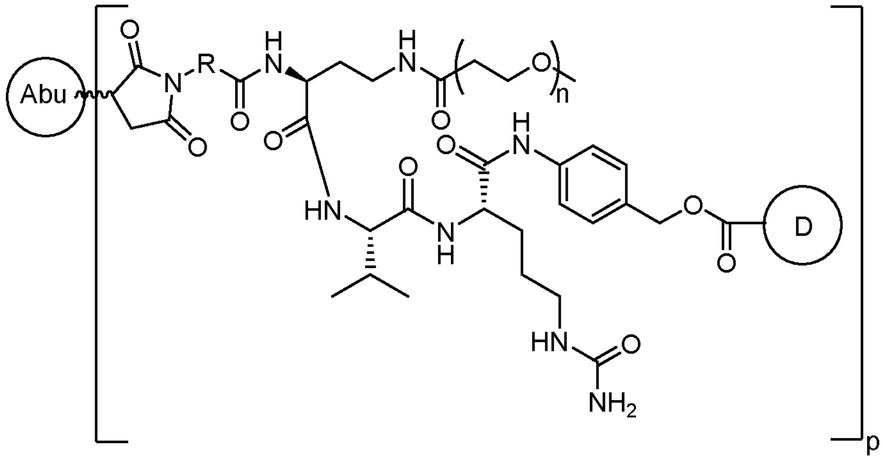

Formula I-2

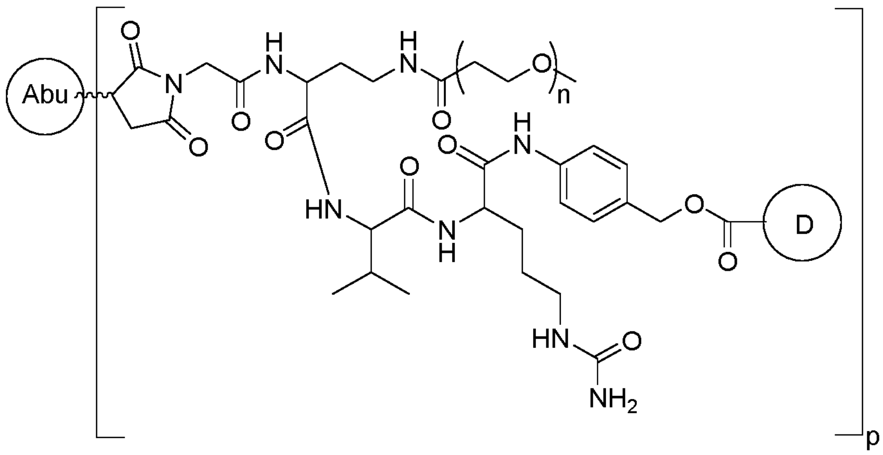

Formula I-3

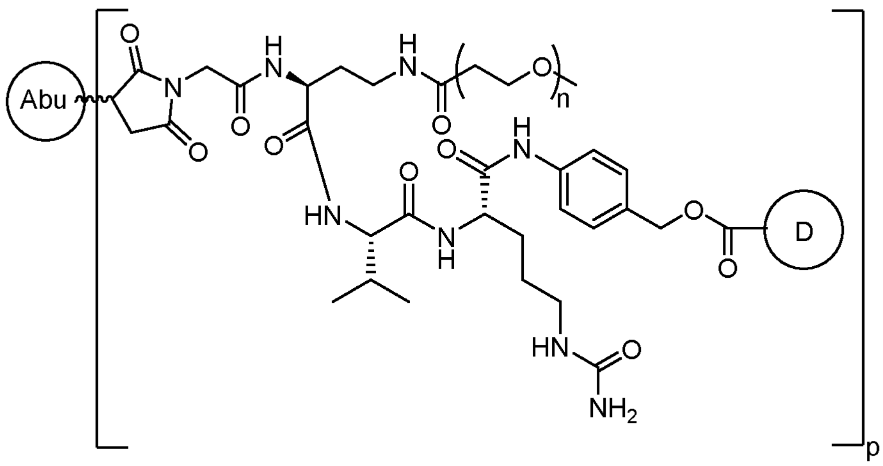

Formula I-4

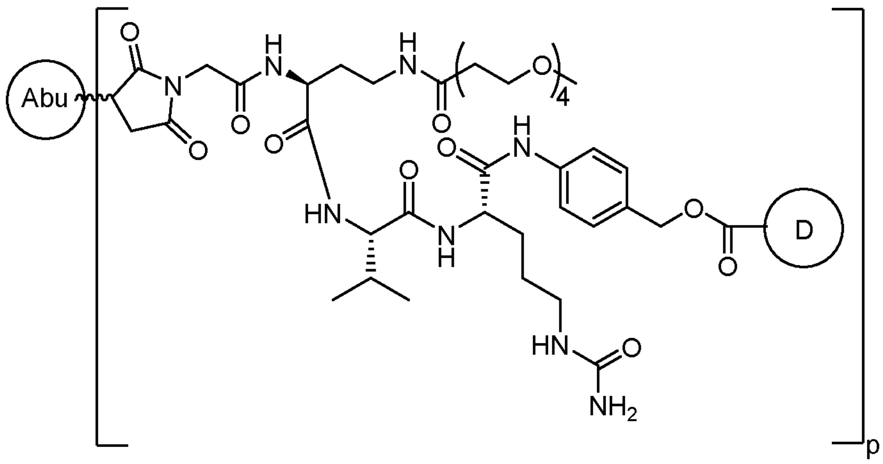

Formula I-5

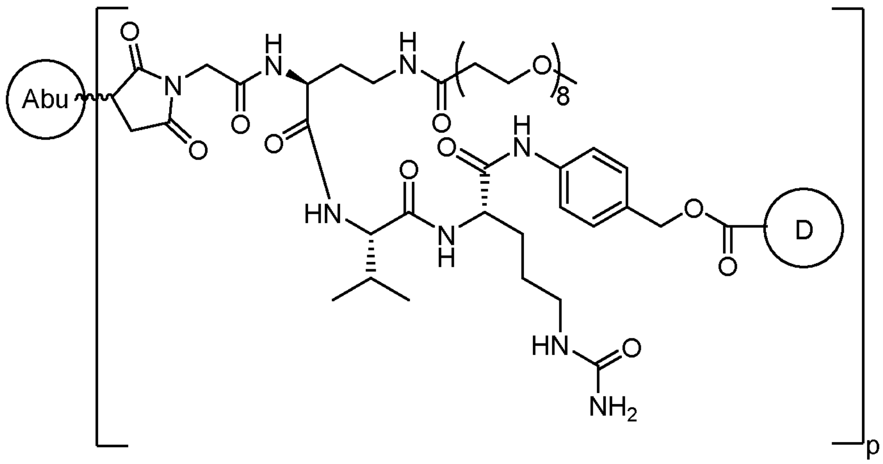

Formula I-6

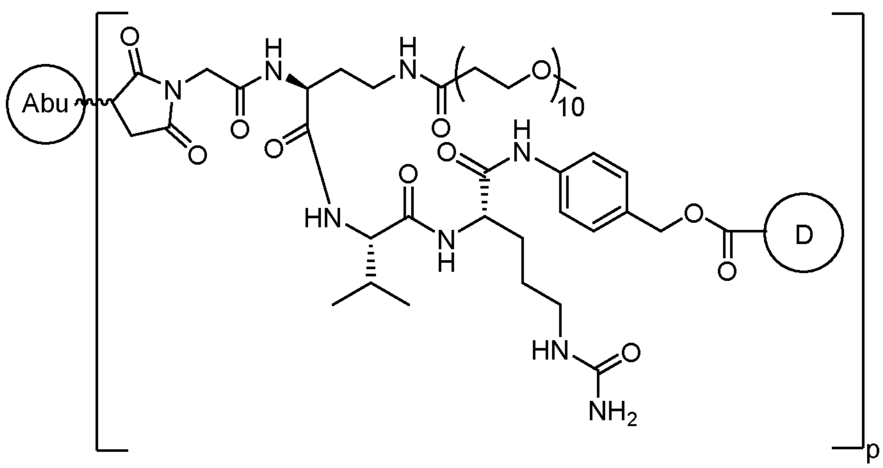

Formula I-7

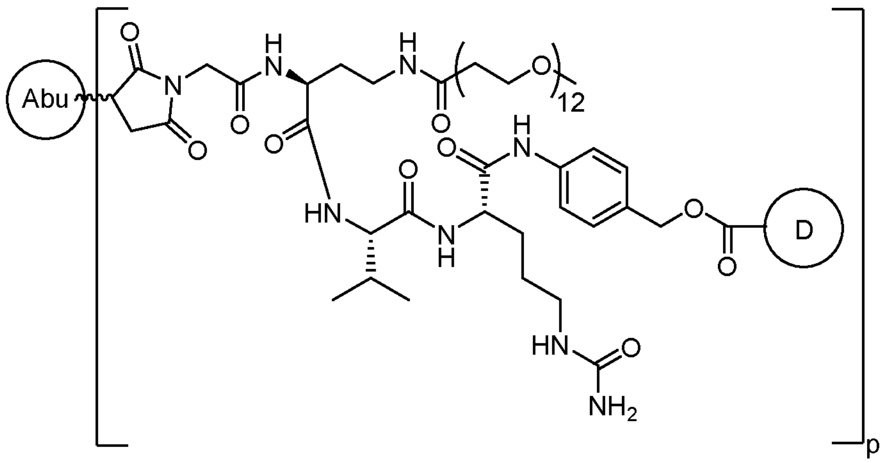

Formula I-8

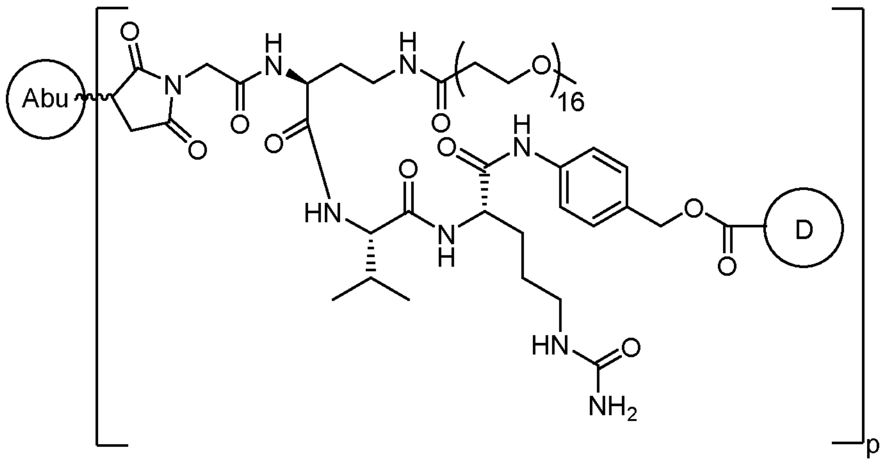

Formula I-9

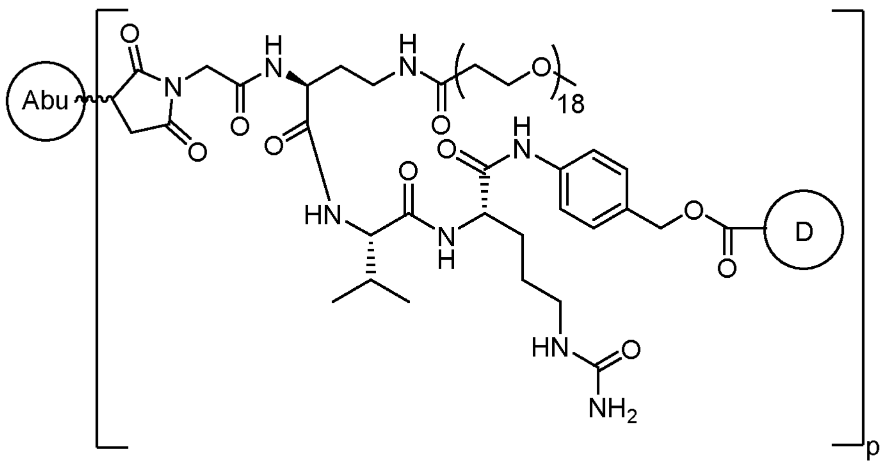

Formula I-10

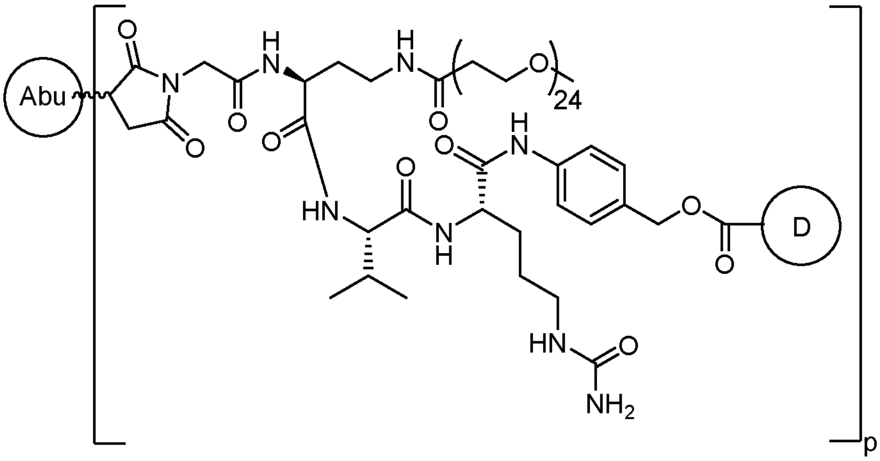

Formula I-11

wherein

Abu is an antibody or an antigen-binding unit thereof that binds to TROP2;

R is selected from: $-(CH_2)_r-$, $-(CHR^m)r-$, C3-C8 carbocyclyl, $-O-(CH_2)_r-$, arylene, $-(CH_2)_r$-arylene-, - arylene-$(CH_2)r-$, $-(CH_2)_r$-(C3-C8 carbocyclyl)-, -(C3-C8 carbocyclyl)-$(CH_2)_r-$, C3-C8 heterocyclyl, - $(CH_2)_r$-(C3-C8 heterocyclyl)-, -(C3-C8 heterocyclyl)-$(CH_2)_r-$, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$ and - $(CH_2CH_2O)_rC(O)NR^m(CH_2)_r-$; wherein each $R^m$ is independently H, C1-C6 alkyl, C3-C8 carbocyclyl, phenyl or benzyl, and each r is independently 1, 2, 3, 4, 5, 6, 7, 8, 9 or 10; or R is $-(CH_2)_r-$, or r is 1 or 5;

D is a drug;

n is an integer from 1-24; or n is 4-12;

p is 1-10, e.g., 1, 2, 3, 4, 5, 6, 7, 8, 9 or 10.

In one or more embodiments, the drug is an anti-cancer drug, a cytotoxic drug, a cell differentiation factor, a stem cell trophic factor, a steroid drug, a drug for treating autoimmune diseases, an antiinflammatory drug or a drug for treating infectious diseases.

In one or more embodiments, the drug is a tubulin inhibitor, a DNA damaging agent, or a DNA topoisomerase inhibitor.

In one or more embodiments, the tubulin inhibitor is selected from dolastatin, auristatins and maytansinoids.

In one or more embodiments, the drug is an auristatin, e.g., MMAE, MMAF, or AF.

In one or more embodiments, the drug is a DNA damaging agent, e.g., a calicheamicin, a duocarmycin, the anthramycin derivative PBD (pyrrolobenzodiazepine).

In one or more embodiments, the drug is a DNA topoisomerase inhibitor or a salt thereof, e.g., irinotecan, irinotecan hydrochloride, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, or N-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan, or a chlorambucil derivative.

In one or more embodiments, the drug is

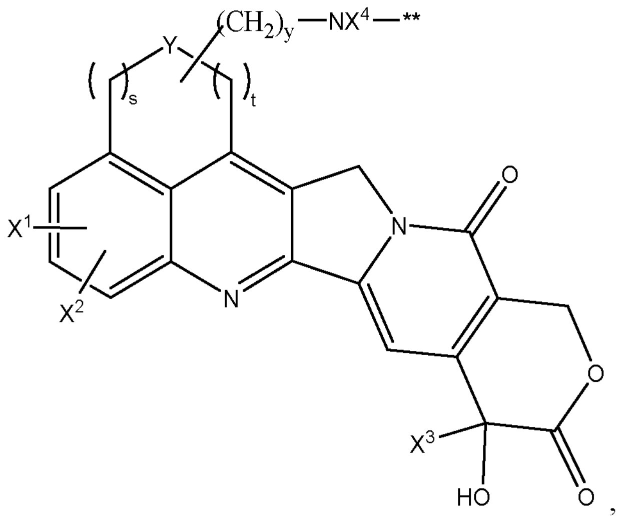

wherein
$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,

C1-C6 alkoxy,

C1-C6 aminoalkoxy,

halogen,

nitro,

cyano,

thiol,

alkylthio,

amino, amino substituted with an amino-protecting group, C1-C6 aminoalkyl optionally substituted at the amino moiety with an amino-protecting group or C1-C6 alkyl,

C1-C6 aminoalkylamino optionally substituted at the amino moiety with an amino-protecting group or C1-C6 alkyl,

C1-C6 alkyl linking to a heterocyclic ring, wherein the heterocyclic ring is optionally substituted with one or more C1-C6 alkyl, C1-C6 alkoxy, amino, halogen, nitro or cyano groups,

C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,

amino-substituted heterocyclyl, which is optionally substituted at a nitrogen atom of the heterocyclyl moiety or at the amino moiety with a protecting group or one or more C1-C6 alkyl groups,

heterocyclylamino, which is optionally substituted at a nitrogen atom of the heterocyclic moiety or at the amino moiety with a protecting group or C1-C6 alkyl,

carbamoyl optionally substituted with a carbamoyl-protecting group or C1-C6 alkyl,

morpholin-1-yl, or

piperidin-1-yl;

$X^3$ is C1-C6 alkyl;

$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10;

** is point of connection;

y is 0, 1 or 2;

Y is O, S or $CR^1R^2$, wherein $R^1$ and $R^2$ are each independently H or C1-C6 alkyl;

s and t are each independently 0, 1 or 2, but not both 0.

In one or more embodiments, $X^4$ is H or C1-C6 alkyl.
In one or more embodiments, the heterocyclyl is azetidine, niverazine, morpholine, pyrrolidine, piperidine, imidazole, thiazole, oxazole or pyridine.
In one or more embodiments, the amino-protecting group is formyl, acetyl, trityl, t-butoxycarbonyl, benzyl, or*p*-methoxybenzyloxycarbonyl.
In one or more embodiments, the drug is

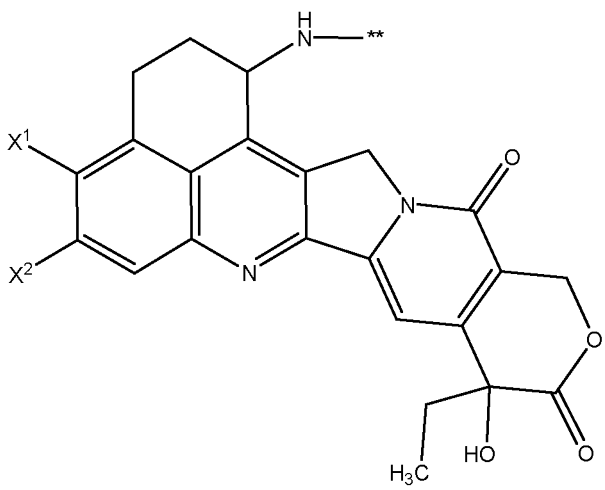

,

wherein $X^1$ and $X^2$ are each independently C1-C6 alkyl, halogen, or -OH; ** is point of connection.
In one or more embodiments, the drug is

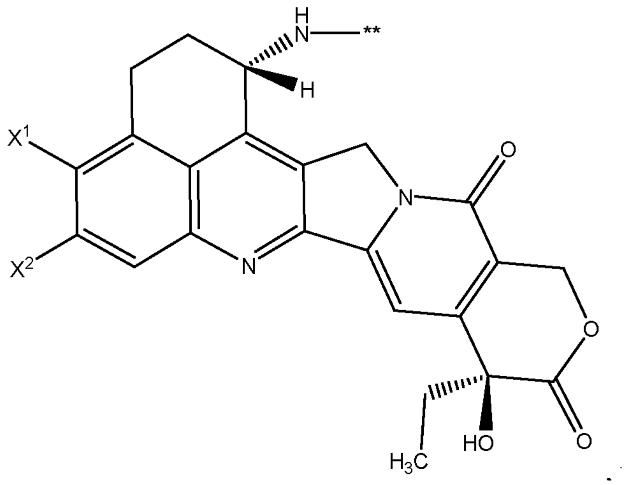

wherein $X^1$ and $X^2$ are each independently C1-C6 alkyl, halogen, or -OH; ** is point of connection.

In one or more embodiments, C1-C6 alkyl is $-CH_3$

In one or more embodiments, halogen is F.

In one or more embodiments, $X^1$ and $X^2$ are each independently $-CH_3$, F or -OH.

In one or more embodiments, $X^1$ and $X^2$ are each independently $-CH_3$.

In one or more embodiments, $X^1$ and $X^2$ are each independently F, Cl, Br, or I.

In one or more embodiments, $X^1$ and $X^2$ are each F.

In one or more embodiments, $X^1$ and $X^2$ are each independently F or $-CH_3$.

In one or more embodiments, $X^1$ is $-CH_3$ and $X^2$ is F.

In one or more embodiments, R is -(CH2)r-.

In one or more embodiments, R is $-(CH_2)_r-$, or r is 1 or 5.

In one or more embodiments, R is $-(CH_2)-$.

In one or more embodiments, n is 4-12.

In one or more embodiments, n is 4-8.

In one or more embodiments, n is 4.

In one or more embodiments, n is 8.

In one or more embodiments, p is 2-8.

In one or more embodiments, p is 4-8.

In one or more embodiments, p is 4-5.

In one or more embodiments, p is 6-8.

In one or more embodiments, p is 7-8.

In one or more embodiments, the antibody-drug conjugate has a structure shown as Formula I-12, I-13, I-14, I-15, I-16, I-17, I-18, I-19, I-20, I-21, I-22, I-23, I-24, or I-25, or a stereoisomer thereof, or a pharmaceutically acceptable salt or solvate thereof:

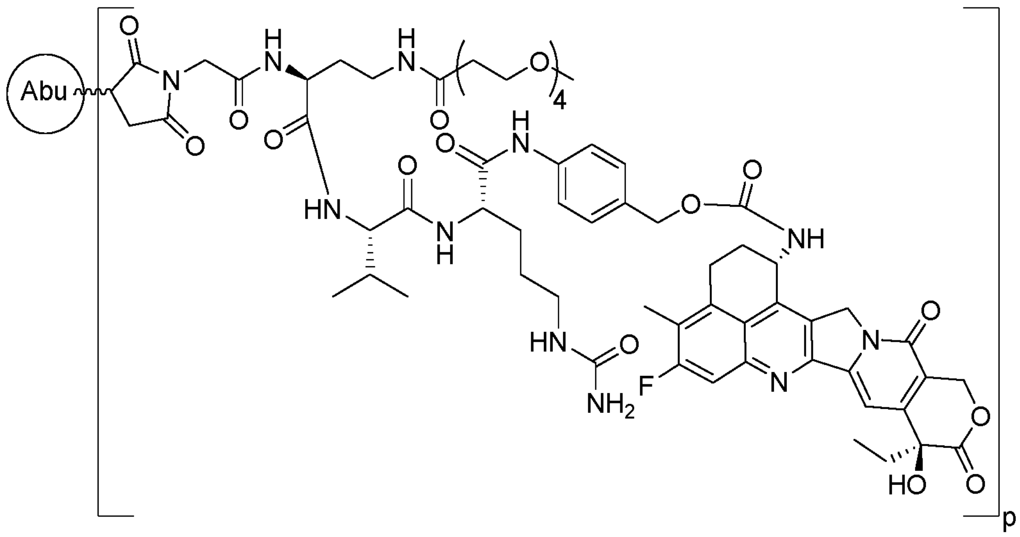

Formula I-12

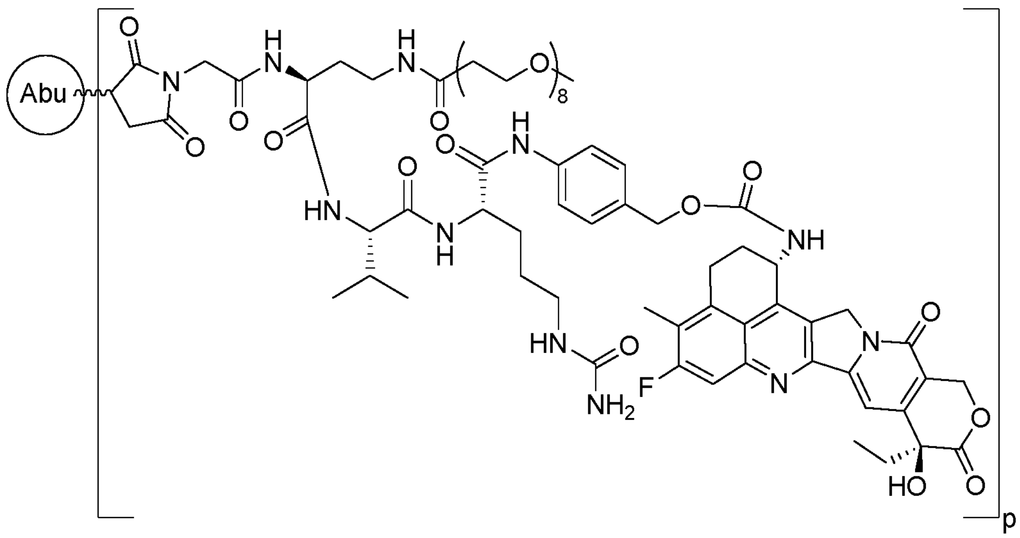

Formula I-13

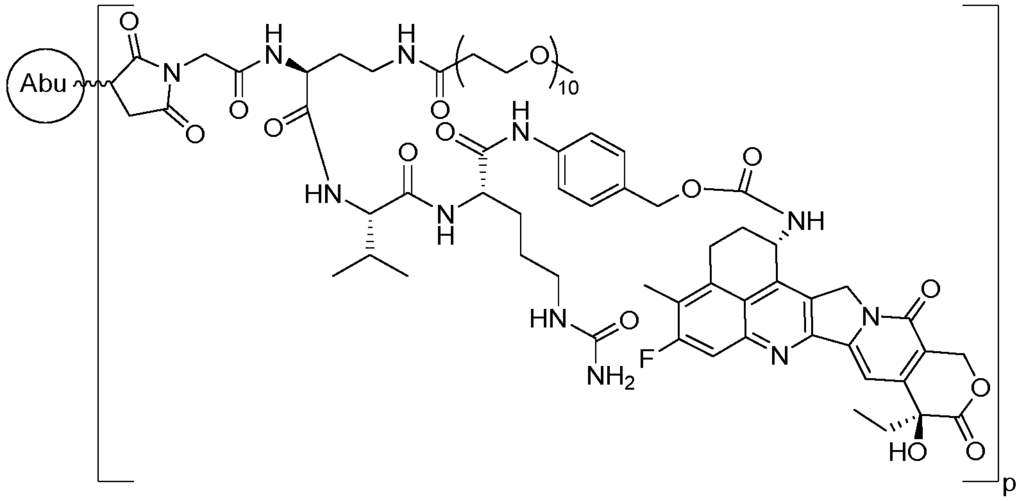

Formula I-14

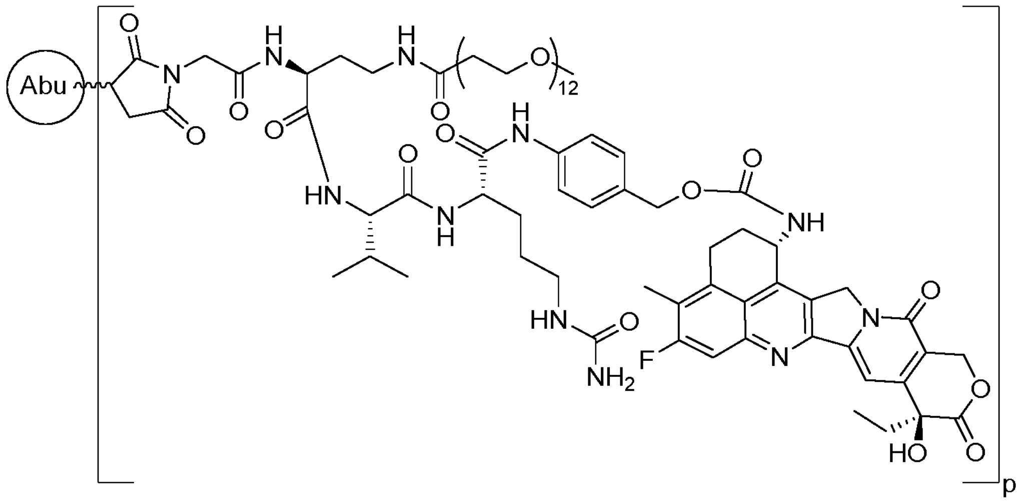

Formula I-15

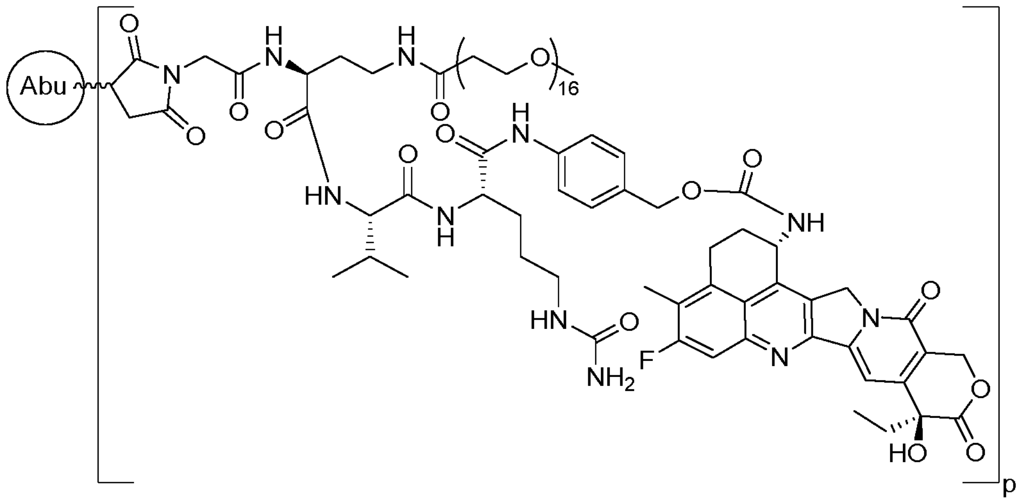

Formula I-16

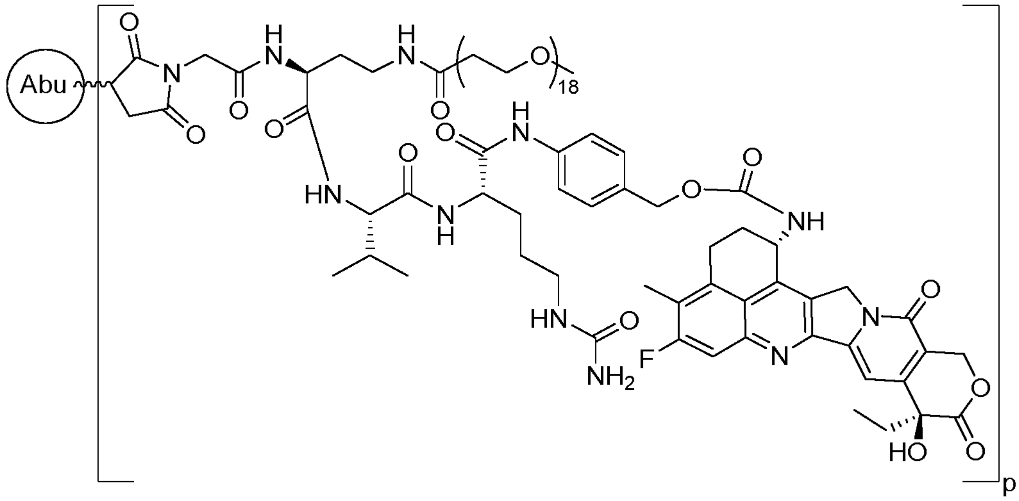

Formula I-17

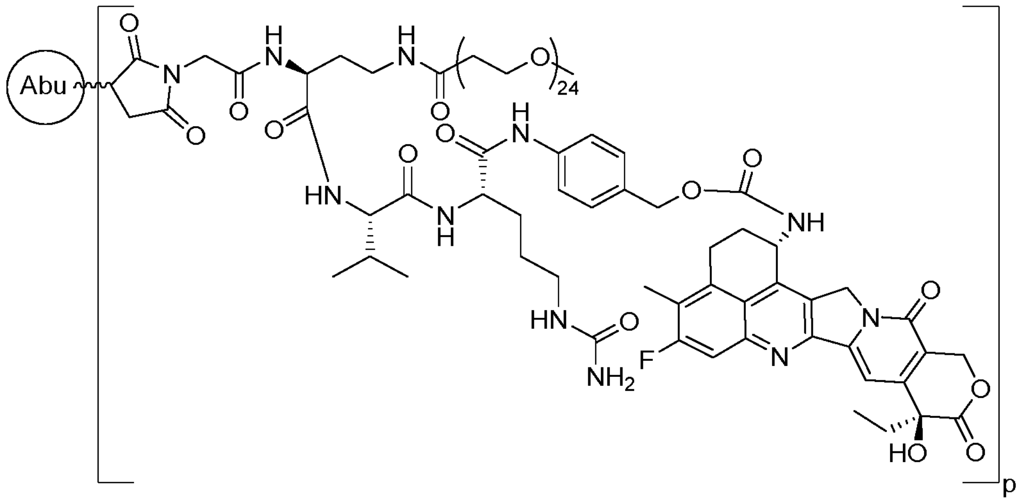

Formula I-18

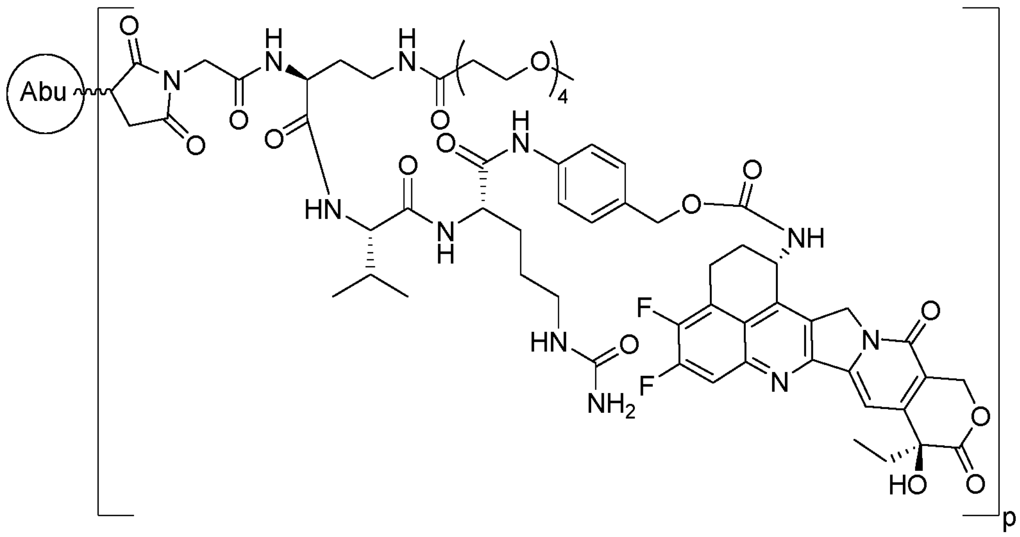

Formula I-19

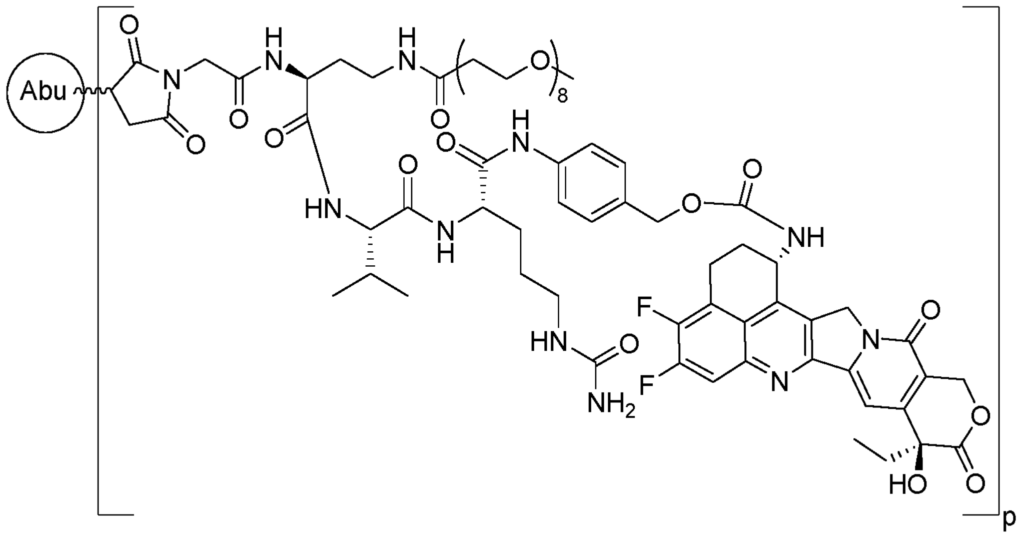

Formula I-20

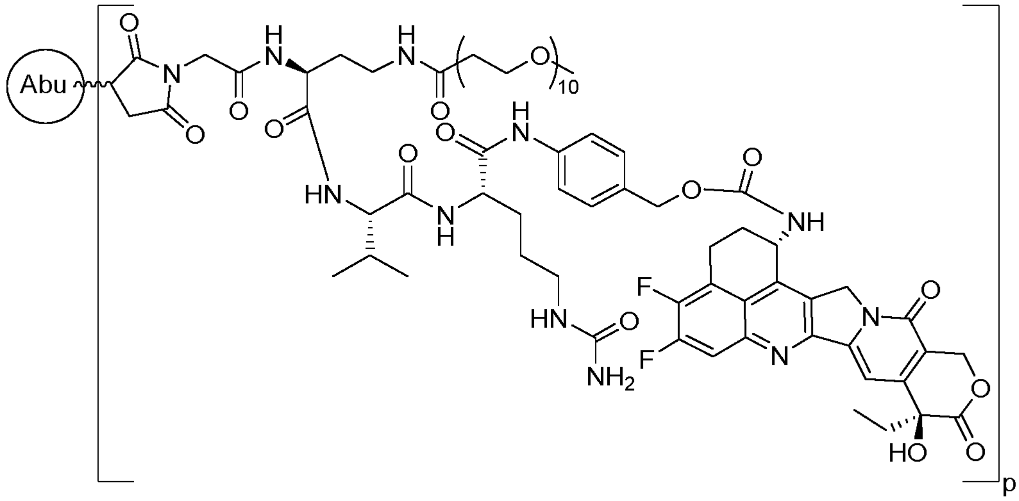

Formula I-21

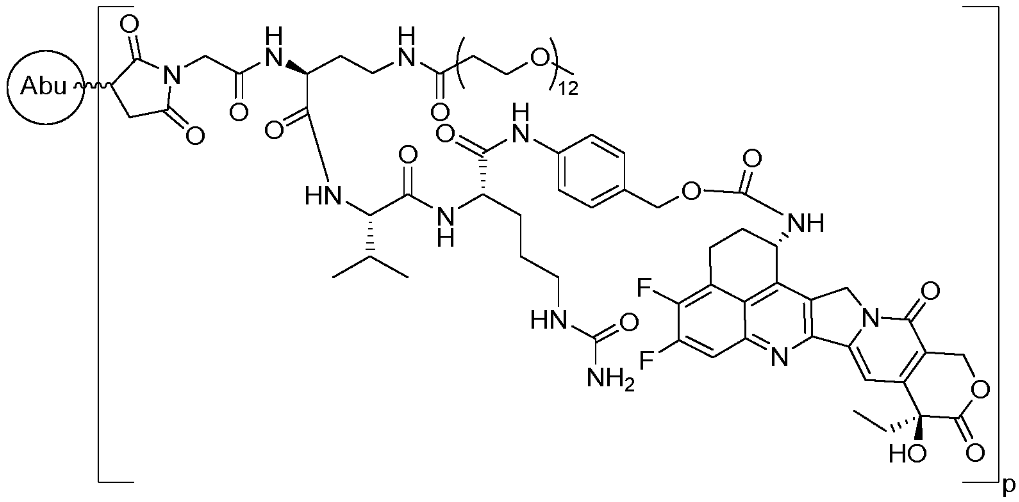

Formula I-22

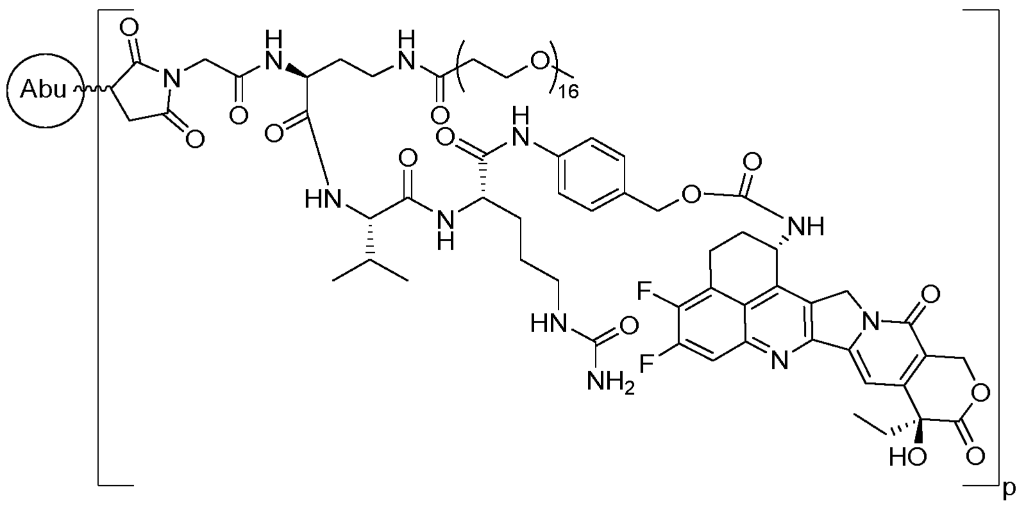

Formula I-23

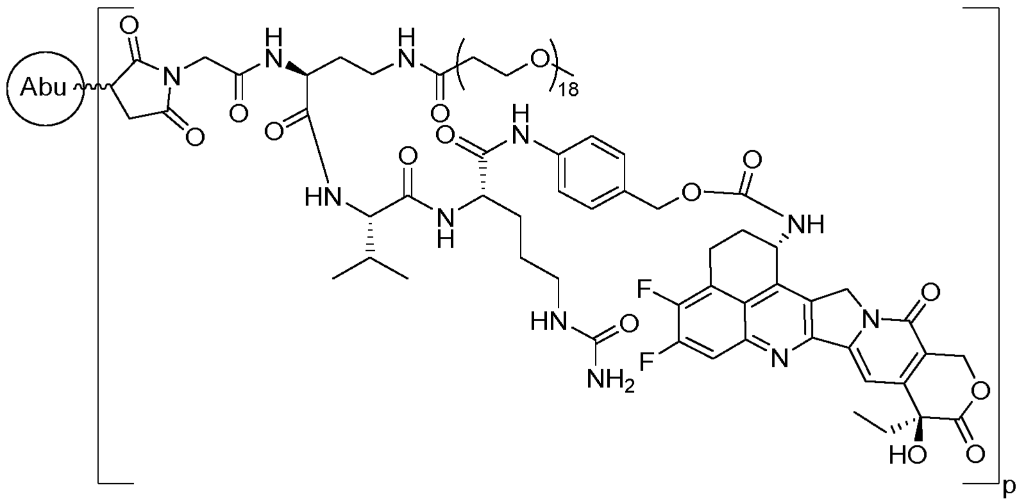

Formula I-24

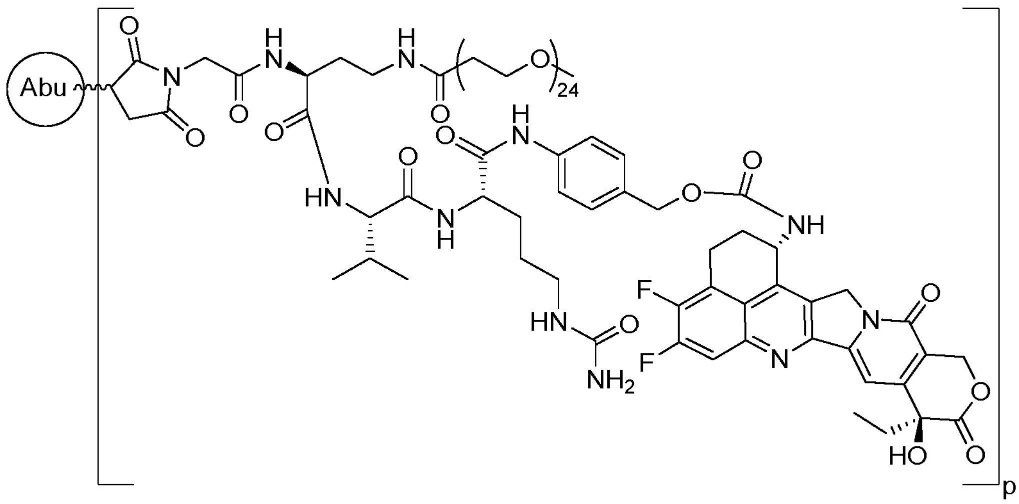

Formula I-25

wherein

Abu is an antibody or an antigen-binding fragment that binds to TROP2(trophoblast cell surface antigen 2);

p is 1-10, e.g., 1, 2, 3, 4, 5, 6, 7, 8, 9 or 10.

In one or more embodiments, p is 2-8.

In one or more embodiments, p is 4-8.

In one or more embodiments, p is 4-5.

In one or more embodiments, p is 6-8.

In one or more embodiments, p is 7-8.

In one or more embodiments, Abu is an anti-Trop2 antibody.

In one or more embodiments, the anti-Trop2 antibody is capable of specifically acting on Trop2 (trophoblast cell surface antigen 2) protein.

In one or more embodiments, the anti-Trop2 antibody is a fully human monoclonal antibody.

In one or more embodiments, the anti-Trop2 antibody is a humanized monoclonal antibody.

In one or more embodiments, the anti-Trop2 antibody is an antibody disclosed in the following patent documents: WO2021147993A1 (e.g., PD3), CN101264325B (e.g., RS7, hRS7), CN105849126B (e.g., hTINA1-H1L1, hTINA1-H2L1, hTINA1-H2L2, hTINA1-H3L3), CN110903395A (e.g., M1, M2, M3), CN113896796A (e.g., 4D3, 7F11), US20130089872

(e.g., K5-70, K5-107, K5-116-2-1, T6-16, T5-86), US5840854A (e.g., BR110), US20130122020 (e.g., 3E9, 6G11, 7E6, 15E2, 18B1), US20120237518 (e.g., 77220, KM4097, KM4590).

In one or more embodiments, the anti-Trop2 antibody is commercially available, including LS-C126418, LS-C178765, LS-C126416, LS-C126417 (LifeSpan BioSciences, Inc., Seattle, WA); 10428-MM01, 10428-MM02, 10428-R001, 10428-R030 (Sino Biological Inc., Beijing, China); MR54 (eBioscience, San Diego, CA); sc-376181, sc-376746, Santa Cruz Biotechnology (Santa Cruz, CA); MM0588-49D6 (Novus Biologicals, Littleton, CO); ab79976 and ab89928 (Cambridge, MA).

In one or more embodiments, the anti-Trop2 is anti-Trop-2 antibodies 162-25.3 and 162-46.2 disclosed by Lipinski et al. (1981, Proc Natl. Acad Sci USA, 78:5147-50) or Pr1E11 anti-Trop-2 antibody disclosed by Ikeda et al. (2015, Biochem Biophys Res Comm 458:877-82), which recognizes a unique epitope on Trop-2.

In one or more embodiments, the anti-Trop2 antibody is an hRS9 antibody, comprising heavy and light chain sequences set forth in SEQ ID NO: 1 and SEQ ID NO: 2, respectively.

The amino acid sequences corresponding to the sequence numbers are shown in Table 1.

**Table1**

| | |
|---|---|
| SEQ ID NO: 1 | QVQLQQSGSELKKPGASVKVSCKASGYTFTNYGMNWVKQAPGQGLKWMGWIN TYTGEPTYTDDFKGRFAFSLDTSVSTAYLQISSLKADDTAVYFCARGGFGSSYWY FDVWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCK VSNKALPAPIEKTISKAKGQPREPQVYTLPPSRDELTKNQVSLTCLVKGFYPSDIA VEWESNGQPENNYKTTPPVLDSDGSFFLYSKLTVDKSRWQQGNVFSCSVMHEAL HNHYTQKSLSLSPGK |
| SEQ ID NO: 2 | DIQLTQSPSSLSASVGDRVSITCKASQDVSIAVAWYQQKPGKAPKLLIYSASYRYT GVPDRFSGSGSGTDFTLTISSLQPEDFAVYYCQQHYITPLTFGAGTKVEIKRTVAA PSVFIFPPSDEQLKSGTASVVCLLNNFYPREAKVQWKVDNALQSGNSQESVTEQD SKDSTYSLSSTLTLSKADYEKHKVYACEVTHQGLSSPVTKSFNRGEC |

In one or more embodiments, Abu is an antibody binding to TROP2 or an antigen-binding unit thereof, comprising a heavy chain set forth in SEQ ID NO: 1 and a light chain set forth in SEQ ID NO: 2.

In one or more embodiments, the antibody-drug conjugate (ADC) is ADC1 or a pharmaceutically acceptable salt or solvate thereof

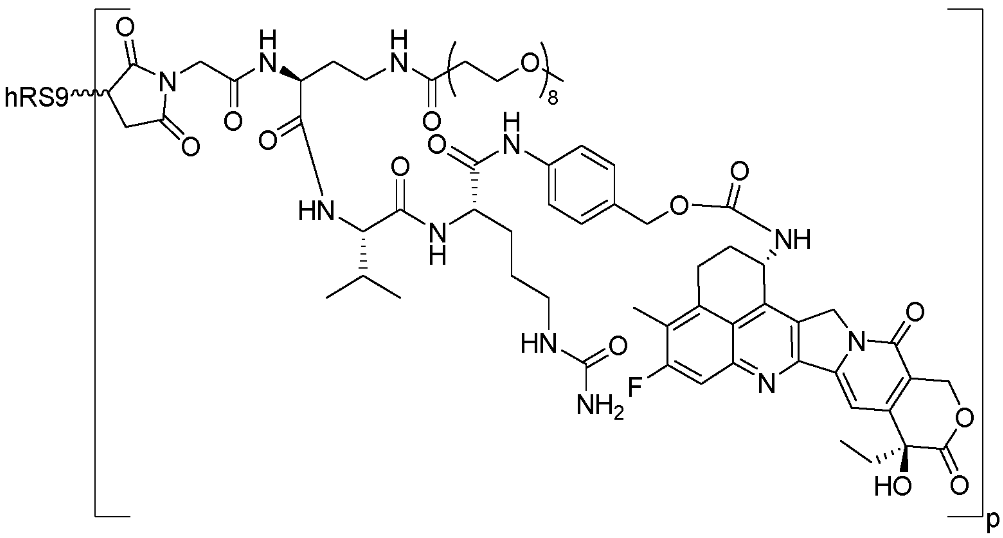

ADC1

wherein, p denotes the average drug-to-antibody ratio and is about 3 to about 6. In one or more embodiments, p is about 4

to about 5. In one or more embodiments, the ADC is ADC2 or a pharmaceutically acceptable salt or solvate thereof:

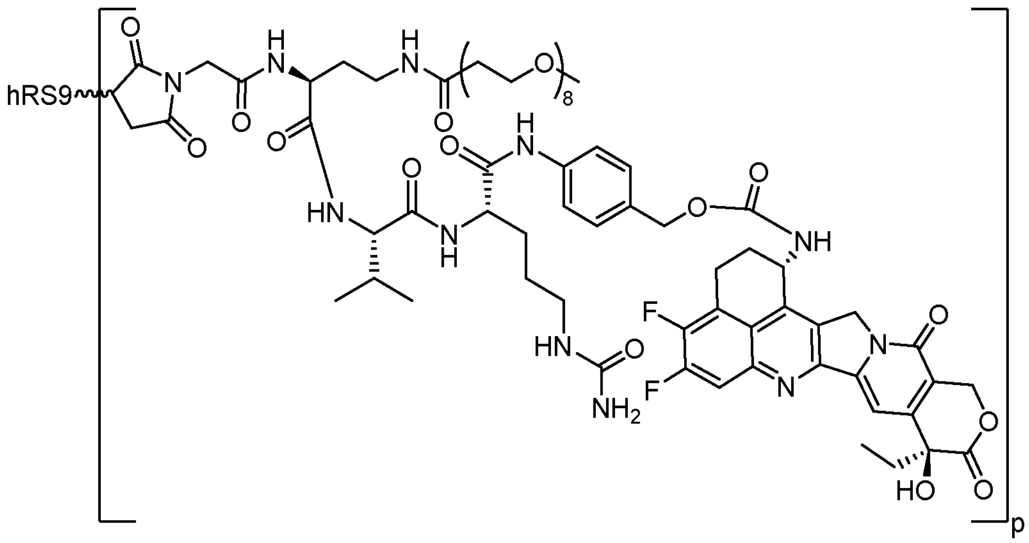

ADC2

wherein, p denotes the average drug-to-antibody ratio and is about 3 to about 6. **In** one or more embodiments, p is about 4 to about 5.

The present invention provides a pharmaceutical composition comprising an effective amount of the antibody-drug conjugate described herein and a pharmaceutically acceptable carrier.

The antibody-drug conjugate or the pharmaceutical composition described herein is used for preparing a medicament for treating a solid tumor, and the medicament comprises an effective amount of the antibody-drug conjugate described herein.

Examples of the solid tumor include, but are not limited to, urothelial carcinoma, triple-negative breast cancer, ovarian cancer, uterine cancer, endometrial cancer, fallopian tube cancer, breast cancer, cervical cancer, gastric cancer, carcinoma of colon and rectum, squamous cell cancer (e.g., epithelial squamous cell cancer), lung cancer (including small cell lung cancer, non-small cell lung cancer, lung adenocarcinoma, and lung squamous cell carcinoma), peritoneal cancer, hepatocellular carcinoma, gastric cancer (including gastrointestinal cancer), pancreatic cancer, glioblastoma, cervical cancer, liver cancer, bladder cancer, urinary tract cancer, hepatoma, breast cancer, colon cancer, rectal cancer, salivary gland cancer, renal cancer, prostate cancer, vulval cancer, thyroid cancer, anal cancer, penile cancer, glioblastoma, medulloblastoma, head and neck cancer, T-cell lymphoma, melanoma, liver cancer, Kaposi's sarcoma, hepatobiliary cancer, carcinoma of colon and rectum, and colorectal cancer.

In one or more embodiments, the solid tumor is urothelial carcinoma, triple-negative breast cancer, gastric adenocarcinoma, esophageal cancer, hepatocellular carcinoma, non-small cell lung cancer, small cell lung cancer, epithelial ovarian cancer, cervical cancer, follicular thyroid carcinoma, glioblastoma multiforme, endometrial cancer, prostate cancer, head and neck squamous cell carcinoma, or clear cell renal cell carcinoma.

In one or more embodiments, the solid tumor is triple-negative breast cancer, non-small cell lung cancer, urothelial carcinoma, small cell lung cancer, or cervical cancer.

In one or more embodiments, the solid tumor is a TROP2-positive solid tumor.

In one or more embodiments, the solid tumor is an advanced TROP2-positive solid tumor.

In one or more embodiments, the antibody-drug conjugate is administered at about 0.1-10 mg/kg per dose. In one or more embodiments, the antibody-drug conjugate is administered at about 0.1 mg/kg, about 0.8 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per dose.

In one or more embodiments, the antibody-drug conjugate is administered at about 5-3000 mg per dose. In one or more embodiments, the antibody-drug conjugate is administered at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700 mg per dose. In one or more embodiments, the antibody-drug conjugate is administered at about 5 mg, about 50 mg, about 100 mg, about 150 mg, about 200 mg, about 250 mg, about 300 mg, about 400 mg, about 500 mg, about 600 mg, about 700 mg, about 1000 mg, about 1500 mg, about 2000 mg, about 2500 mg, or about 3000 mg, or a range between any two of these values (inclusive) or any value therein per dose.

In one or more embodiments, the effective amount of the antibody-drug conjugate is administered once every 1, 2, 3, 4, 5, 6, or 7 weeks at about 0.8 mg/kg to about 3.6 mg/kg per dose.

In one or more embodiments, the effective amount of the antibody-drug conjugate is administered once every 1, 2, 3, 4, 5, 6, or 7 weeks at about 2.4 mg/kg to about 3.6 mg/kg per dose.

In one or more embodiments, the effective amount of the antibody-drug conjugate is administered once every 1, 2, 3, 4, 5, 6, or 7 weeks at about 2.7 mg/kg to about 3.3 mg/kg per dose.

In one or more embodiments, the effective amount of the antibody-drug conjugate is administered once every 1, 2, 3, 4, 5, 6, or 7 weeks at about 0.8 mg/kg, about 1.2 mg/kg, about 2.4 mg/kg, about 2.7 mg/kg, about 3.0 mg/kg, about 3.3 mg/kg, or about 3.6 mg/kg per dose.

In one or more embodiments, the effective amount of the antibody-drug conjugate is administered once every 1, 2, 3, 4, 5, 6, or 7 weeks at about 2.4 mg/kg, about 2.7 mg/kg, about 3.0 mg/kg, about 3.3 mg/kg, or about 3.6 mg/kg per dose.

In one or more embodiments, the effective amount of the antibody-drug conjugate is administered once every 1, 2, 3, 4, 5, 6, or 7 weeks at about 3.0 mg/kg per dose.

In one or more embodiments, one treatment cycle is at least 1 week, at least 2 weeks, at least 3 weeks, at least 4 weeks, at least 5 weeks, at least 6 weeks, or at least 7 weeks. In one or more embodiments, one treatment cycle is 1 week, 2 weeks, 3 weeks, 4 weeks, 5 weeks, 6 weeks, or 7 weeks, or a range between any two of these values (inclusive) or any value therein. In one or more embodiments, the antibody-drug conjugate is administered about once every week, or about once every 2 weeks, every 3 weeks, every 4 weeks, every 5 weeks, every 6 weeks, or every 7 weeks. In one or more embodiments, the antibody-drug conjugate is administered about once every 2 weeks.

In one or more embodiments, the effective amount of the antibody-drug conjugate is administered once every 2, 3, or 4 weeks at about 2.4 mg/kg to about 3.6 mg/kg per dose.

In one or more embodiments, the effective amount of the antibody-drug conjugate is administered once every 2 weeks.

In one or more embodiments, the effective amount of the antibody-drug conjugate is administered once every 3 weeks.

In one or more embodiments, the effective amount of the antibody-drug conjugate is administered once every 2 weeks at about 2.4 mg/kg to about 3.6 mg/kg per dose.

In one or more embodiments, the effective amount of the antibody-drug conjugate is administered once every 3 weeks at about 2.4 mg/kg to about 3.6 mg/kg per dose.

In one or more embodiments, the effective amount of the antibody-drug conjugate is administered once every 2 weeks at about 2.7 mg/kg to about 3.3 mg/kg per dose.

In one or more embodiments, the effective amount of the antibody-drug conjugate is administered once every 3 weeks at about 2.7 mg/kg to about 3.3 mg/kg per dose.

In one or more embodiments, the effective amount of the antibody-drug conjugate is administered once every 2 weeks at about 2.7 mg/kg per dose.

In one or more embodiments, the effective amount of the antibody-drug conjugate is administered once every 3 weeks at about 2.7 mg/kg per dose.

In one or more embodiments, the effective amount of the antibody-drug conjugate is administered once every 2 weeks at about 3 mg/kg per dose.

In one or more embodiments, the effective amount of the antibody-drug conjugate is administered once every 3 weeks at about 3 mg/kg per dose.

In one or more embodiments, the effective amount of the antibody-drug conjugate is administered once every 2 weeks at about 3.3 mg/kg per dose.

In one or more embodiments, the effective amount of the antibody-drug conjugate is administered once every 3 weeks at about 3.3 mg/kg per dose.

In one or more embodiments, the effective amount of the antibody-drug conjugate is administered once every 2 weeks at about 3.6 mg/kg per dose.

In one or more embodiments, the effective amount of the antibody-drug conjugate is administered once every 3 weeks at about 3.6 mg/kg per dose.

In one or more embodiments, the antibody-drug conjugate is administered about once every week at about 0.1-30 mg/kg per dose. In one or more embodiments, the antibody-drug conjugate is administered about once every week at about 0.1-10 mg/kg per dose. In one or more embodiments, the antibody-drug conjugate is administered about once every week at about 0.1 mg/kg, about 0.8 mg/kg, about 1 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per dose. In one or more embodiments, the antibody-drug conjugate is administered about once every week at about 5-3000 mg per dose. In one or more embodiments, the antibody-drug conjugate is administered about once every week at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700

mg per dose. In one or more embodiments, the antibody-drug conjugate is administered about once every week at about 5 mg, about 50 mg, about 100 mg, about 150 mg, about 200 mg, about 250 mg, about 300 mg, about 400 mg, about 500 mg, about 600 mg, about 700 mg, about 1000 mg, about 1500 mg, or about 2000 mg, or a range between any two of these values (inclusive) or any value therein per dose.

In one or more embodiments, the antibody-drug conjugate is administered about once every 2 weeks at about 0.1-30 mg/kg per dose. In one or more embodiments, the antibody-drug conjugate is administered about once every 2 weeks at about 0.1-10 mg/kg per dose. In one or more embodiments, the antibody-drug conjugate is administered about once every 2 weeks at about 0.5-4 mg/kg per dose. In one or more embodiments, the antibody-drug conjugate is administered about once every 2 weeks at about 0.1 mg/kg, about 0.5 mg/kg, about 0.8 mg/kg, about 1 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per dose. In one or more embodiments, the antibody-drug conjugate is administered about once every 2 weeks at about 0.8 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, or about 7.2 mg/kg per dose. In one or more embodiments, the antibody-drug conjugate is administered about once every 2 weeks at about 5-3000 mg per dose. In one or more embodiments, the antibody-drug conjugate is administered about once every 2 weeks at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700 mg per dose. In one or more embodiments, the antibody-drug conjugate is administered about once every 2 weeks at about 5 mg, about 50 mg, about 100 mg, about 150 mg, about 200 mg, about 250 mg, about 300 mg, about 400 mg, about 500 mg, about 600 mg, about 700 mg, about 1000 mg, about 1500 mg, about 2000 mg, about 2500 mg, or about 3000 mg, or a range between any two of these values (inclusive) or any value therein per dose.

In one or more embodiments, the antibody-drug conjugate is administered about once every 3 weeks at about 0.1-30 mg/kg per dose. In one or more embodiments, the antibody-drug conjugate is administered about once every 3 weeks at about 0.1-10 mg/kg per dose. In one or more embodiments, the antibody-drug conjugate is administered about once every 3 weeks at about 0.5-4 mg/kg per dose. In one or more embodiments, the antibody-drug conjugate is administered about once every 3 weeks at about 0.1 mg/kg, about 0.5 mg/kg, about 0.8 mg/kg, about 1 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per dose. In one or more embodiments, the antibody-drug conjugate is administered about once every 3 weeks at about 0.8 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, or about 7.2 mg/kg per dose. In one or more embodiments, the antibody-drug conjugate is administered about once every 3 weeks at about 5-3000 mg per dose. In one or more embodiments, the antibody-drug conjugate is administered about once every 3 weeks at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700 mg per dose. In one or more embodiments, the antibody-drug conjugate is administered about once every 3 weeks at about 5 mg, about 50 mg, about 100 mg, about 150 mg, about 200 mg, about 250 mg, about 300 mg, about 400 mg, about 500 mg, about 600 mg, about 700 mg, about 1000 mg, about 1500 mg, about 2000 mg, about 2500 mg, or about 3000 mg, or a range between any two of these values (inclusive) or any value therein per dose.

In one or more embodiments, the antibody-drug conjugate is administered about once every 4 weeks at about 0.1-30 mg/kg per dose. In one or more embodiments, the antibody-drug conjugate is administered about once every 4 weeks at about 0.1-10 mg/kg per dose. In one or more embodiments, the antibody-drug conjugate is administered about once every 4 weeks at about 0.1 mg/kg, about 0.5 mg/kg, about 0.8 mg/kg, about 1 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per dose. In one or more embodiments, the antibody-drug conjugate is administered about once every 4 weeks at about 5-3000 mg per dose. In one or more embodiments, the antibody-drug conjugate is administered about once every 4 weeks at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700 mg per dose. In one or more embodiments, the antibody-drug conjugate is administered about once every 4 weeks at about 5 mg, about 50 mg, about 100 mg, about 150 mg, about 200 mg, about 250 mg, about 300 mg, about 400 mg, about 500 mg, about 600 mg, about 700 mg, about 1000 mg, about 1500 mg, about 2000 mg, about 2500 mg, or about 3000 mg, or a range between any two of these values (inclusive) or any value therein per dose.

In one or more embodiments, the antibody-drug conjugate is administered about once every 5 weeks at about 0.1-30 mg/kg per dose. In one or more embodiments, the antibody-drug conjugate is administered about once every 5 weeks at about 0.1-10 mg/kg per dose. In one or more embodiments, the antibody-drug conjugate is administered about once every 5 weeks at about 0.1 mg/kg, about 0.5 mg/kg, about 0.8 mg/kg, about 1 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per

dose. In one or more embodiments, the antibody-drug conjugate is administered about once every 5 weeks at about 5-3000 mg per dose. In one or more embodiments, the antibody-drug conjugate is administered about once every 5 weeks at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700 mg per dose. In one or more embodiments, the antibody-drug conjugate is administered about once every 5 weeks at about 5 mg, about 50 mg, about 100 mg, about 150 mg, about 200 mg, about 250 mg, about 300 mg, about 400 mg, about 500 mg, about 600 mg, about 700 mg, about 1000 mg, about 1500 mg, about 2000 mg, about 2500 mg, or about 3000 mg, or a range between any two of these values (inclusive) or any value therein per dose.

In one or more embodiments, the antibody-drug conjugate is administered about once every 6 weeks at about 0.1-30 mg/kg per dose. In one or more embodiments, the antibody-drug conjugate is administered about once every 6 weeks at about 0.1-10 mg/kg per dose. In one or more embodiments, the antibody-drug conjugate is administered about once every 6 weeks at about 0.1 mg/kg, about 0.5 mg/kg, about 0.8 mg/kg, about 1 mg/kg, about 1.2 mg/kg, about 2.4 mg/kg, about 2.7 mg/kg, about 3.0 mg/kg, about 3.3 mg/kg, about 3.6 mg/kg, about 4.8 mg/kg, about 6.0 mg/kg, about 7.2 mg/kg, about 8.4 mg/kg, or about 10 mg/kg, or a range between any two of these values (inclusive) or any value therein per dose. In one or more embodiments, the antibody-drug conjugate is administered about once every 6 weeks at about 5-3000 mg per dose. In one or more embodiments, the antibody-drug conjugate is administered about once every 6 weeks at about 5-2000 mg, 5-1500 mg, 5-1000 mg, 5-800 mg, 5-600 mg, 48-432 mg, 48-216 mg, 72-504 mg, 100-2000 mg, 200-1000 mg, 300-800 mg, or 400-700 mg per dose. In one or more embodiments, the antibody-drug conjugate is administered about once every 6 weeks at about 5 mg, about 50 mg, about 100 mg, about 150 mg, about 200 mg, about 250 mg, about 300 mg, about 400 mg, 500 mg, about 600 mg, about 700 mg, about 1000 mg, about 1500 mg, about 2000 mg, about 2500 mg, or about 3000 mg, or a range between any two of these values (inclusive) or any value therein per dose.

In one or more embodiments, the patient receives one cycle of treatment. In one or more embodiments, the patient receives more than one (e.g., 2, 3, 4, 5, 6, 7, 8, 9, 10, 11, 12, 13, 14, 15, 16, 17, 18, 19, 20, 21, 22, 23, 24, 25 or 26, or a range between any two of these values (inclusive) or any value therein) cycles of treatment. In one or more embodiments, the patient receives the treatment until the condition is alleviated and no treatment is required.

In one or more embodiments, in the method for treating the solid tumor disclosed herein, the antibody-drug conjugate may be administered by any convenient route, for example, by infusion or bolus injection, or by absorption through epidermis, skin, or mucosa (e.g., oral mucosa, rectal and intestinal mucosa, etc.). Accordingly, the pharmaceutical composition may be administered intravenously, subcutaneously, orally, rectally, parenterally, intracerebrally, intravaginally, intraperitoneally, topically (e.g., by powder, ointment, drop, or transdermal patch), or buccally, or by oral or nasal spray. In one or more embodiments, the administration route is intravenous infusion or subcutaneous injection.

In one or more embodiments, the antibody-drug conjugate may be formulated into a pharmaceutical composition comprising the antibody-drug conjugate at the single dose as described above, e.g. a sterile isotonic aqueous solution, and administered to the patient in a form suitable for the selected administration route, e.g., parenteral, intravenous (i.v.), intramuscular, local, or subcutaneous (s.c.) administration. In one or more embodiments, the administration route is intravenous infusion or subcutaneous injection.

In one or more embodiments, in the method or use for treating the solid tumor disclosed herein, the antibody-drug conjugate is used in combination with one or more additional therapies. Suitable additional therapies include existing drugs (second drugs) and/or surgical therapies for specific applications (such as cancer). For example, the antibody-drug conjugate is used in combination with one or more additional chemotherapeutic or anti-tumor agents. Alternatively, the additional therapy is radiotherapy. In one or more embodiments, the chemotherapeutic agent is an apoptosis inducer.

In one or more embodiments, the antibody-drug conjugate and the additional agent are formulated into a single therapeutic composition, and the antibody-drug conjugate and the additional agent are administered simultaneously. Alternatively, the antibody-drug conjugate and the additional agent are separated from each other, e.g., prepared into separate therapeutic compositions, and the antibody-drug conjugate and the additional agent are administered simultaneously, or the antibody-drug conjugate and the additional agent are administered at different time points in the treatment regimen. For example, the antibody-drug conjugate is administered prior to the administration of the additional agent, the antibody-drug conjugate is administered subsequent to the administration of the additional agent, or the antibody-drug conjugate and the additional agent are administered in an alternating manner. Herein, the antibody-drug conjugate and the additional agent are administered at a single dose or at multiple doses.

In one or more embodiments, in the method for treating the solid tumor disclosed herein, the antibody-drug conjugate and the additional agent may be administered by any convenient route, for example, by infusion or bolus injection, or by absorption through epidermis, skin, or mucosa (e.g., oral mucosa, rectal and intestinal mucosa, etc.), and may be administered in combination with an additional bioactive agent. Accordingly, the pharmaceutical composition may be administered intravenously, subcutaneously, orally, rectally, parenterally, intracerebrally, intravaginally, intraperitoneally, topically (e.g., by powder, ointment, drop, or transdermal patch), or buccally, or by oral or nasal spray. In one or more embodiments, the administration route is intravenous infusion or subcutaneous injection.

In one or more embodiments, the antibody-drug conjugate and/or the additional agent in combination with the antibody-drug conjugate may be formulated into a pharmaceutical composition and administered to the patient in a form suitable for the selected administration route, e.g., parenteral, intravenous (i.v.), intramuscular, local, or subcutaneous (s.c.) administration. In one or more embodiments, the administration route is intravenous infusion or subcutaneous injection.

In one or more embodiments, the antibody-drug conjugate or the pharmaceutical composition comprising the antibody-drug conjugate is administered by intravenous (i.v.) infusion (i.e., intravenous infusion). In one or more embodiments, the duration of the intravenous infusion of the antibody-drug conjugate or the pharmaceutical composition comprising the antibody-drug conjugate is about 15 min, about 30 min, about 35 min, about 40 min, about 45 min, about 50 min, about 55 min, about 60 min, about 65 min, about 70 min, about 75 min, about 80 min, about 85 min, about 90 min, about 95 min, about 100 min, about 105 min, about 110 min, about 105 min, about 120 min, about 135 min, about 150 min, about 165 min, about 180 min, about 195 min, about 210 min, about 225 min, or about 240 min, or a range between any two of these values (inclusive) or any value therein. In one or more embodiments, the duration of the first intravenous infusion of the antibody-drug conjugate or the pharmaceutical composition comprising the antibody-drug conjugate is ≥3 hours, and the duration of each subsequent intravenous infusion is 1-2 hours.

In one or more embodiments, the intravenous infusion rate of the antibody-drug conjugate or the pharmaceutical composition comprising the antibody-drug conjugate is ≤2000 mg/hr. In one or more embodiments, the intravenous infusion rate of the antibody-drug conjugate or the pharmaceutical composition comprising the antibody-drug conjugate is ≤1000 mg/hr. In one or more embodiments, the intravenous infusion rate of the antibody-drug conjugate or the pharmaceutical composition comprising the antibody-drug conjugate is ≤500 mg/hr. In one or more embodiments, the rate of the first intravenous infusion of the antibody-drug conjugate or the pharmaceutical composition comprising the antibody-drug conjugate is ≤500 mg/hr, and the rate of each subsequent intravenous infusion is ≤1000 mg/hr. In one or more embodiments, when the antibody-drug conjugate or the pharmaceutical composition comprising the antibody-drug conjugate is administered by intravenous infusion, the rate is ≤200 mg/hr for 0 to 15 min (initial rate) and can be increased by 50-200 mg/hr every 15 to 30 min since 15 min. In one or mose embodiments, when the antibody-drug conjugate or the pharmaceutical composition comprising the antibody-drug conjugate is administered at the first intravenous infusion dose, the rate is ≤50 mg/hr for 0 to 15 min (initial rate) and can be increased by 50 mg/hr every 15 to 30 min since 15 min. For each subsequent dose, the rate is 100-200 mg/hr for 0 to 15 min (initial rate) and can be increased by 100-200 mg/hr every 15 to 30 min since 15 min.

## BRIEF DESCRIPTION OF THE DRAWINGS

FIG. 1 shows the bystander effects of ADC1 and ADC2;
FIG. 2 shows the changes in the plasma concentration of ADC1 in rats;
FIG. 3 shows the effect of ADC1 in inhibiting tumor growth;
FIG. 4 shows the effects of ADC1 and ADC3 in inhibiting tumor growth.

## DETAILED DESCRIPTION

"About" refers to a general error range for corresponding values as readily understood by those skilled in the relevant art. In some embodiments, "about" mentioned herein refers to the values described and ranges thereof of ± 10%, ± 5%, or ± 1%.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of the extent of disease, stabilized (i.e., not worsening) state of disease, delay or slowing of progressive disease, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from the administration of the antibody or the pharmaceutical composition disclosed herein for detection, diagnostic procedures, and/or treatment.

The terms "TROP2-positive solid tumor" includes cancer cells with TROP2 levels higher than normal tissues. Examples of TROP2-positive solid tumor include but are not limited to TROP2-positive triple-negative breast cancer, TROP2-positive pancreatic cancer, urothelial cancer, non-small cell lung cancer, small cell lung cancer, TROP2 positive three negative breast cancer, TROP2 positive pancreatic cancer, urothelial cancer, non small cell lung cancer, small cell lung cancer, glioblastoma, neuroblastoma, breast cancer, head and neck cancer, kidney cancer, ovarian cancer, gastric

cancer, Kaposi sarcoma, lung cancer, cervical cancer, carcinoma of colon and rectum, esophageal cancer, oral squamous cell cancer, prostate cancer, thyroid cancer, bladder cancer, neuroglioma, hepatobiliary cancer, carcinoma of colon and rectum, T-cell lymphoma, colorectal cancer, prostate cancer, melanoma and liver cancer.

As used herein, the term "effective amount" or "therapeutically effective amount" refers to the amount of a drug, e.g., an antibody or ADC, that is sufficient to reduce or ameliorate the severity and/or duration of a condition (e.g., cancer) or one or more symptoms thereof, prevent the progression of a condition, cause regression of a condition, prevent the recurrence, development, onset or progression of one or more symptoms associated with a condition, detect a condition, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with cancer. For example, the effective amount may improve progression-free survival (PFS), improve overall survival (OS), or decrease the likelihood of recurrence.

The terms "patient" and "subject" are used interchangeably and refer to any mammal in need of diagnosis, prognosis, or treatment, including, but not limited to, humans, dogs, cats, guinea pigs, rabbits, rats, mice, horses, cattle, etc., particularly animals with one or more conditions of solid tumors such as a TROP2-positive solid tumor. In one or more embodiments, the patient is a human.

"Survival" means that the patients remain alive, and includes disease-free survival (DFS), progression-free survival (PFS), and overall survival (OS). Survival can be evaluated by the Kaplan-Meier method, and any difference in the survival can be calculated using a hierarchical log-rank test.

"Disease-free survival" refers to the time a patient survives from the initiation of therapy or the initial diagnosis without recurrence of the cancer. Events used in DFS analysis may include local, regional and distant cancer recurrence, secondary cancer occurrence, and death from any cause in patients without prior events.

"Extended survival" means increased PFS, DFS, and/or OS in a treated patient relative to an untreated patient or relative to a control treatment regimen.

"Chemotherapy" or "chemotherapeutic agents"is the use of chemotherapeutic agents that can be used to treat cancer.

"$IC_{50}$" represents 50% inhibitory concentration, i.e., a concentration of drug or inhibitor required to inhibit half of a given biological process.

The term "administration" as used herein refers to administering a substance for therapeutic purposes (e.g., treating a tumor).

The term "drug for treating a tumor" as used herein refers to an agent having the functional property of inhibiting in a human the development or progression of a tumor, particularly a malignant (cancerous) lesion, such as cancer, sarcoma, lymphoma, or leukemia. Inhibition of metastasis is a property of anti-tumor drugs in many cases.

MTD definition: MTD (maximum tolerated dose) is defined as the highest dose level at which ≤ 1/6 subjects in a dose group were observed to experience DLT during the DLT evaluation period.

PK (pharmacokinetic) parameters used herein are defined as follows:

PK parameters of single administration:$C_{max}$, $T_{max}$, $T_{1/2}$, CL, Vd, Ke, MRT, $AUC_{(0-\tau)}$, $AUC_{(0-\infty)}$:

| | |
|---|---|
| $C_{max}$ | Peak concentration. Directly obtained from the measured plasma concentration-time data. |
| $T_{max}$ | Time to peak. Directly obtained from the measured plasma concentration-time data. |
| $t_{1/2}$ | Terminal elimination half-life. $t_{1/2}=\ln 2/\lambda_z$ |
| CL | Apparent clearance. $CL= D_{po} / AUC_{0-\infty}$ |
| Vd | Apparent volume of distribution. $Vd=D_{po}/ AUC_{0-\infty}/\lambda_z$ |
| Ke ($\lambda_z$) | Elimination rate constant, a slope of the terminal segment of a semi-logarithmic log of drug concentration-time curve calculated by using the linear regression method. |
| $MRT_{0-t}$ | Mean residence time, calculated by the formula: $AUMC_{0-t}/AUC_{0-t}$, where the calculation formula for $AUMC_{0-t}$ is: $AUMC_{(i,\ i+1)}= (t_{i+1} - t_i)(t_{i+1} * C_{i+1} - t_i * C_i)/2$, and AUMC is the sum of all $AUMC_{(i,\ i+1)}$ |
| $MRT_{0-\infty}$ | Calculation formula: $AUMC_{0-\infty}/AUC_{0-\infty}$, where $AUMC_{0-\infty}= AUMC_{0-t}+ T_{last} *\lvert C_{last}\rvert/\lambda_z+\lvert C_{last}\rvert/(\lambda_z*\lambda_z)$ |
| $AUC_{0-t}$ | Area under the curve from zero to the time at which the lowest plasma concentration can be detected. Calculated by the linear trapezoidal rule: $AUC_{(i,\ i+1)} = (T_{i+1} - T_i)(C_i + C_{i+1})/2$, where $AUC_{0-t}$ is the sum of all $AUC_{(i,\ i+1)}$. |
| $AUC_{0-\infty}$ | Area under the curve of time extrapolated from zero to infinity. $AUC_{0-\infty} = AUC_{0-t} + C_t/\lambda_z$ ($C_t$ is the last measurable plasma concentration). |

(continued)

| | |
|---|---|
| $AUC_{_\%Extrap}$ | The proportion of AUC theoretically extrapolated from the last point to infinity relative to $AUC_{0-\infty}$ |

PK parameters of multiple doses: $C_{max,ss}$, $C_{avg,ss}$, $C_{min,ss}$, $AUC_{(0-\tau)ss}$, $AUC_{(0-\infty)ss}$, $T_{max,ss}$, $T_{1/2,ss}$, CL, $V_{ss}$, Ke, MRT, accumulation index (Rac), and fluctuation index DF:

| | |
|---|---|
| $C_{max, ss}$ | Peak concentration at steady state. Directly obtained from the measured plasma concentration-time data at steady state within dosing intervals. |
| $C_{min, ss}$ | Minimum concentration at steady state. Directly obtained from the measured plasma concentration-time data at steady state within dosing intervals. |
| $C_{avg,ss}$ | Mean plasma concentration at steady state within dosing intervals. Cav = $AUC_{0-\tau}/\tau$, where $\tau$ is the dosing interval |
| $AUC_{(0-t)ss}$ | Area under the curve at steady state from zero to the time at which the lowest plasma concentration can be detected. Calculated by the linear trapezoidal rule: $AUC_{(i,\ i+1),ss} = (T_{i+1} - T_i)(C_i + C_{i+1})/2$, $AUC_{0-t}$, where $_{ss}$ is the sum of all $AUC_{(i,\ i+1),ss}$. |
| $AUC_{(0-\infty)ss}$ | Area under the curve of time at steady state extrapolated from zero to infinity. $AUC_{0-\infty,ss} = AUC_{0-t,ss} + C_t/\lambda_z$ ($C_t$ is the last measurable plasma concentration at steady state). |
| $T_{max,ss}$ | Time to peak. Directly obtained from the measured plasma concentration-time data. |
| $t_{1/2,ss}$ | Terminal elimination half-life. $t_{1/2,ss} = \ln 2/\lambda_z$ |
| $CL_{ss}$ | Apparent clearance at steady state. $CL_{ss}= D_{po} /AUC_{ss}$. |
| Vd | Volume of distribution at steady state, Vd $=D_{po}/ AUC_{0-\infty,ss}/\lambda_z$ |
| Ke ($\lambda_z$) | Elimination rate constant, a slope of the terminal segment of a semi-logarithmic log of drug concentration-time curve calculated by using the linear regression method. |
| $MRT_{0-\infty,\ ss}$ | Calculation formula: $AUMC_{0-\infty}$,ss/$AUC_{0-\infty,ss}$, where $AUMC_{0-\infty,\ ss} = AUMC_{0-t,ss} + T_{last} \times \lvert C_{last}\rvert/\lambda_z + \lvert C_{last}\rvert/(\lambda_z \times \lambda_z)$ |
| Rac | $\mathrm{Rac}=\frac{1}{1-e^{-Ke\tau}}$ Accumulation ratio. |
| DF | The degree of fluctuation. DF=( $C_{max,ss}$- $C_{min,ss}$)/ $C_{avg,ss}$ |
| $AUC_{_\%Extrap}$ | The proportion of AUC theoretically extrapolated from the last point to infinity relative to $AUC_{0-\infty}$ |

ORR (objective response rate): the proportion of patients whose tumors have shrunk by a certain amount and have been maintained for a certain period of time, including cases of CR (complete response) and PR (partial response).

Duration of response (DOR): DOR is defined as the time from the first evaluation of objective response to the first evaluation of PD (progressive disease) or any-cause death before PD, reflecting the duration of ORR.

Disease control rate (DCR): the proportion of patients whose tumors have shrunk or stabilized and have been maintained for a certain period of time, including cases of CR, PR, and SD (stable disease).

Progression-free survival (PFS): the time from the first dose to the occurrence of objective progression or all-cause death of the tumor (whichever occurs first).

Overall survival (OS): the time from the date of the first dose to the occurrence of death from any cause, and for subjects who are still alive at the time of analysis, the deadline will be the date of their last contact.

The term "antibody-drug conjugate" and "ADC", are used interchangeably and refer to a binding protein (e.g., an antibody or an antigen-binding unit thereof) that is linked to one or more chemical drugs, which may optionally be a therapeutic agent or a cytotoxic agent. In some embodiments, the ADC comprises an antibody, a drug (e.g., a cytotoxic drug), and a linker capable of attaching or conjugating the drug to the antibody. Non-limiting examples of drugs that can be included in the ADC include a mitotic inhibitor, an anti-tumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor), and a radiosensitizer.

The term "drug to antibody ratio" or "DAR" refers to the number of drugs (e.g., Exatecan) that are attached to one antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of drugs in a composition, i.e., the mean drug binding number of an antibody, or the mean drug to antibody ratio, the value is selected from about 0 to about 10, or about 2 to about 8. In some embodiments, the mean drug to antibody ratio is about 3 to about 6. In some embodiments, the mean drug to antibody ratio is about 4 to about 5. In other embodiments, the mean drug to antibody ratio is about 6 to about 8, or about 7 to about 8. DAR values may be denoted herein by p. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

The "antibody" or "antigen-binding fragment" refers to a polypeptide or polypeptide complex that specifically recognizes and binds to an antigen. The antibody may be an intact antibody and any antigen-binding fragment or single chain thereof. The term "antibody" thus includes any protein or peptide comprising at least a portion of an immunoglobulin molecule that has biological activity for binding to an antigen. The antibody and the antigen-binding fragment include, but are not limited to, complementarity determining regions (CDRs) of a heavy or light chain or a ligand binding portion thereof, heavy chain variable regions (VHs), light chain variable regions (VLs), heavy chain constant regions (CHs), light chain constant regions (CLs), framework regions (FRs) or any portion thereof, or at least a portion of a binding protein. The CDRs include light chain CDRs (LCDR1-3) and heavy chain CDRs (HCDR1-3). An antibody or an antigen-binding fragment can specifically recognize and bind to one or more (e.g., two) polypeptides or polypeptide complexes of antigens. The antibody or the antigen-binding fragment that specifically recognizes and binds to multiple (e.g., two) antigens can be referred to as a multispecific (e.g., bispecific) antibody or an antigen-binding fragment.

The term "antibody fragment" or "antigen-binding fragment" refers to a part of an antibody, and the composition of the antibody fragment of the present invention may be similar to F(ab')2, F(ab)2, Fab', Fab, Fv, scFv and the like in monospecific antibody fragments. Regardless of its structure, the antibody fragment binds to the same antigen recognized by an intact antibody. The term "antibody fragment" includes aptamers, mirror isomers, and bivalent antibodies. The term "antigen-binding fragment" also includes any synthetic or genetically engineered protein that functions as an antibody by binding to a specific antigen to form a complex.

The "polypeptide" refers to a molecule consisting of two or more amino acid monomers linearly linked by amide bonds (also referred to as peptide bonds), and may include dipeptides, tripeptides, tetrapeptides, oligopeptides, etc.

The "amino acid" refers to an organic compound containing both amino and carboxyl, such as $\alpha$-amino acids and $\beta$-amino acids. Amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), valine (Val, V), sarcosine (Sar), theanine (Thea), hydroxyproline (Hypro), hydroxylysine (Hylys), $\beta$-aminoisobutyric acid ($\beta$-AiBA), citrulline (Cit), $\beta$-alanine ($\beta$-Ala), etc.

Those skilled in the art will appreciate that when an amino acid or polypeptide is a constituent part of a molecule (such as an antibody or ADC), the amino acid or polypeptide refers to the amino acid residue or polypeptide residue (whether written or not), that is, when it's connected with other parts of the molecule, some of its groups (such as a hydrogen atom of its amino group and/or a hydroxyl group of a carboxyl group) are lost due to the formation of covalent bonds (such as amide bonds) with other parts of the molecule.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino, and aryl) is indicated by the prefix "Cx-Cy" or "Cx-y", wherein x is the minimum number of carbon atoms and y is the maximum number of carbon atoms. Thus, for example, "C1-C6 alkyl" refers to an alkyl containing 1 to 6 carbon atoms. If a substituent is described as "substituted with ...", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. For illustration, monofluoroalkyl is an alkyl substituted with one fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with ...", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, thiol, alkylthio, amino, C1-C6 aminoalkyl, C1-C6 aminoalkylamino, C1-C6 alkyl connected to a heterocyclic ring, C1-C6 alkylamino connected to a heterocyclic ring, heterocyclyl, amino-substituted heterocyclyl, heterocyclylamino, carbamoyl, morpholin-1-yl, piperidin-1-yl, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8

heterocyclyl, $-(CH_2)_q$-(C3-C8 heterocyclyl)-$CH_3$, -(C3-C8 heterocyclyl)-$(CH_2)_q$-$CH_3$, $-(CH_2)_qC(O)NR^n(CH_2)_q-CH_3$, $-(CH_2CH_2O)_q-CH_3$, $-(CH_2CH_2O)_q-CH_2-CH_3$, $-(CH_2)_qC(O)NR^n(CH_2CH_2O)_q-CH_3$, $-(CH_2)_qC(O)NR^n(CH_2CH_2O)_q-CH_2-CH_3$, $-(CH_2CH_2O)_qC(O)NR^n(CH_2CH_2O)_q-CH_3$, $-(CH_2CH_2O)_qC(O)NR^n(CH_2CH_2O)_q-CH_2-CH_3$, or $-(CH_2CH_2O)_qC(O)NR^n(CH_2)_q-CH_3$; wherein each $R^n$ is independently H, C1-C6 alkyl, C3-C8 carbocyclyl, phenyl, or benzyl, and each q is independently 1, 2, 3, 4, 5, 6, 7, 8, 9, or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, n-propyl, isopropyl, n-butyl, isobutyl, sec-butyl, tert-butyl, n-pentyl, neopentyl, n-hexyl, etc. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphono, etc.

The term "alkenyl", by itself or as part of another substituent, refers to an unsaturated branched, linear, or cyclic alkyl having at least one carbon-carbon double bond derived by the removal of one hydrogen atom from a single carbon atom of a parent olefin. Typical alkenyl includes, but is not limited to, ethenyl; propenyl (e.g., prop-1-en-1-yl, prop-1-en-2-yl, prop-2-en-1-yl, prop-2-en-2-yl, or cycloprop-1-en-1-yl); cycloprop-2-en-1-yl; butenyl (e.g., but-1-en-1-yl, but-1-en-2-yl, 2-methyl-prop-1-en-1-yl, but-2-en-1-yl, but-2-en-2-yl, but-1,3-dien-1-yl, but-1,3-dien-2-yl, cyclobut-1-en-1-yl, cyclobut-1-en-3-yl, cyclobut-1,3-dien-1-yl, etc.), and the like.

The term "alkynyl", by itself or as part of another substituent, refers to an unsaturated branched, linear, or cyclic alkyl having at least one carbon-carbon triple bond derived by the removal of one hydrogen atom from a single carbon atom of a parent alkyne. Typical alkynyl includes, but is not limited to, ethynyl; propynyl (e.g., prop-1-yn-1-yl, prop-2-yn-1-yl, etc.); butynyl (e.g., but-1-yn-1-yl, but-1-yn-3-yl, but-3-yn-1-yl, etc.), and the like.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically indicated, the carbocyclyl may be optionally substituted with one or more substituents independently selected from: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated $\pi$-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphono, etc. Examples of unsubstituted aryl include, but are not limited to, phenyl, naphthyl, and anthracenyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4H)-ylidene, tetrahydrofuranyl, trioxacyclohexyl, trithianyl, triazinanyl, tetrahydropyranyl, thiomorpholinyl, thiomorpholinyl, 1-oxo-thiomorpholinyl, and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, n-propoxy, 1-methylethoxy (isopropoxy), n-butoxy, isobutoxy, sec-butoxy, and tert-butoxy. Alkoxy may be substituted or unsubstituted.

The "halogen" refers to fluoro (F), chloro (Cl), bromo (Br), or iodo (I).

The "amino" refers to-$NH_2$.

The "cyano" refers to -CN.

The "nitro" refers to -$NO_2$.

The "hydroxy" refers to -OH.

The "carboxyl" refers to -COOH.

The "thiol" refers to -SH.

The "carbonyl" refers to C=O.

"Stereoisomers" refer to isomeric compounds having the same linking order of atoms but different spatial arrangements of the atoms. Stereoisomers may have one or more stereocenters and each center may exist as R or S, and stereoisomers may also be cis-trans isomers. Stereoisomers of the compounds provided herein include any one of all diastereomeric, enantiomeric and cis-trans isomeric forms thereof, or suitable mixtures thereof.

The pharmaceutically acceptable salt includes those derived from the compound with a variety of organic and inorganic counterions well known in the art, and salts as examples only include organic or inorganic salts such as lithium salt, sodium salt, potassium salt, calcium salt, magnesium salt, ammonium salt, isopropylamine, trimethylamine, diethylamino, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethyl, piperidine, polyamine resin, and tetraalkylammonium salt when the molecule contains an acidic functional group; and organic or inorganic acid salts such as hydrochloride salt, hydrobromide salt, tartrate salt, mesylate salt, acetate salt, maleate salt, and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. These salts can generally be prepared by conventional methods by reacting, for example, an appropriate acid or base with the compound. The solvate includes hydrates.

Other chemical terms herein are used according to conventional usage in the art, such as the McGraw-Hill Dictionary of Chemical Terms (Ed. Parker, S., McGraw-Hill, San Francisco (1985)).

All the publications and patents cited herein are incorporated by reference in their entirety for all purposes.

The antibody-drug conjugate of the present invention is a TROP2 antibody-drug conjugate (TROP2-ADC) formed by conjugating an antibody or an antigen-binding unit thereof that binds to TROP2 to a drug. Antibody-drug conjugates (ADCs) can increase the therapeutic efficacy of antibodies in treating diseases (e.g., cancer) due to their ability to selectively deliver one or more drugs to a target tissue (e.g., a tumor expressing TROP2). In one or more embodiments, the TROP2-ADC of the present invention has properties including, but not limited to, binding to TROP2 (e.g., human TROP2) *in vitro,* binding to cells expressing TROP2, high affinity, strong internalization capability, and reducing or inhibiting the growth of cancer cells or tumors.

In one or more embodiments, the TROP2-ADC has a structure shown as Formula I, or a stereoisomer thereof., or a pharmaceutically acceptable salt or solvate thereof.

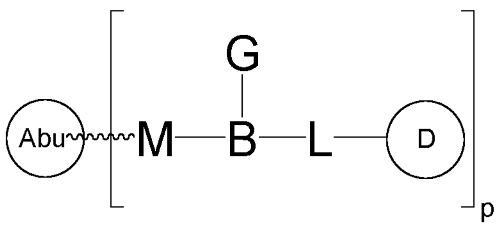

Formula I

wherein Abu, M, B, G, L, D, and p are as defined herein.

In one or more embodiments, Abu is an hRS9 antibody..

In one or more embodiments, the antibody-drug conjugate described herein has a significant bystander effect.

The present invention further provides a preparation method for intermediates and antibody-drug conjugates. The intermediates and the antibody-drug conjugates of the present invention can be prepared using known formulations and methods. In one or more embodiments, the preparation method is as follows.

Preparation method for compound as shown in Formula II

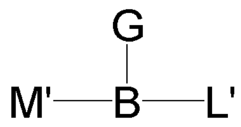

Formula II

wherein

M' is

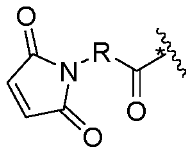

,

wherein * links to B, R is selected from: $-(CH_2)_r-$, $-(CHR^m)_r-$, C3-C8 carbocycl yl, $-O-(CH_2)_r-$, arylene, $-(CH_2)_r-$arylene-, -arylene-$(CH_2)_r-$, $-(CH_2)_r-$(C3-C8 carbocyclyl)-, -(C3-C8 carb ocyclyl)-$(CH_2)_r-$, C3-C8 heterocyclyl, $-(CH_2)_r-$(C3-C8 heterocyclyl)-, -(C3-C8 heterocyclyl)-$(CH_2)_r-$, - $(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC$ $(O)NR^m(CH_2CH_2O)_r-CH_2-$, $-(CH_2CH_2O)_rC(O)NR^m(CH_2CH_2O)_r-$, $-(CH_2CH_2O)_rC(O)NR^m(CH_2CH_2O)_r-CH_2-$ and $-(CH_2CH_2O)_rC(O)NR^m(CH_2)_r-$; wherein each $R^m$ is independently H, C1-C6 alkyl, C3-C8 car bocyclyl, phenyl or benzyl; and each r is independently 1, 2, 3, 4, 5, 6, 7, 8, 9 or 10;

L' is $-(AA)_i-(FF')_f$, wherein, AA, i, f are described as in Formula I; each FF' is independently

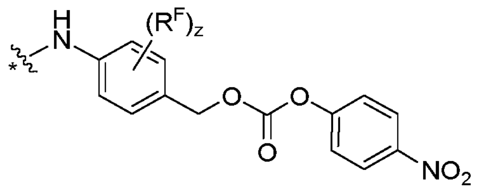

,

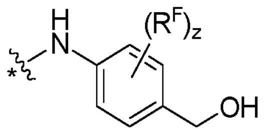

,

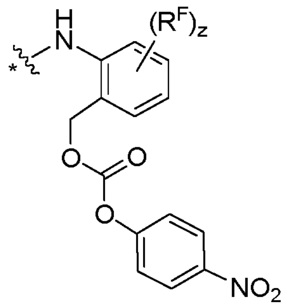

,

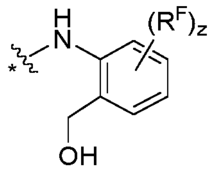

,

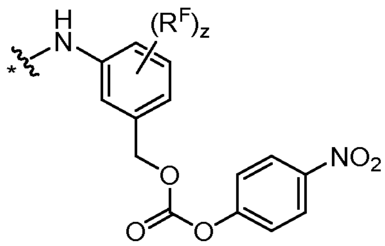

or

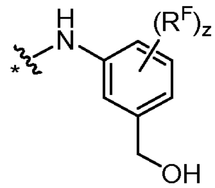

,

wherein each $R^F$ is independently C1-C6 alkyl, C1-C6 alkoxy, $-NO_2$ or halogen; z is 0, 1, 2, 3 or 4; wherein * links to AA;

B, G are described as in Formula I.

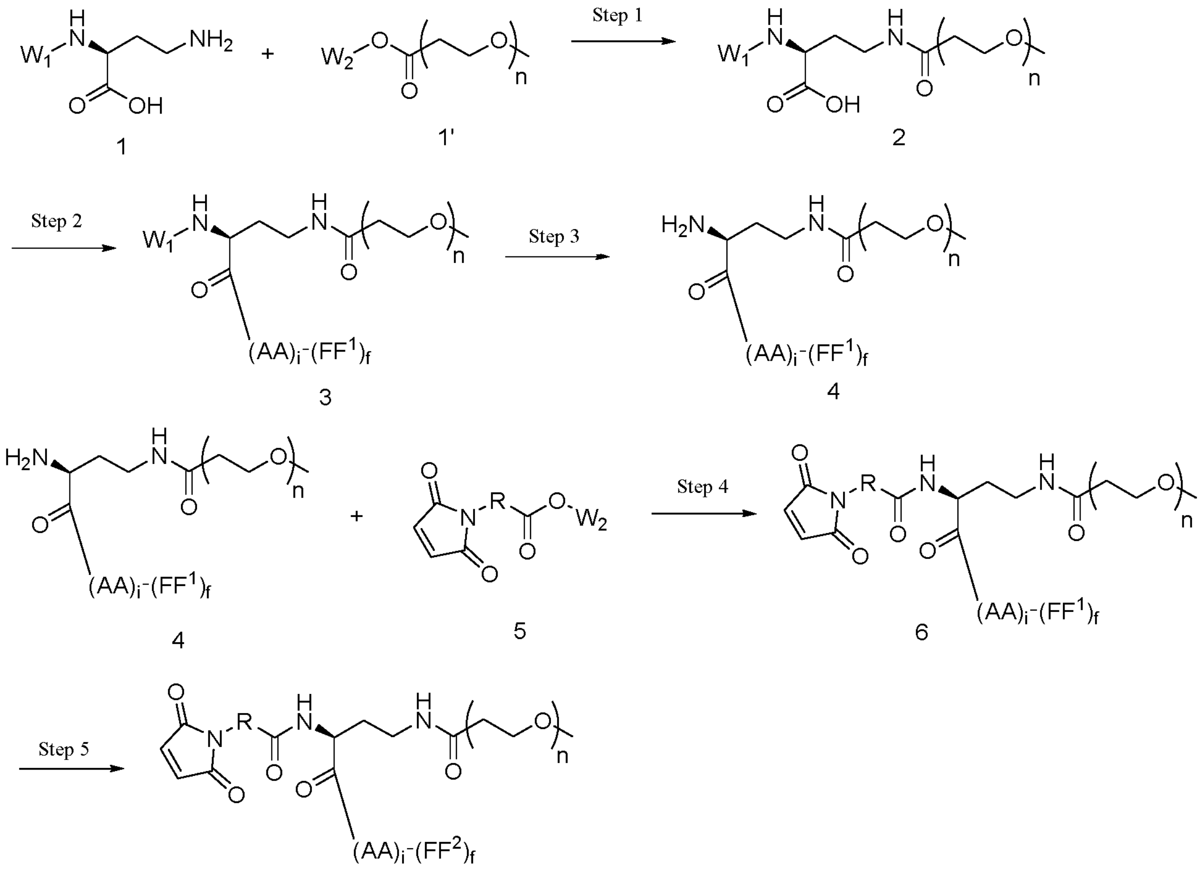


1 1' 2 3 4 4 5 6 7

Step 1: reacting a compound of general Formula 1 with a compound of general Formula 1' under a basic condition to obtain a compound of general Formula 2;

Step 2: reacting the compound of general Formula 2 with general Formula $(AA)_i$-$(FF^1)_f$ in the presence of a condensing agent under a basic condition to obtain a compound of general Formula 3;

Step 3: removing the amino-protecting group $W_1$ of the compound of general Formula 3 to obtain a compound of general Formula 4;

Step 4: reacting the compound of general Formula 4 with a compound of general Formula 5 under a basic condition to obtain a compound of general Formula 6; and

Step 5: reacting the compound of general Formula 6 with bis(p-nitrophenyl)carbonate under a basic condition to obtain a compound of general Formula 7.

wherein

$W_1$ is an amino-protecting group, e.g., 9-fluorenylmethyloxycarbonyl; $W_2$ is a carboxylic acid active ester, for example, a succinimidyl ester.

wherein n, AA, R, i, f are described as in Formula II herein, each $FF^1$ is independently

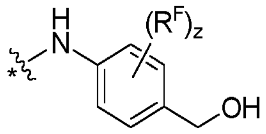, 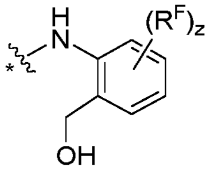 or 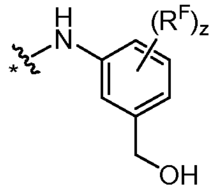,

wherein each $R^F$ is independently C1-C6 alkyl, C1-C6 alkoxy, $-NO_2$ or halogen; z is 0, 1, 2, 3 or 4; wherein * links to AA; each $FF^2$ is independently

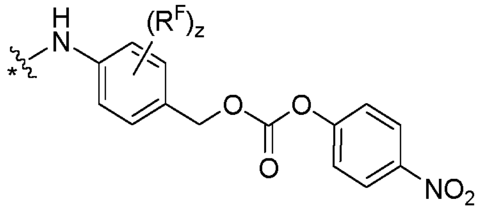, 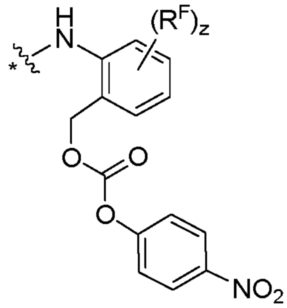

or

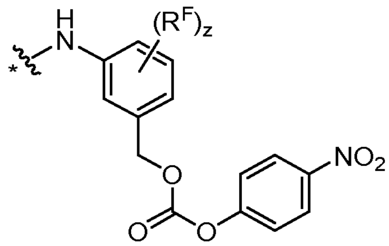,

wherein each $R^F$ is independently C1-C6 alkyl, C1-C6 alkoxy, $-NO_2$ or halogen;z is 0, 1, 2, 3 or 4;wherein * links to AA.

In one or more embodiments, $R^F$ is F.

In one or more embodiments, z is 0.

In one or more embodiments, z is 1 or 2.

The basic conditions described above can be provided using reagents including organic bases and inorganic bases, wherein the organic bases include, but are not limited to, triethylamine, diethylamine, *N*-methylmorpholine, pyridine, piperidine, *N*,*N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N',N'-tetramethyl-O-(7-azabenzotriazol-1-yl) urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azobenzotriazol, O-benzotriazol-N,N,N',N'-tetramethyluronium hexafluorophosphate, 2-(7-azobenzotriazol)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method for intermediate as shown in Formula III

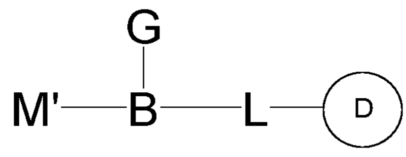

Formula III

wherein, B, G, L, D are described as in Formula I, M' is described as in Formula II.

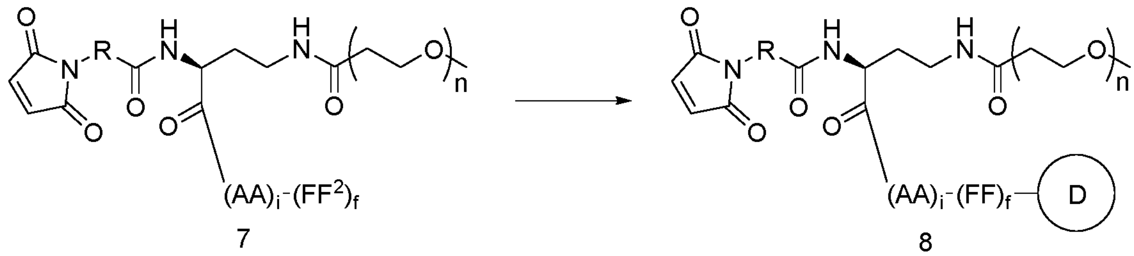

7 8

wherein n, AA, R, i, f, FF$^2$, FF, D are described herein.

The compound of general Formula 7 and D are reacted in the presence of a condensing agent under a basic condition to obtain a compound of general Formula 8.

The basic conditions described above can be provided using reagents including organic bases and inorganic bases, wherein the organic bases include, but are not limited to, triethylamine, diethylamine, N-methylmorpholine, pyridine, piperidine, *N*,*N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N',N'-tetramethyl-O-(7-azabenzotriazol-1-yl) urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azobenzotriazol, O-benzotriazol-N,N,N',N'-tetramethyluronium hexafluorophosphate, 2-(7-azobenzotriazol)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

Preparation method for

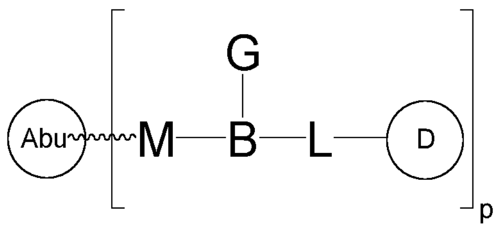

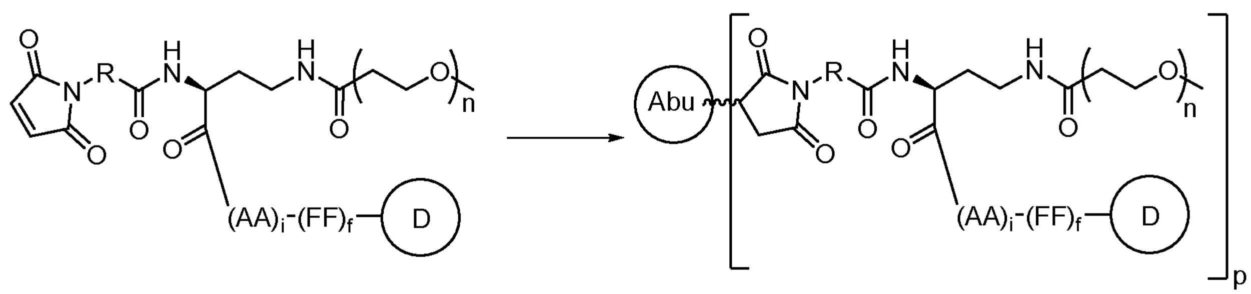

8 9

wherein n, AA, R, i, f, FF, D and Abu are described herein.

The compound of general Formula 8 is conjugated to Abu under a weakly acidic condition to obtain a compound

of Formula 9.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, acetic acid, hypochlorous acid, hydrofluoric acid, sulfurous acid, hydrosulohuric acid, silicic acid, metasilicic acid, phosphoric acid, metaphosphoric acid, sodium bicarbonate, and sodium bisulfite.

In one or more embodiments, the ADC of the present invention may be used in combination with other therapeutic or prophylactic regimens, including use of one or more ADCs of the present invention together with or in combination with one or more other therapeutic agents or methods. For combination therapy, the ADC may be administered simultaneously or separately with other therapeutic agents. When administered separately, the ADC of the present invention may be administered before or after the administration of another additional therapeutic agent.

## Examples

The materials, reagents, etc., used in the following examples can be commercially available or obtained using known methods unless otherwise stated.

## Example 1. Preparation of antibodies

The following antibodies were prepared using conventional methods. After vector construction, eukaryotic cells such as HEK293 cells (Life Technologies Cat. No. 11625019) were transiently transfected, or CHO cells were stably transfected, and stable cell lines were selected and purified for expression.

Preparation and purification of hRS9 antibody: Referring to the method of Wood et al., J Immunol., 145:3011 (1990*),* etc., an anti-Trop2 specific monoclonal antibody was produced in CHO cells. Expression vectors containing antibody genes were constructed using conventional molecular biological methods, wherein the amino acid sequences of the heavy chain and the light chain of the recombinant humanized anti-Trop2 monoclonal antibody hRS9 antibody were set forth in SEQ ID NO: 1 and SEQ ID NO: 2, respectively.

## Example 2. Synthesis Procedures for Compound (1S,9S)-1-amino-9-ethyl-4,5-difluoro-9-hydroxy-1,2,3,9,12,15-hexahydro-10H,13H-benzo[de]pyrano[3',4':6,7]indolizino[1,2-b]quinolin-10,13-dione hydrochloride (D-1)

1. Synthesis of*N*,*N*'-(3,4-difluoro-8-oxo-5,6,7,8-tetrahydronaphthalene-1,7-diyl)diacetamide

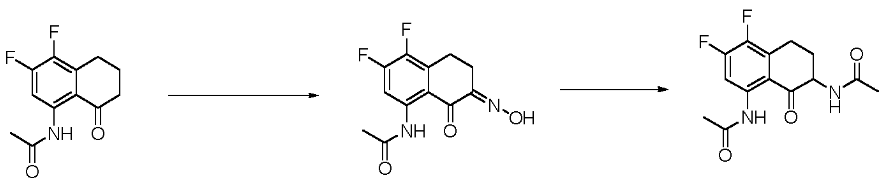

A solution of potassium tert-butoxide in tetrahydrofuran (42 mL, 1 M) was added to a dry reaction flask under a nitrogen atmosphere, stirred, and cooled to 0-5 °C. N-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by tert-butyl nitrite (4.32 g, 2 eq) (with temperature controlled at 0-5 °C). The resulting mixture was warmed to 15-20 °C and stirred for 2 hours (h or hr). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). With temperature maintained at 5-10 °C, zinc powder (8 eq) was added according to the amount of N-(3,4-difluoro-8-oxo-5,6,7,8-tetrahydronaphthalen-1-yl)acetamide. The mixture was stirred at 20-25 °C for 1 h. The mixture was filtered, and the solid was rinsed with ethyl acetate (50 mL). The temperature was reduced to 0-5 °C, and 15% aqueous solution of $NaCO_3$ (50 mL) was added dropwise for three washings. Ethyl acetate (25 mL) was added for extraction. The organic phases were pooled and washed with a saturated aqueous solution of NaCl. Ethyl acetate (10 mL) was added, and the mixture was stirred at 40 °C for 30 min, slowly cooled to 0-5 °C, and stirred for 2 h. The mixture was filtered, and the solid was washed with ethyl acetate/petroleum ether (1/2, 10 mL). Drying was performed in vacuo to obtain a gray powder (2.1 g, 33.7%). LC-MS: $[M+H]^+$=297.

2. Synthesis of N-(8-amino-5,6-difluoro-1-oxo-1,2,3,4-tetrahydronaphthalen-2-yl)acetamide

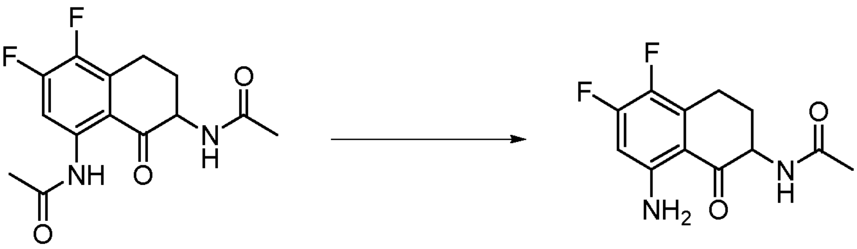

*N,N'*-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The resulting mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: $[M+H]^+$=255.

3. Synthesis of *N*-((9*S*)-9-ethyl-4,5-difluoro-9-hydroxy-10,13-dioxo-2,3,9,10,13,15-hexahydro-1*H*,12*H*-benzo[*de*]pyrano[3';4':6,7]indolizino[1,2-*b*]quinolin-1-yl)acetamide

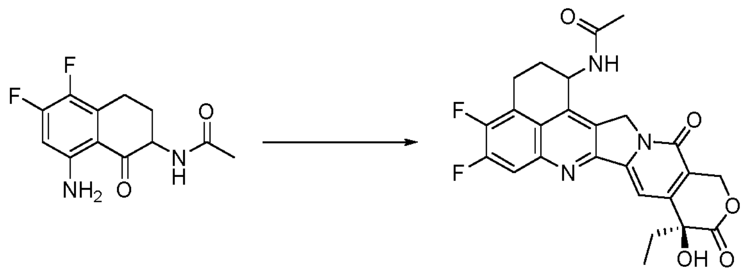

N-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-*f*]indolizine-3,6,10(4*H*)-trione (1.15 g, 1 eq) and toluene (50 ml) were added to a reaction flask under a nitrogen atmosphere. The mixture was warmed to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: $[M+H]^+$=482.

4. Synthesis of (1*S*,9*S*)-1-amino-9-ethyl-4,5-difluoro-9-hydroxy-1,2,3,9,12,15-hexahydro-10*H*,13*H*-benzo[*de*]pyrano[3',4':6,7]indolizino[1,2-*b*]quinolin-10,13-dione hydrochloride

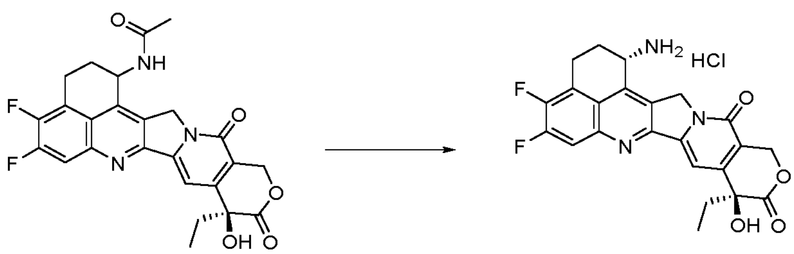

N-((9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)acetamide (1.1 g, 2.28 mmol) and a 6 M aqueous solution of hydrochloric acid (44

mL) were added to a reaction flask. The mixture was refluxed with stirring under a nitrogen atmosphere for 4 h. The mixture was concentrated to remove the solvent, and the residue was purified by HPLC to obtain a white powder (200 mg, 18%). LC-MS: $[M+H]^+$=440.

**Example 3. Synthesis Procedures for 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amino)benzyl (4-nitrophenyl) carbonate (CB07)**

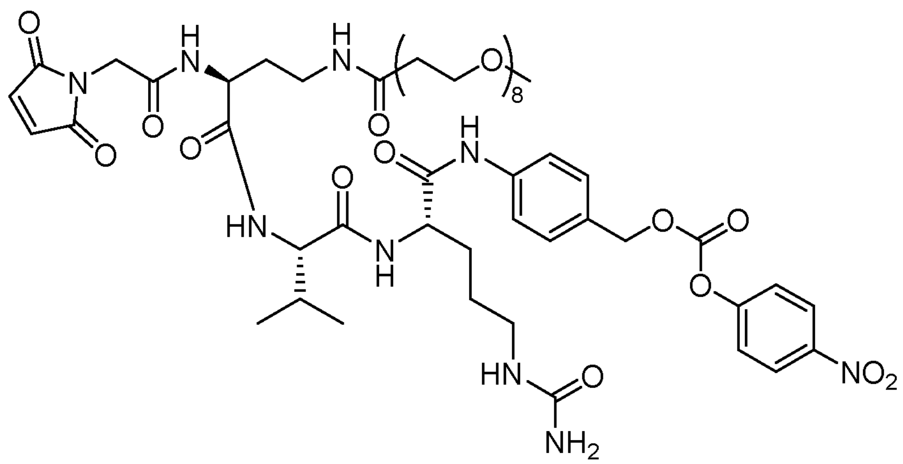

1) Synthesis of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01).

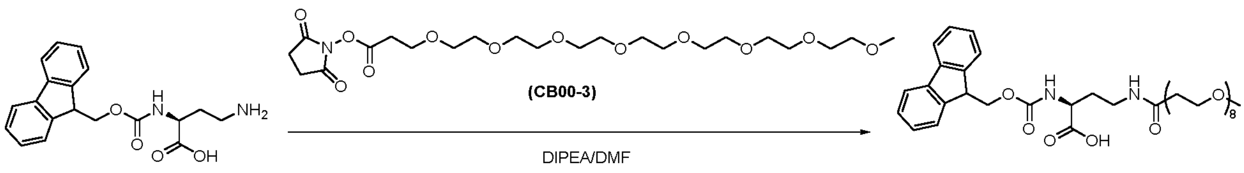


(CB00-2) (CB01)

Under a nitrogen atmosphere at 0-5 °C, 8.14 g of N2-fluorenylmethoxycarbonyl-L-2,4-diaminobutyric acid (CB00-2) was dissolved in 40 mL of dimethylformamide (DMF), and 10 g of*N*-succinimidyl 4,7,10,13,16,19,22,25-octaoxahexacosanoate (CB00-3) and 10 mL of DMF were added. 6.5 mL of DIPEA was dropwise added with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol volume ratio 20:1 as elution solvents) to obtain CB01 in the form of a pale yellow oil (14.2 g).

2) Synthesis of (9*H*-fluoren-9-yl)methyl [(*S*)-1-[[(*S*)-1-[[4-(hydroxymethyl)phenyl] amino]-1-oxo-5-ureidopentan-2-yl] amino]-3-methyl-1-oxobutan-2-yl]carbamate (CB02).

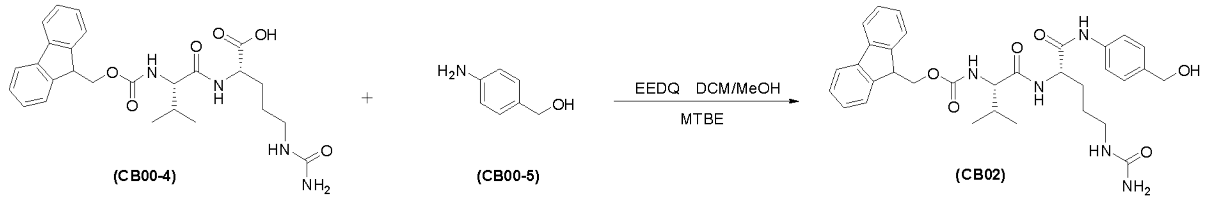

(CB00-4) (CB00-5) (CB02)

11 g of (*S*)-2-((*S*)-2-((((9*H*-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol at room temperature under a nitrogen atmosphere, and 17 g of 2-ethoxy-1-ethoxycarbonyl-1,2-dihydroquinoline (EEDQ) were added in portions with mechanical stirring. The resulting mixture was allowed to react for 15 h in the absence of light. After the completion of the reaction, the solvent was removed under reduced pressure to obtain a paste solid, which was subjected to silica gel column chromatography (with dichloromethane and methanol volume ratio 20:1 as elution solvents) to obtain an off-white solid (11.9 g).

3) Synthesis of (*S*)-2-((*S*)-2-amino-3-methylbutanamido)-*N*-(4-(hydroxymethyl)phenyl)-5-ureidopentanamide (CB03)

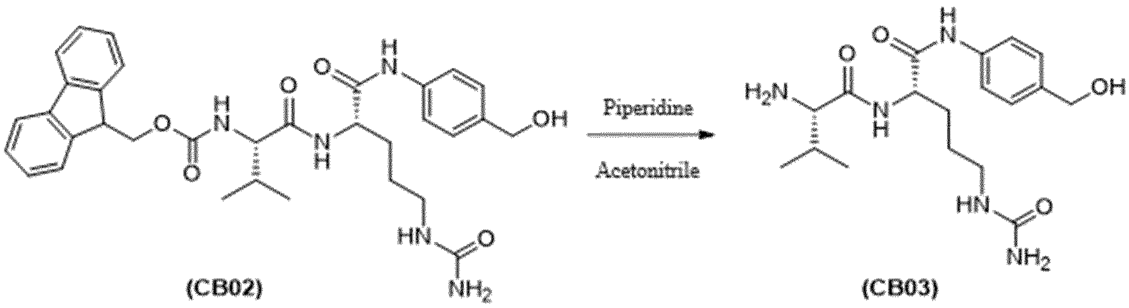

(CB02) (CB03)

Under a nitrogen atmosphere at room temperature, 300 mL of acetonitrile was added to 11.9 g of (9H-fluoren-9-yl)methyl [(S)-1-[[(S)-1-[[4-(hydroxymethyl)phenyl]amino]-1-oxo-5-ureidopentan-2-yl]amino]-3-methyl-1-oxobutan-2-yl] carbamate (CB02), and 18 mL of piperidine was added dropwise with stirring. After the dropwise addition, the mixture was allowed to react at room temperature for 2 h. After the completion of the reaction, the mixture was distilled under reduced pressure to remove the solvent and piperidine, and the residue was subjected to silica gel column chromatography (with dichloromethane and methanol volume ratio 20:1 as elution solvents) to obtain CB03 in the form of a white solid (7.5 g).

4) Synthesis of (9*H*-fluoren-9-yl)methyl ((30*S*,33*S*,36S)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04).

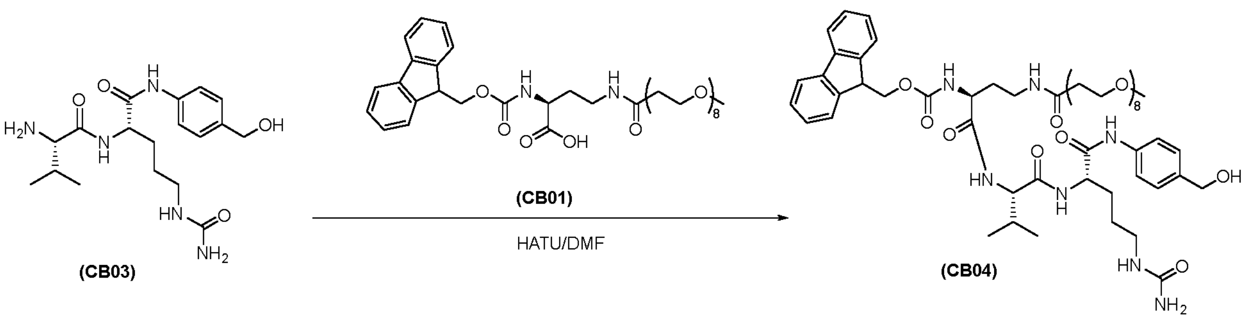

(CB03) (CB01) (CB04)

At 0 °C under a nitrogen atmosphere, 14.2 g of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01) was dissolved in 100 mL of DMF, and 11 g of *N*,*N*,*N*',*N*'-tetramethyl-*O*-(7-azabenzotriazol-1-yl)urea hexafluorophosphate (HATU) was added in portions. After 30 min of stirring, 7.5 g of (*S*)-2-((*S*)-2-amino-3-methylbutanamido)-*N*-(4-(hydroxymethyl)phenyl)-5-ureidopentanamide

(CB03) was added, and the mixture was allowed to react for 2.5 h with 0 °C maintained. After the completion of the reaction, the mixture was distilled under reduced pressure to remove the solvent, and the residue was subjected to silica gel column chromatography (with dichloromethane and methanol volume ratio 10:1 as elution solvents) to obtain CB04 in the form of a solid (9.66 g).

5) Synthesis of *N*-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05).

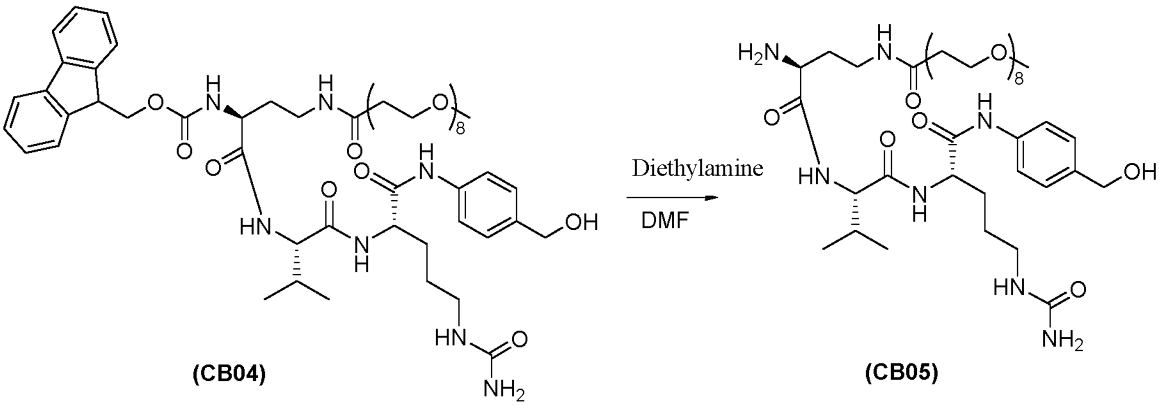


9.66 g of (9*H*-fluoren-9-yl)methyl ((30*S*,33*S*,36S)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04) was dissolved in 50 mL of DMF at room temperature under a nitrogen atmosphere, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol volume ratio 7.5:1 as elution solvents) to obtain CB05 in the form of a pale yellow solid (7.7 g).

6) Synthesis of *N*-((*S*)-3-(2-(2,5-dioxo-2,5-dihydro-1*H*-pyrrol-1-yl)acetamido)-4-(((*S*)-1-(((S)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

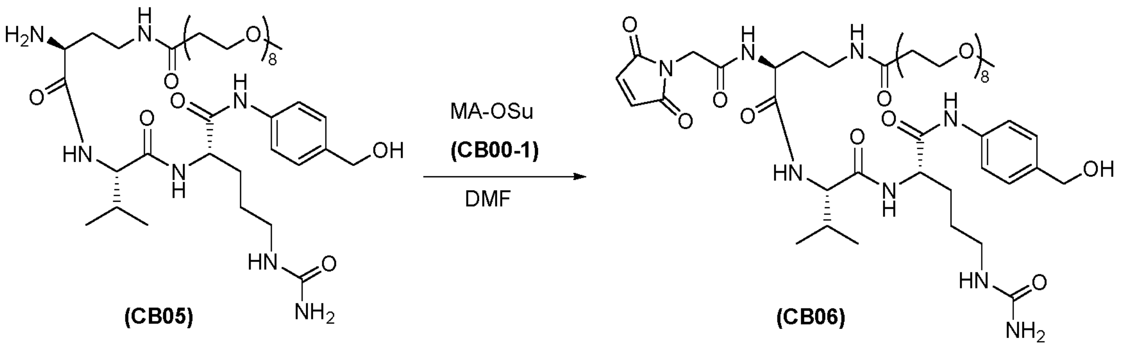


7.7 g of N-((*S*)-3-amino-4-(((S)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5°C. The mixture was allowed to react at 0-5°C for 4 h. After the completion of the reaction, the mixture was distilled under reduced pressure to remove the solvent, and the residue was subjected to silica gel column chromatography (with dichloromethane and methanol as volume ratio 10:1 elution solvents) to obtain CB06 in the form of a pale yellow solid (9.5 g).

7) Synthesis of 4-((30*S*,33*S*,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amino)benzyl (4-nitrophenyl) carbonate (CB07).

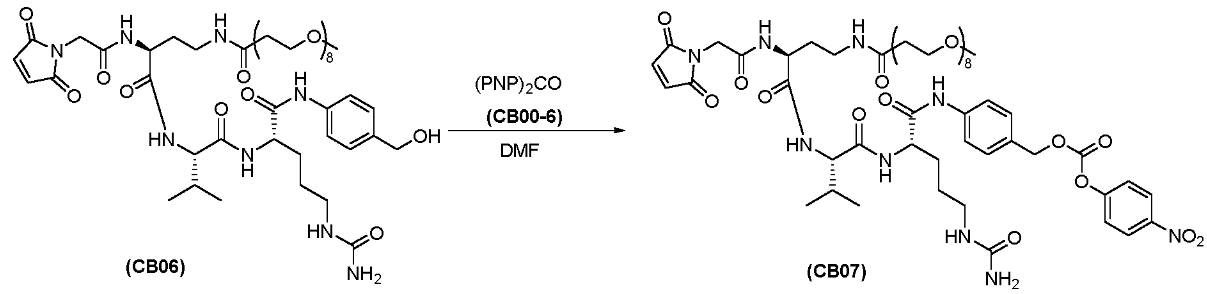


(CB06) (CB07)

9.5 g of *N*-((*S*)-3-(2-(2,5-dioxo-2,5-dihydro-1*H*-pyrrol-1-yl)acetamido)-4-(((*S*)-1-(((S)-1-((4-(hydroxymethyl) phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(p-nitrophenyl)carbonate ((PNP)$_2$CO) was added under a nitrogen atmosphere at 0 °C, followed by 8.2 mL of *N*,*N*-diisopropylethylamine (DIPEA) after dissolution. The mixture was allowed to react for 4 h with the 0 °C maintained. After the completion of the reaction, the mixture was distilled under reduced pressure to remove the solvent, and the residue was subjected to silica gel column chromatography (with dichloromethane and methanol volume ratio 8:1 as elution solvents) to obtain CB07 in the form of a white solid (2.6 g).

**Example 4. Synthesis of 4-((18*S*,21S,24S)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (CB14).**

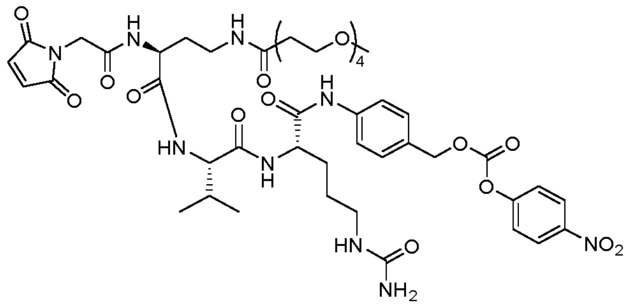

(CB14)

CB14 was prepared by referring to the synthesis of CB07 in Example 3, with *N*-succinimidyl 4,7,10,13,16,19,22,25-octaoxahexacosanoate replaced by *N*-succinimidyl 4,7,10,13-tetraoxatetradecanoate. CB14 was eventually obtained in the form of a white solid.

**Example 5. Synthesis of Intermediate (CB07-Exatecan)**

Synthesis of 4-((30*S*,33*S*,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino [1,2-*b*]quinolin-1-yl)carbamate.

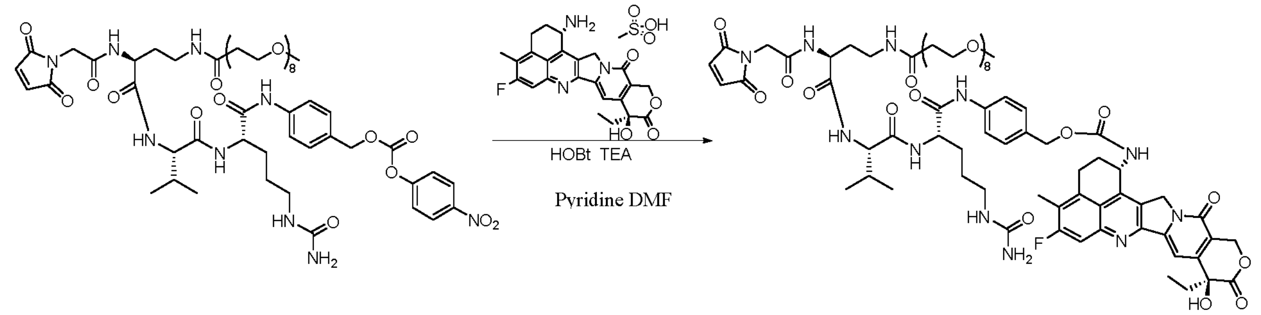


4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (2.6 g, 2.21 mmol) and N,N-dimethylformamide (23 mL) were added to a reaction flask R1, stirred under a nitrogen atmosphere and cooled to 0-5 °C. At the same time, (1S,9S)-1-amino-9-ethyl-5-fluoro-9-hydroxy-4-methyl-1,2,3,9,12,15-hexahydro-10H,13H-benzo[3',4':6,7]indolizino[1,2-b]quinolin-10,13-dione methanesulfonate (Exatecan, 0.98 g, 1.84 mmol, Advanced ChemBlocks) and N,N-dimethylformamide (5 mL) were taken and added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with N,N-dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5°C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder (1.6 g, 59%). LC-MS: $[1/2M+H]^+$=737.

**Example 6. Synthesis of Intermediate (CB07-D-1)**

Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1S,9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)carbamat.

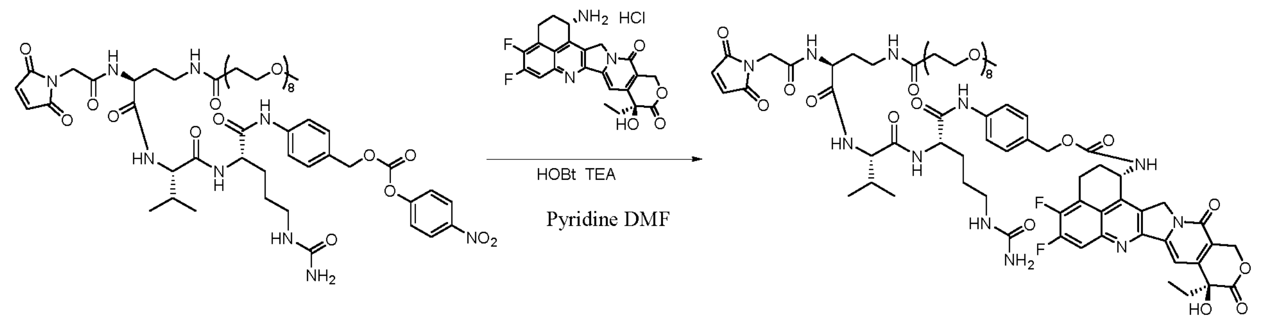


4-((30*S*,33*S*,36S)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl(4-nitrocarbonate) (CB07) (220 mg, 0.189 mmol) and *N*,*N*-dimethylformamide (5 mL) were added to a reaction flask R1, stirred under a nitrogen atmosphere and cooled to 0-5 °C. At the same time, (1*S*,9*S*)-1-amino-9-ethyl-4,5-difluoro-9-hydroxy-1,2,3,9,12,15-hexahydro-10*H*,13*H*-benzo[de]pyrano[3',4':6,7]indolizino[1,2-b]quinolin-10,13-dione hydrochloride (D-1) (90 mg, 0.189 mmol) and N,N-dimethylformamide (5 mL) were taken and added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5°C for 10 min, warmed to room temperature, and stirred for 3 h. After the reaction was completed, the mixture was concentrated under reduced pressure to remove the solvent. The residue was purified by preparative high performance liquid chromatography (prep-HPLC) and lyophilized to obtain a white powder (55 mg, 20%). LC-MS: $[1/2M+H]^+$=739.

**Example 7. Synthesis of Intermediate (CB14-Exatecan)**

Synthesis of 4-((18*S*,21*S*,24*S*)-18-(2-(2,5-dioxane-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)-(1*S*,9*S*)-9-ethyl-5-fluoro-9-hydroxy-4-methyl-10,13-dioxo-2,3,9,10,13,15-hexahydro-1*H*,12*H*-benzo[*de*]pyran[3',4':6,7]indolizino[1,2-*b*]quinolin-1-carbamate.

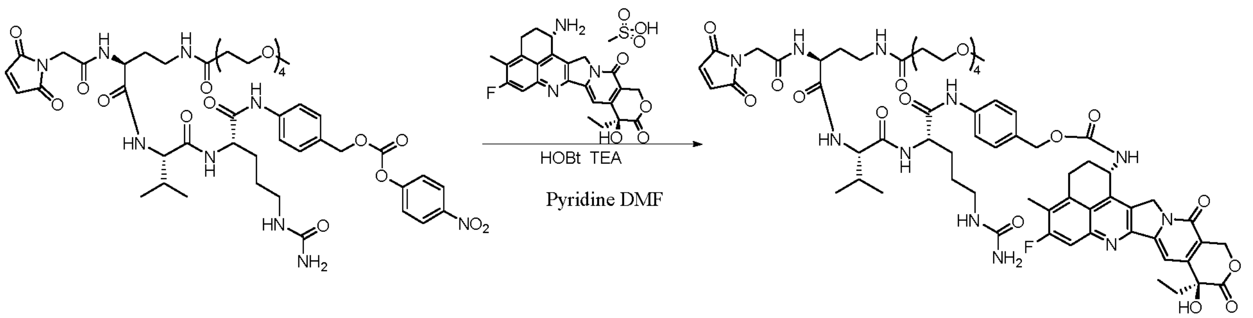


Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxo-15,20,23-triazapentacosan-25-amino)benzyl(4-nitrophenyl)carbonate (190 mg, 0.19 mmol)and *N*,*N*-dimethylformamide (23 mL) were added to a reaction flask R1, stirred under a nitrogen atmosphere, and cooled to 0-5 °C. At the same time, (1*S*,9*S*)-1-amino-9-ethyl-5-fluoro-9-hydroxy-4-methyl-1,2,3,9,12,15-hexahydro-10*H*,13*H*-benzo[3',4':6,7]indolizino[1,2-*b*]quinolin-10,13-dione methanesulfonate (Exatecan methanesulfonate; 101 mg, 0.19 mmol; Advanced ChemBlocks) and *N*,*N*-dimethylformamide (5 mL) were taken and added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N*,*N*-dimethylformamide (1 mL), and the washing solution was added to reaction flask R1. 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the reaction was completed, the mixture was concentrated at 35 °C under reduced pressure to remove the solvent. The residue was purified by preparative high performance liquid chromatography (prep-HPLC) and lyophilized to obtain a white powder.

**Example 8: Synthesis of ADC1**

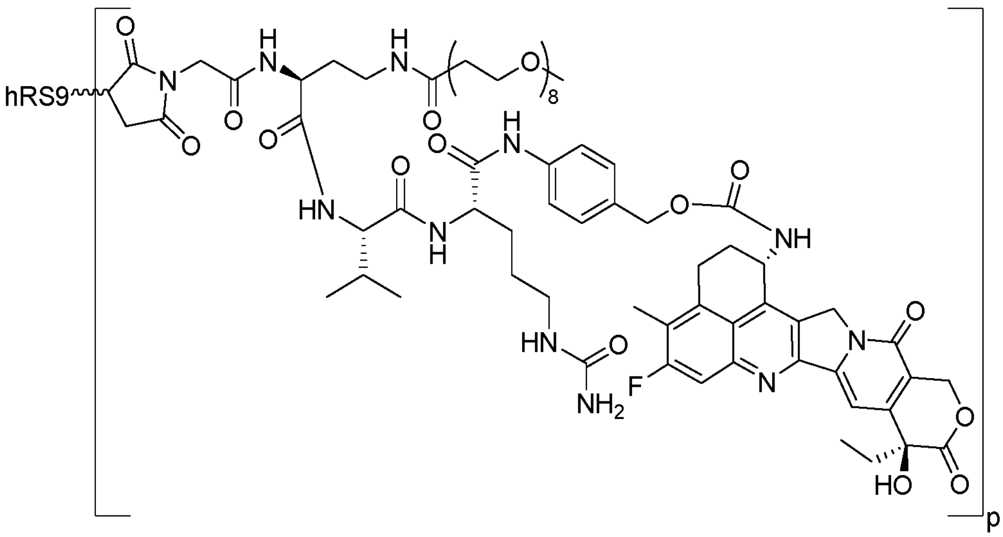

To hRS9 antibody was added 2.7 molar equivalents of TCEP according to the amount of the antibody substance, and then the system was adjusted to pH 7 with 1 M Tris base. The system was incubated at 25 °C for 2 h for reduction. TCEP

was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 6 molar equivalents of CB07-Exatecan was added according to the amount of the antibody substance, and after stirring at 25 °C for 2 h, 0.1 M acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

After measurement, the protein concentration of ADC1 was 8.46 mg/mL, and the DAR, i.e., p, was 4.2 as measured by RP-HPLC.

**Example 9: Synthesis of ADC2**

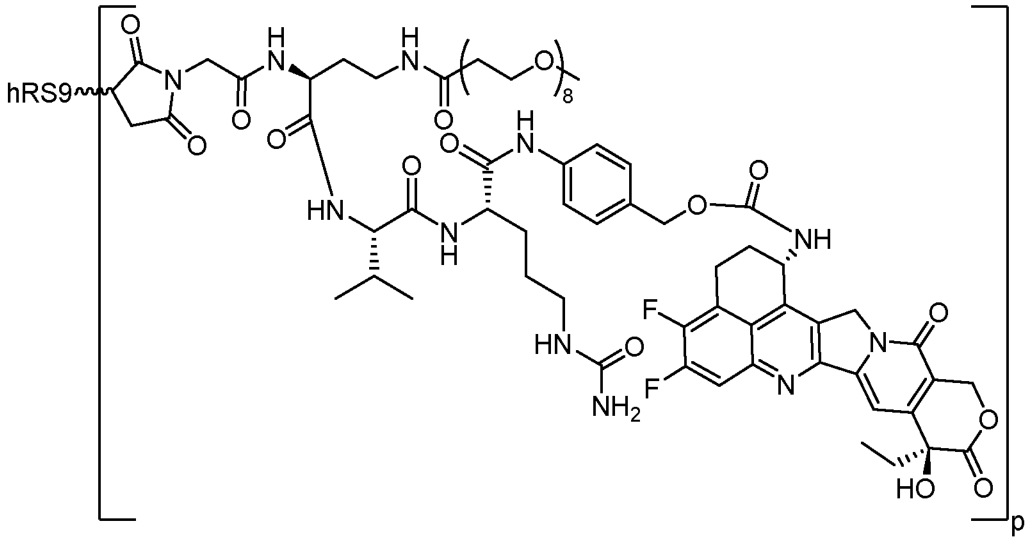

To hRS9 antibody was added 2.7 molar equivalents of TCEP according to the amount of the antibody substance, and then the system was adjusted to pH 7 with 1 M Tris base. The system was incubated at 25 °C for 2 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 6 molar equivalents of CB07-D-1 was added according to the amount of the antibody substance, and after stirring at 25 °C for 2 h, 0.1 M acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

After measurement, the protein concentration of ADC2 was 6.5 mg/mL, and the DAR, i.e., p, was 4.8 as measured by RP-HPLC.

**Example 10: Synthesis of ADC3**

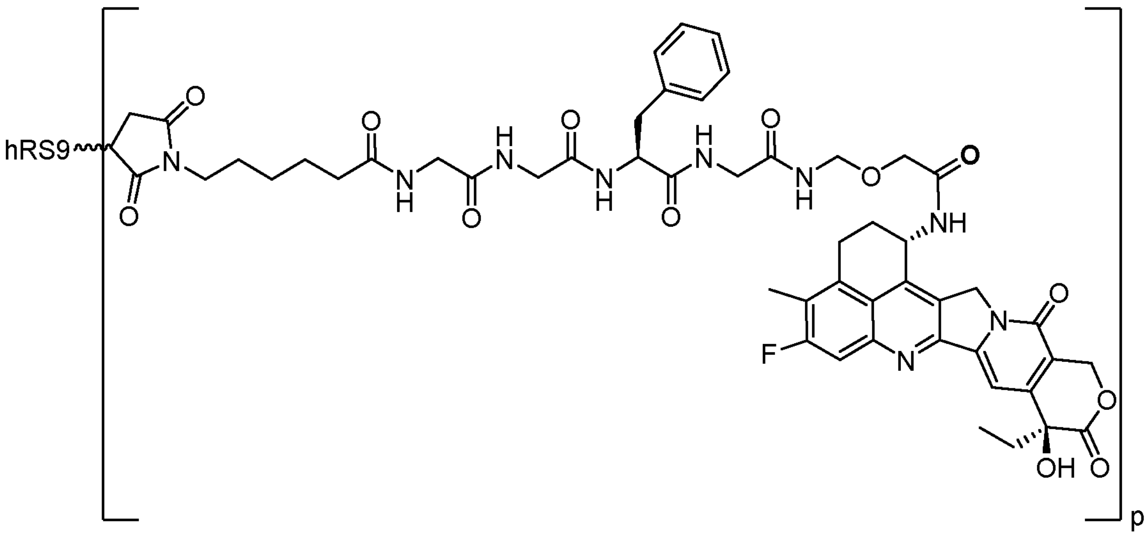

To hRS9 antibody was added 2.7 molar equivalents of TCEP according to the amount of the antibody substance, and then the system was adjusted to pH 7 with 1 M Tris base. The system was incubated at 25 °C for 2 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 6 molar equivalents of MC-GGFG-Dxd (Deruxtecan, MCE, Cat.#HY-13631E/CS-0045125) was added according to the amount of the antibody substance, and after stirring at 25 °C for 2 h, 0.1 M acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

After measurement, the protein concentration of ADC3 was 2.83 mg/mL, and the DAR, i.e., p, was 4.6 as measured by RP-HPLC.

**Example 11: Synthesis of ADC4**

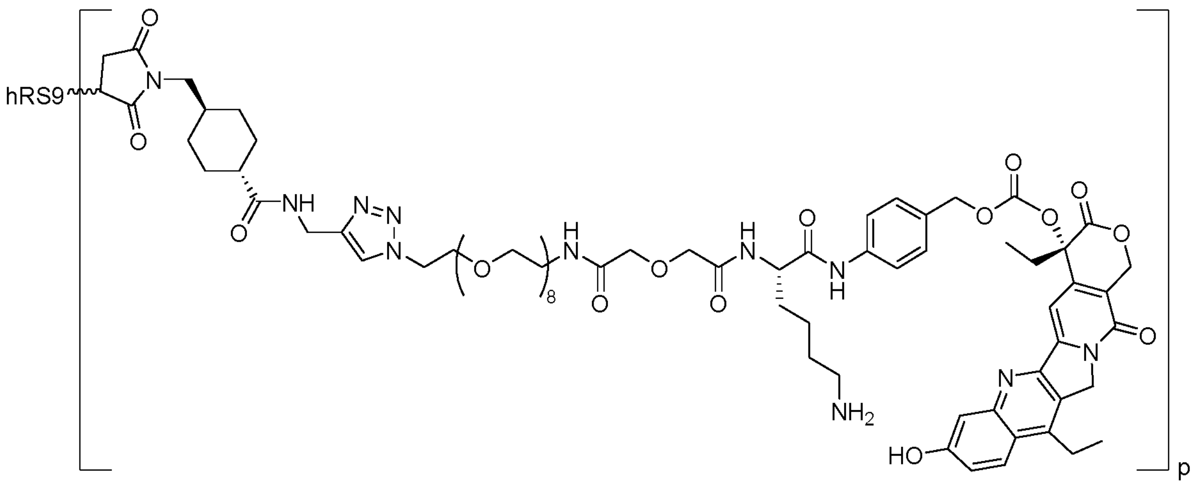

To hRS9 antibody was added 4.5 molar equivalents of TCEP according to the amount of the antibody substance, and then the system was adjusted to pH 8 with 1 M Tris base. The system was incubated at 25 °C for 2 h for reduction. TCEP was removed by ultrafiltration for buffer exchange using 10 mM succinic acid. The sulfhydryl antibody value was determined by absorbance measurement, and the sulfhydryl concentration was determined by reacting sulfhydryl with DTNB (5,5'-dithiobis(2-nitrobenzoic acid), Aldrich) and then measuring the absorbance at 412 nm.

In the conjugation reaction, 12 molar equivalents of CL2A-SN38 (see patent US2018161440A1 for details) was

added according to the amount of the antibody substance, and after stirring at 25 °C for 2 h, 0.1 M acetylcysteine was added until a final concentration of 2 mM was reached. The mixture was stirred for another 15 min, and the reaction was terminated. The reaction mixture was filtered through a 0.22 micron filter and eluted through Sephadex G-25 resin with 10 mM succinic acid for exchange.

After measurement, the protein concentration of ADC4 was 6.79 mg/mL, and the DAR, i.e., p, was about 8.3 as measured by RP-HPLC.

### Example 12: *In Vitro Bioactivity* of ADC1

The inhibition of the growth of tumor cells by ADC1 antibody was assessed using Trop2 positive breast tumor cell lines MDA-MB-453, MDA-MB-468 and MX-1, as well as Trop2 negative cell line HGC27 (purchased from Cell Bank of Chinese Academy of Sciences, Shanghai Institutes for Biological Sciences). Briefly, the cells were dissociated by digestion with trypsin after growing in the logarithmic phase and then suspended in 100 μL of complete medium. 4000-8000 cells were seeded in a 96-well plate for culture at 37 °C for 3-5 h or overnight, adhering to the walls. Then 100 μL of media with different concentrations of ADC1 were added. After 120 h, the media in the Petri dish was removed, and relative cell proliferation analysis was performed using cell counting kit-8 (CCK-8, Dojindo, Japan) reagent. The results show that ADC1 has a growth inhibition effect on all of the Trop2 positive cells, and the growth inhibition effect $EC_{50}$ on the negative cells HGC27 > 15000 ng/mL.

**Table 2**

| Cell line | $EC_{50}$ (ng/mL) ADC1 |
|---|---|
| MX-1 | 233 |
| MDA-MB-468 | 157.8 |
| MDA-MB-453 | 640 |
| HGC27 | > 15000 |

### Example 13: Bystander Effects of ADC1 and ADC2

The Trop2 positive cells MDA-MB-468 and Trop2 negative cells HGC27 were seeded into a 6-well plate in a density ratio of 3:1 (150 thousand + 50 thousand). After 3-5 h of culture, ADC1, ADC2 and the control drug ADC3 were added at final concentrations of 0.1 nM, 5 nM and 50 nM, respectively. After 120 h of culture at 37 °C, the cells were digested with pancreatin and washed once with PBS after termination. The cells were counted, and then labeled with an FITC-labeled anti-TROP2 antibody at 4 °C for 30 min to 1 h. The proportions of different cells were measured using a flow cytometer, and the cell proportions in the Petri dish after different treatments were calculated. As can be seen from FIG. 1, bystander effects were seen with ADC1, ADC2 and ADC3, among which ADC1 showed stronger bystander effects than ADC2 and the control drug ADC3.

### Example 14: *In Vivo* Pharmacokinetics Testing in Rats

A 6-8 week-old healthy adult Sprague Dawley rat was subjected to intravenous infusion of ADC1 at the tail for about 1 min ± 10 s. The volume for administration was 5 mL/kg, and the concentration for administration was 20 mg/kg. Blood was collected at 0.083 h, 1 h, 2 h, 8 h, 24 h, 48 h, 72 h, 96 h, 120 h, 144 h and 168 h after the administration was finished, and the serum was centrifugally isolated within 30-120 min. The concentrations of total antibodies (Tab) and ADC in the blood samples were measured by conventional ELISA.

The measurement method for total antibodies was briefly described as follows. Coating with Trop2-His was performed at 4 °C overnight at a concentration of 0.75 μg/mL. Blocking with 5% skim milk powder was performed at 37 °C for 2 h. A standard curve and quality control point samples were added for incubation for 2 h, with the measuring range for the standard curve being 64 ng/mL to 0.5 ng/mL. Two-fold serial dilution was performed from 64 ng/mL. The quality control concentration points were set to 60 ng/mL, 6 ng/mL and 0.6 ng/mL. The recovery rate for the quality control point should be in 80%-120%. Then 1:8000 diluted anti-human κ light chain peroxidase secondary antibody (Sigma, Cat.# A7164-1ML) produced in goats was added for incubation for 1 h. After 8 washings with PBST, TMB was added for color development, and the reaction was terminated with 0.1 M sulfuric acid. The plate was read using an OD450 microplate reader, and the blood sample concentration was calculated at different time points using the analysis software of the microplate reader, SoftMax Pro.

The measurement method for ADC was briefly described as follows. Coating with Trop2-His was performed at 4 °C overnight at a concentration of 0.75 μg/mL. Blocking with 5% skim milk powder was performed at 37 °C for 2 h. A standard curve and quality control point samples were added for incubation for 2 h, with the measuring range for the standard curve being 64 ng/mL to 0.5 ng/mL. Two-fold serial dilution was performed from 64 ng/mL. The quality control concentration points were set to 60 ng/mL, 6 ng/mL and 0.6 ng/mL. The recovery rate for the quality control point should be in 80%-120%. Then a secondary antibody (obtained by Genscript immunization) of a rabbit polyclonal antibody small molecule (1:5000 dilution) was added for incubation for 1 h. Fc fragment-specific peroxidase affinipure goat anti-rabbit IgG (Jackson immunono research, 111-035-008) (1:8000 dilution) was added for indubation for 1 h. After 8 washings with PBST, 100 μL of single-component TMB solution (InnoReagents, TMB-S-001) was added for color development, and the reaction was terminated with 0.1 M sulfuric acid. The plate was read using an OD450 microplate reader, and the blood sample concentration was calculated at different time points using the analysis software of the microplate reader, SoftMax Pro. A curve was plotted by ELISA for the change in the drug concentration in the blood, as shown in FIG. 2. The ADC1 concentration and the total antibody concentration substantially coincide with low detachment and are stable in the blood.

**Example 15: *In Vivo* Efficacy of ADC1 in Capan-1**

In this experiment, pharmacodynamic studies of ADC1 in a subcutaneous xenograft female BALB/c nude mouse animal model of human pancreatic cancer Capan-1 cell line were assessed. There were 10 mice in each group. The test protocol was designed as follows, with ADC1 as a test drug and ADC3 and ADC4 as control drugs.

**Table 3. Administration route, dosage and regimen in the subcutaneous xenograft model of human pancreatic cancer Capan-1**

| Group | Number of animals | Administration group | Dosage (mg/kg) | Administration route | Administration period |
|---|---|---|---|---|---|
| 1 | 10 | Vehicle control | -- | i.v. | QW×2 weeks |
| 2 | 10 | ADC3 | 5 | i.v. | Single administration |
| 3 | 10 | ADC4 | 5 | i.v. | QW×2 weeks |
| 4 | 10 | ADC1 | 5 | i.v. | Single administration |
| Note: 1. The day of grouping was defined as day 0 and the administration was started at day 0. | | | | | |

All drugs were diluted with PBS to a given concentration and administered. The test animals were 7-9 week-old BALB/c nude female mice (Beijing AniKeeper Biotech Co., Ltd.).

The Capan-1 cells were cultured in IMDM medium containing 20% fetal bovine serum. Capan-1 cells in the exponential phase were collected and resuspended in PBS to an appropriate concentration for subcutaneous tumor grafting in nude mice. The test mice were inoculated subcutaneously at the right anterior scapula with $5 \times 10^6$ Capan-1 cells, which were resuspended in PBS 1:1 mixed with matrigel (0.1 mL/mouse). The growth of the tumors was observed periodically. When the mean volume of the tumors reached 159.77 $mm^3$, the mice were randomly grouped for administration according to the tumor size and the mouse weight. The day of grouping was defined as day 0 and the administration was started at day 0.

After tumor grafting, routine monitoring included the effect of tumor growth and treatment on the normal behavior of the test animals, specifically the activity, food and water intake, weight gain or loss (the weight was measured twice a week), the eyes, the coat and other abnormal conditions. Clinical symptoms observed during the experiment were recorded in the raw data. The calculation formula for tumor volume: Tumor volume ($mm^3$) = $1/2 \times (a \times b^2)$ (where a represents long diameter and b represents short diameter). In the experiment, StudyDirector™ (version 3.1.399.19, Studylog System, Inc., S. San Francisco, CA, USA) software was employed to collect data.

**Table 4. Efficacy analysis for each group in the xenograft model of Capan-1**

| Experimental group | Mean tumor volume (day 0)[a] | Mean tumor volume (day 25)[a] | TGI (%)[b] | P value (relative to control group)[c] |
|---|---|---|---|---|
| Vehicle, 0 mg/kg, | 159.77±4.49 | 525.58±46.30 | - | - |
| ADC3, 5 mg/kg | 159.78±4.69 | 153.57±21.09 | 101.70 | 2.09e-06*** |
| ADC4, 5 mg/kg, | 159.75±4.31 | 601.83±118.59 | -20.85 | 1.00e+00[ns] |

(continued)

| Experimental group | Mean tumor volume (day 0)[a] | Mean tumor volume (day 25)[a] | TGI (%)[b] | P value (relative to control group)[c] |
|---|---|---|---|---|
| ADC1, 5 mg/kg | 159.77±4.34 | 107.08±18.05 | 114.40 | 4.09e-09*** |

Note: a. Data were expressed as "mean ± standard error";
b. TGI% = [1 - ($T_i$ - $T_0$)/($C_i$ - $C_0$)] × 100, where $T_0$ and $C_0$ are the mean tumor volumes of the administration group and vehicle control group at the day of grouping (day 0), respectively, and $T_i$ and $C_i$ are the mean tumor volumes of the administration group and vehicle control group at day 25, respectively;
c. *P < 0.05, **P < 0.01 and ***P < 0.001 compared to the tumor volume of the vehicle control group.

As shown in FIG. 3, at a dosage of 5 mg/kg, ADC1 has significantly greater efficacy than the control drug ADC4 and greater efficacy than ADC3; ADC4 has substantially no tumor growth inhibition effect on this model at a concentration of 5 mg/kg; the TGI of ADC 1 on tumors was up to 114% at day 25 after a single administration.

**Example 16: Pharmacodynamic Study of Drugs ADC1 and ADC3 in Subcutaneous Xenograft Female BALB/c Nude Mouse Animal Model of Human Triple-Negative Breast Cancer MX-1 Cell Line**

Test drugs: ADC1 (2.5 mg/kg and 5 mg/kg)
ADC3 (2.5 mg/kg and 5 mg/kg)

Preparation method: all were diluted with PBS

Test animals: BALB/c nude, 6 per group (Beijing AniKeeper Biotech Co., Ltd.).

Test method: The MX-1 cells were cultured in RPM1640 medium containing 10% fetal bovine serum. MX-1 in the exponential phase were collected and resuspended in PBS to an appropriate concentration for subcutaneous tumor grafting in nude mice. The test mice were inoculated subcutaneously on the right dorsum with 5 × $10^6$ MX-1 cells, which were resuspended in PBS (0.1 mL/mouse). The growth of the tumors was observed periodically. When the mean volume of the tumors reached 149.03 $mm^3$, the mice were randomly grouped for administration according to the tumor size and the mouse weight. The day of grouping was defined as day 0 and the administration was started at day 0.

After tumor grafting, routine monitoring included the effect of tumor growth and treatment on the normal behavior of the animals.

Test results: The tumor volume changed as shown in Table 5 and FIG. 4, and the test results show that ADC1 and ADC3 have similar efficacy after a single administration, and the TGIs are 109.48% and 108.84%, respectively, at an administration concentration of 5 mg/kg. There was no apparent abnormality or weight loss in the groups of mice during the experiment.

**Table 5**

| Experimental group | Mean tumor volume (day 0)[a] | Mean tumor volume (day 21)[a] | TGI (%)[b] | P value (relative to control group)[c] |
|---|---|---|---|---|
| Vehicle, 0 mg/kg | 149.15±10.37 | 1646.06±422.20 | - | - |
| ADC1, 2.5 mg/kg | 148.93±10.42 | 351.33±74.53 | 86.48 | 3.66e-03** |
| ADC1, 5 mg/kg | 149.09±10.58 | 7.14±1.82 | 109.48 | 9.65e-11*** |
| ADC3, 2.5 mg/kg | 149.08±10.20 | 279.24±84.24 | 91.30 | 7.72e-05*** |

(continued)

| Experimental group | Mean tumor volume (day 0)[a] | Mean tumor volume (day 21)[a] | TGI (%)[b] | P value (relative to control group)[c] |
|---|---|---|---|---|
| ADC3, 5 mg/kg, | 149.01±10.31 | 16.69±12.80 | 108.84 | 5.53e-11*** |

Note: a. Data were expressed as "mean ± standard error";
b. TGI% = [1 - ($T_i$ - $T_0$)/($C_i$ - $C_0$)] × 100, where $T_0$ and $C_0$ are the mean tumor volumes of the administration group and vehicle control group at the day of grouping (day 0), respectively, and $T_i$ and $C_i$ are the mean tumor volumes of the administration group and vehicle control group at day 21, respectively;
c. *P < 0.05, **P < 0.01 and ***P < 0.001 compared to the tumor volume of the vehicle control group.

**Example 17: Preclinical pharmacokinetic studies of ADC1**

In this study, 18 naive cynomolgus monkeys were used and randomized into 3 groups, half males and half females in each group. Animals in groups 1-3 were given ADC1 at doses of 1 mg/kg, 3 mg/kg, and 10 mg/kg, respectively, by a single intravenous infusion over a period of 30 min. Blood samples were collected until day 43 (1008 h) post-dose. The concentration of exatecan in the plasma samples was detected by LC-MS/MS in the liquid chromatography-mass spectrometry laboratory, and the ADC1 and total antibodies in the serum samples were detected by ELISA in the ligand-binding analysis laboratory.

After a single intravenous injection of ADC1 at 1 mg/kg, 3 mg/kg, or 10 mg/kg (dose ratio = 1:3:10) in cynomolgus monkeys, the inter-group ratios of $C_{max}$ and $AUC_{(0-t)}$ of total antibodies in the sera of male animals were 1:2:9 and 1:3:11, respectively, while those in the sera of female animals were 1:3:9 and 1:4:13, respectively. The inter-group ratios of Cmax and $AUC_{(0-t)}$ of the ADC in the sera of male animals were 1:2:8 and 1:2:11, respectively, while those in the sera of female animals were 1:2:10 and 1:3:13, respectively. The inter-group ratios of Cmax and $AUC_{(0-t)}$ of exatecan in the plasma of male animals were 1:2:6 and 1:70:768, respectively. The $AUC_{(0-t)}$ ratio of males to females was in the range of 0.85-1.48. The results show that after a single intravenous injection of ADC1 at 1 mg/kg, 3 mg/kg, or 10 mg/kg was given to cynomolgus monkeys, the total antibodies and the systemic exposure of the ADC in the serum increased along with the dose, and the increase rates were basically consistent with that of the dose.

The pharmacokinetic study results of ADC1 given to cynomolgus monkeys by a single intravenous infusion show no significant difference in exposure between male and female animals in each dose group.

**Example 18: Clinical study on ADC1 in human**

This study was a multicenter, open-label Phase I clinical study of the safety, tolerability, pharmacokinetics (PK), and preliminary efficacy of ADC1 for injection in patients with advanced solid tumors.

Patients were those with histopathologically or cytologically confirmed, standard treatment-intolerable or -refractory advanced or metastatic epidermis-derived solid tumors, including, but not limited to: urothelial carcinoma, triple-negative breast cancer, gastric adenocarcinoma, esophageal cancer, hepatocellular carcinoma, non-small cell lung cancer, small cell lung cancer, epithelial ovarian cancer, cervical cancer, follicular thyroid carcinoma, glioblastoma multiforme, endometrial cancer, prostate cancer, head and neck squamous cell carcinoma, and clear cell renal cell carcinoma.

The clinical study of ADC1 included two stages. The first stage (dose-escalation tolerability study) was intended to explore the safety and tolerability of study treatment using the accelerated titration rule or the "3 + 3" dose escalation rule in 7 dose groups: A (0.8 mg/kg), B (1.2 mg/kg), C (2.4 mg/kg), D (2.7 mg/kg), E (3.0 mg/kg), F (3.3 mg/kg), and G (3.6 mg/kg). Among these, the 0.8 mg/kg group adopted the accelerated titration method for dose escalation, and the rest dose groups adopted the standard "3 + 3" rule for dose escalation. In the second stage (cohort expansion study), according to the preliminary safety and efficacy results in the previous stage, appropriate doses and tumor types were selected for the expansion study to further explore the safety and clinical efficacy of ADC1 and provide a basis for subsequent clinical studies.

The "3 + 3" dose escalation rule is as follows:
The first dose group adopted the accelerated titration dose escalation scheme, i.e., the cohort size = 1. First, 1 subject was included in group A. If no treatment-related toxicity of grade ≥2 was observed within 21 days, the dose escalation could directly proceed to the next group. By analogy with this rule ("1 + 1"), the dose escalation was accelerated to the standard 3 + 3 group. During the accelerated dose escalation, if any treatment-related toxicity of grade ≥2 was observed, the dose escalation based on accelerated titration was discontinued, 2 additional subjects in the dose group were enrolled, and the study was conducted following the standard "3 + 3" dose escalation (i.e., cohort size = 3) rule until the MTD or MAD was reached. Starting from group B, the dose escalation pattern was adjusted to the standard "3 + 3" rule, as shown in the table

below:

| Subjects with DLT (dose-limiting toxicity)/subjects in current dose group | Dose escalation/de-escalation decision |
|---|---|
| 0/3 | 3 new subjects were enrolled in the next higher dose group |
| 1/3 | 3 new subjects were enrolled in the current dose group |
| 1/3 + 0/3 (i.e., 1/6) | 3 new subjects were enrolled in the next higher dose group |
| ≥ 2/3 or ≥ 2/6 | It is indicated that this dose exceeded the MTD (maximum tolerated dose), and dose escalation should be stopped. If 6 subjects were enrolled in the previous dose group, the previous dose should be used as the MTD. If only 3 subjects were enrolled in the previous dose group, 3 more subjects should be enrolled in the previous dose group. After 3 more subjects were enrolled, if there were a total of ≥2 DLTs in the previous dose group, the MTD should be de-escalated again. |

During the tolerability exploration according to the "3 + 3" dose escalation rule, all subjects in the previous dose group must complete a 21-day DLT observation before the enrollment for the next dose group (cohort). In the dose-escalation study, the time interval for the first dose between one subject and the next subject was at least 72 hours in each dose group.

After the completion of the above escalation study, whether a higher dose level group should be added could be considered as appropriate to further explore the tolerability according to factors such as safety, etc.; or a new dose between the dose where the escalation stopped and the previous lower dose group could be selected for investigation according to the above rules to determine a more precise maximum tolerated dose, depending on the particular circumstances and investigator's discussion.

AEs were graded according to National Cancer Institute Common Terminology Criteria for Adverse Events (NCI CTCAE), version 5.0. The DLT refers to any grade 3 or higher AE related to the study treatment that occurs within 21 days after a subject receives the first dose and meets the following definitions:

· Grade 5 toxicity.

· Definition of hepatotoxicity DLT:

Grade 4 elevation of AST (aspartate transaminase) or ALT (alanine transaminase); subjects with hepatocellular carcinoma or liver metastasis, e.g., AST or ALT ≤ 3-fold ULN (upper limit of normal) at baseline, AST or ALT elevation > 5-fold ULN in a DLT evaluation period and lasting > 5 days;

subjects with hepatocellular carcinoma or liver metastasis, e.g., AST or ALT > 3-fold ULN at baseline, AST or ALT elevation > 8-fold ULN in a DLT evaluation period and lasting > 5 days;

subjects without liver metastasis, AST or ALT elevation > 5-fold ULN and lasting > 5 days;

AST or ALT > 5-fold upper limit of normal (ULN), with ≥ Grade 2 blood bilirubin elevation.

· Definition of hematological toxicity DLT:

Grade 4 neutropenia lasting > 7 days;

≥ Grade 3 neutropenia with fever (single body temperature > 38.3 °C or sustained body temperature ≥ 38 °C for more than 1 hour);

Grade 4 anemia;

Grade 4 thrombocytopenia;

≥ Grade 3 thrombocytopenia lasting > 7 days;

≥ Grade 3 thrombocytopenia with bleeding.

Definition of non-hepatotoxicity and non-hematological toxicity DLT:
Other ≥ Grade 3 non-hepatotoxicity and non-hematological toxicity.

The following AEs were not considered DLTs:

Grade 3 nausea, vomiting, diarrhea, anorexia, or fatigue that is controlled by standard supportive treatment and relieved to ≤ Grade 2 within 3 days;

Grade 3 skin and mucosal toxicity can be controlled with standard treatment and relieved to ≤ Grade 2 within 7 days

abnormalities in laboratory examinations without clinically relevant symptoms or signs, including elevated ALP, elevated uric acid, elevated blood amylase, elevated lipase, or decreased lymphocyte to Grade 3 or 4, with Grade 1 hyponatremia at baseline escalating to Grade 3 but lasting < 72 hours;

Grade 3 infusion reaction that can be relieved within 6 hours after treatment.

Dosage regimen: intravenous infusion once every 2 weeks (Q2W) or once every 3 weeks (Q3W) on the first day of each cycle. The infusion was slowly conducted at the beginning. If the vital signs were stable and no infusion reaction was observed, the infusion rate could be gradually increased according to the following suggestions:

| Infusion rate | First dose | Subsequent dose |
|---|---|---|
| Initial speed (first 15 min) | ≤50 mg/hr | 100~200 mg/hr |
| Gradual rate increase (every 15 to 30 min) | 50 mg/hr | 100~200 mg/hr |
| Maximum rate | 500mg/hr | 1000 mg/hr |

When infusion reactions or abnormal vital signs were observed, the investigator could make a decision to slow down, interrupt, or discontinue the infusion of the study drug according to the specific clinical situations. It is recommended that the infusion duration should be controlled at ≥3 h for the first dose. If the first dose was well tolerated, the duration of subsequent doses could be controlled within 1 to 2 h. The vital signs and the occurrence of infusion reactions were closely monitored during the administration and within 30 min post-dose. After the infusion, the infusion pipeline should be slowly rinsed with normal saline.

**Evaluation of tolerability and safety:**

Tolerability evaluation indexes: dose-limiting toxicity (DLT) events and their incidence.
Safety evaluation indexes: Vital signs and physical examination, laboratory examinations (complete blood count, blood biochemistry, troponin, coagulation function, urinalysis, and pregnancy test), ECOG score, electrocardiogram, cardiac color ultrasound, and adverse events . The nature, frequency, severity, occurrence time, etc., of the adverse events were evaluated; the changes in clinical laboratory examination results before, during, and after the treatment were evaluated.

**Pharmacokinetic evaluation:**

All subjects in the dose groups were required to collect blood samples at specified time points during the treatment period (first 6 treatment cycles) to study pharmacokinetic characteristics. The drug serum concentrations of ADC1, the anti-Trop2 total antibody, and Exatecan were observed at the time points specified in the protocol, and planed to collect about 4.0mL of blood samples at each time point. For unexpected situations such as infusion reactions, additional PK blood samples should be collected.
The following PK (pharmacokinetic) parameters were calculated from the actual administration doses and actual sampling time by the non-compartmental model: $C_{max}$, $T_{max}$, $T_{1/2}$, CL, Vd, Ke, MRT, $AUC_{(0-\tau)}$, and $AUC_{(0-\infty)}$ at single administration; and $C_{max,ss}$, $C_{avg,ss}$, $C_{min,ss}$, $AUC_{(0-\tau),ss}$, $AUC_{(0-\infty),ss}$, $T_{max,ss}$, $T_{1/2,ss}$, CL, $V_{ss}$, Ke, MRT, accumulation index (Rac), and fluctuation index (DF) at multiple administration.

**Effectiveness evaluation indicators:**

The anti-tumor efficacy indicators include: objective response rate (ORR), duration of response (DOR), disease control rate (DCR), progression free survival (PFS), and overall survival (OS) based on RECIST(Response Evaluation Criteria in Solid Tumors) evaluation criteria and CT/MRI as the main evaluation method for imaging efficacy assessment.

## Claims

1. A method for treating a solid tumor, being **characterized in that** comprises administering to a patient with a solid tumor an effective amount of an antibody-drug conjugate, wherein, the effective amount of the antibody-drug conjugate is administered once every 1-8 weeks at 0.1-30 mg/kg per dose, the antibody-drug conjugate has a structure shown as Formula I, or a stereoisomer thereof, or a pharmaceutically acceptable salt or solvate thereof:

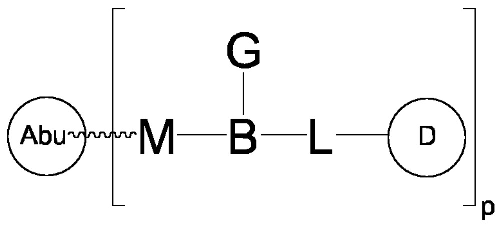

Formula I

wherein,

Abu is an antibody or an antigen-binding unit thereof that binds to TROP2;
D is drug,
M is

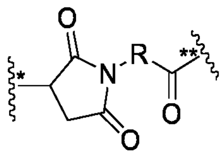

,

wherein * links to Abu, ** links to B, R is selected from: $-(CH_2)_r-$, - $(CHR^m)_r-$, C3-C8 carbocyclyl, $-O-(CH_2)_r-$, arylene, $-(CH_2)_r$-arylene-, -arylene-$(CH_2)$r-, $-(CH_2)_r$-(C3-C8carbocyclyl-)-, -(C3-C8 carbocyclyl)-$(CH_2)_r$-, C3-C8 carbocyclyl, $-(CH_2)_r$-(C3-C8 heterocyclyl)-, - (C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, - $(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_rC(O)NR^m(CH_2CH_2O)_r-CH_2-$ and - $(CH_2CH_2O)_rC(O)NR^m(CH_2)_r-$; wherein each $R^m$ is independently H, C1-C6 alkyl, C3-C8 carbocyclyl, phenyl or benzyl; and each r is independently 1, 2, 3, 4, 5, 6, 7, 8, 9 and 10;
B is

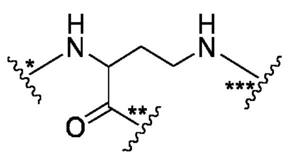

,

wherein * links to M, ** links to L, *** links to G;
L is $-(AA)_i-(FF)_f-$, wherein each AA is independently amino acid or polypeptide, i is 1, 2, 3, 4, 5, 6, 7, 8, 9, 10, 11, 12, 13, 14, 15, 16, 17, 18, 19 or 20; each FF is independently

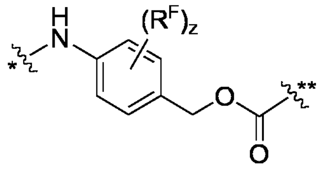

,

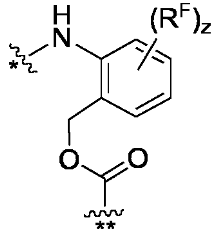

or

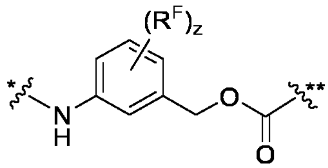

,

wherein each $R^F$ is independently C1-C6 alkyl, C1-C6 alkoxy, $-NO_2$ or halogen; z is 0, 1, 2, 3 or 4, wherein * links to AA, ** links to D; f is 1, 2, 3, 4, 5, 6, 7, 8, 9 or 10;
G is

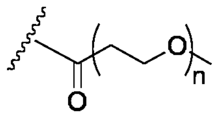

,

wherein n is an integer from 1-24;
p is 1-10.

2. The method according to claim 1, the characteristic is that, B is

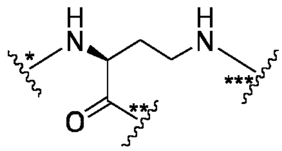

,

wherein * links to M, ** links to L, *** links to G.

3. The method according to claim 1 or 2, the characteristic is that, each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly.

4. The method according to claim 3, the characteristic is that, AA is Val-Cit.

5. The method according to any one of claims 1-4, the characteristic is that, i is 1.

6. The method according to any one of claims 1-5, the characteristic is that, each FF is independently

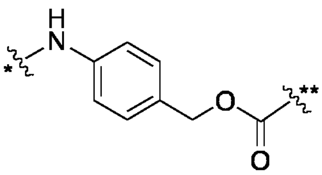

,

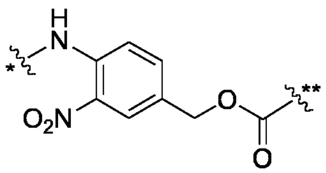

,

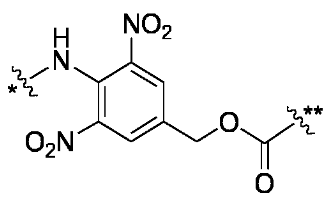

,

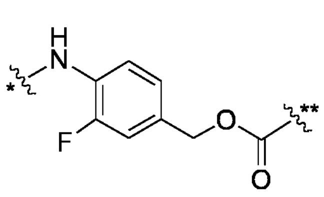, 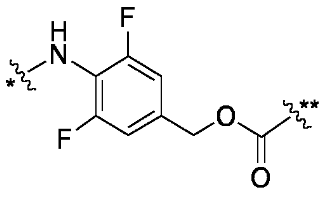, 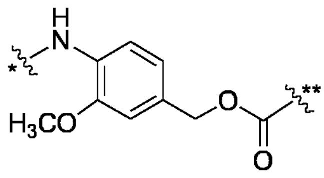,

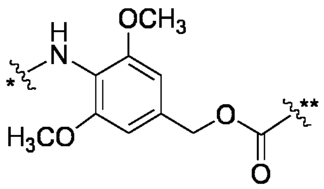, 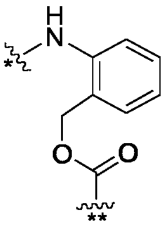, 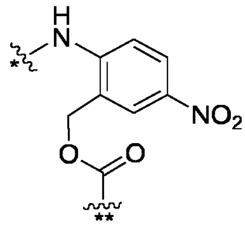, 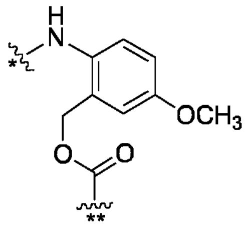,

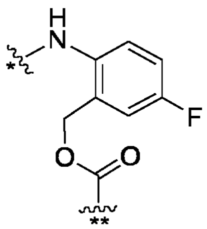, 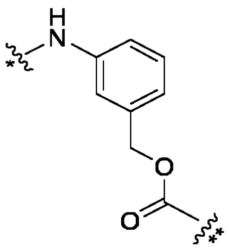, 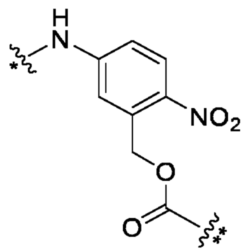, 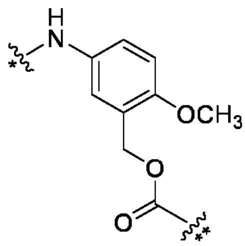, or

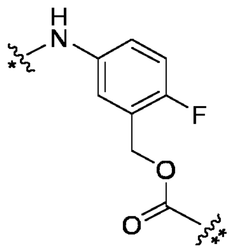,

wherein * links to AA, ** links to D.

**7.** The method according to claim 6, the characteristic is that, FF is

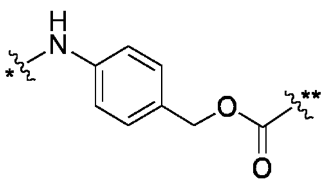,

wherein * links to AA, ** links to D.

**8.** The method according to any one of claims 1-7, the characteristic is that, f is 1.

**9.** The method according to claim 8, the characteristic is that, L is

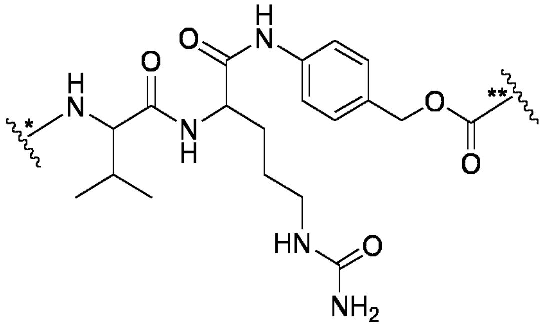

or

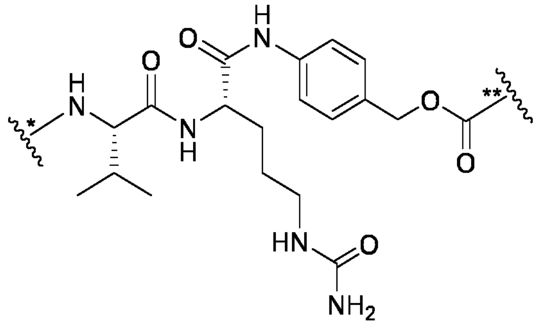

,

wherein * links to B, ** links to D

**10.** A method for treating a solid tumor, being **characterized in that** comprises administering to a patient with a solid tumor an effective amount of an antibody-drug conjugate, wherein, the effective amount of the antibody-drug conjugate is administered once every 1-8 weeks at 0.1-30 mg/kg per dose, the antibody-drug conjugate has a structure shown as Formula I-1, I-2, I-3, I-4, I-5, I-6, I-7, I-8, I-9, I-10, or I-11, or a stereoisomer thereof, or a pharmaceutically acceptable salt or solvate thereof, wherein

the Formula I-1 is:

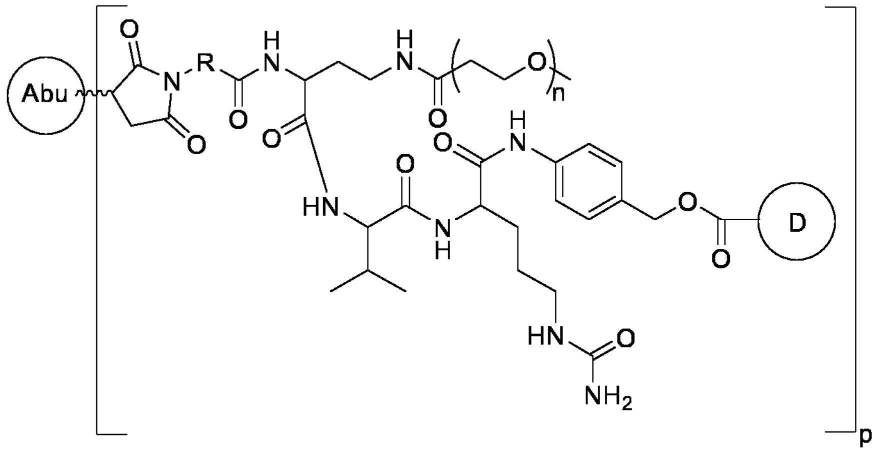

Formula I-1

the Formula I-2 is:

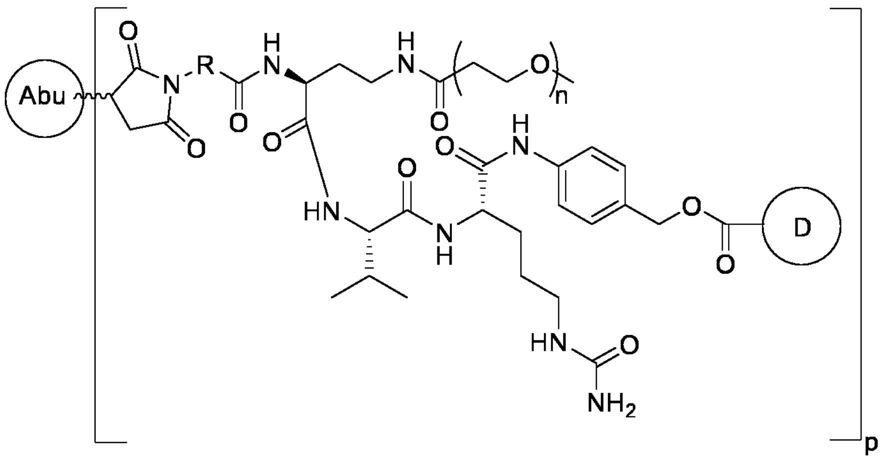

Formula I-2

the Formula I-3 is:

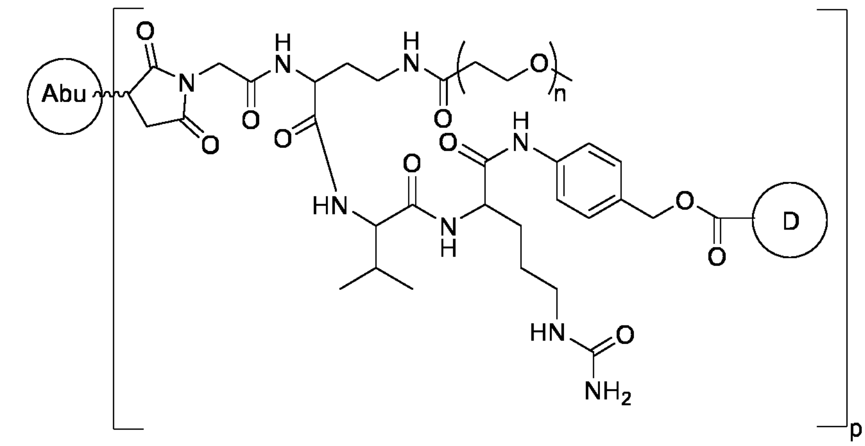

Formula I-3

the Formula I-4 is:

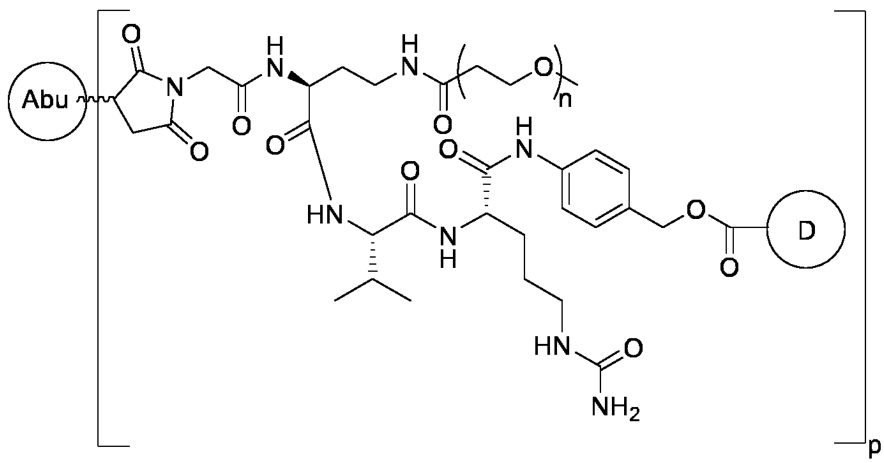

Formula I-4

the Formula I-5 is:

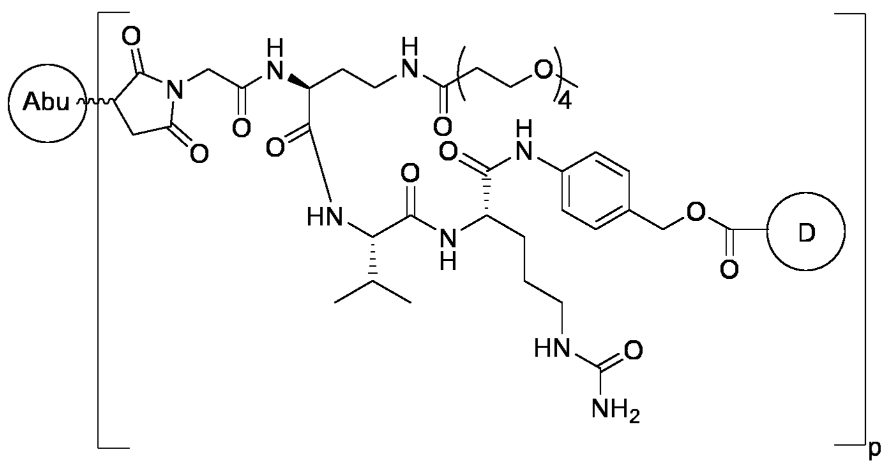

Formula I-5

the Formula I-6 is:

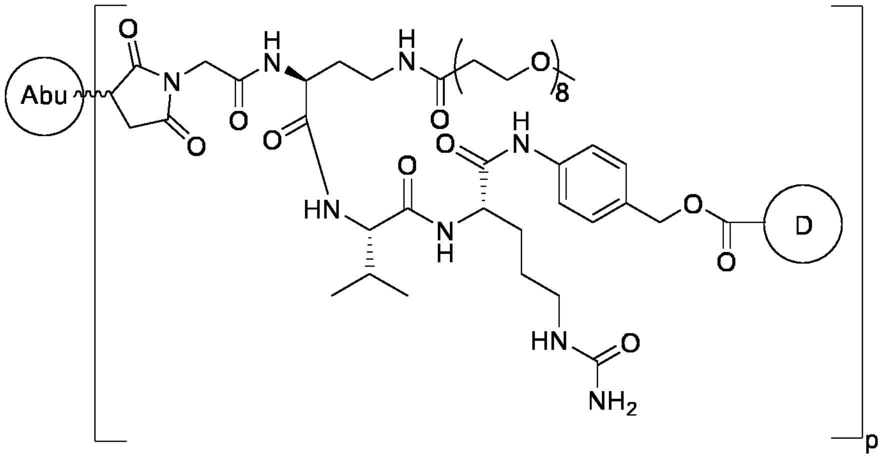

Formula I-6

the Formula I-7 is:

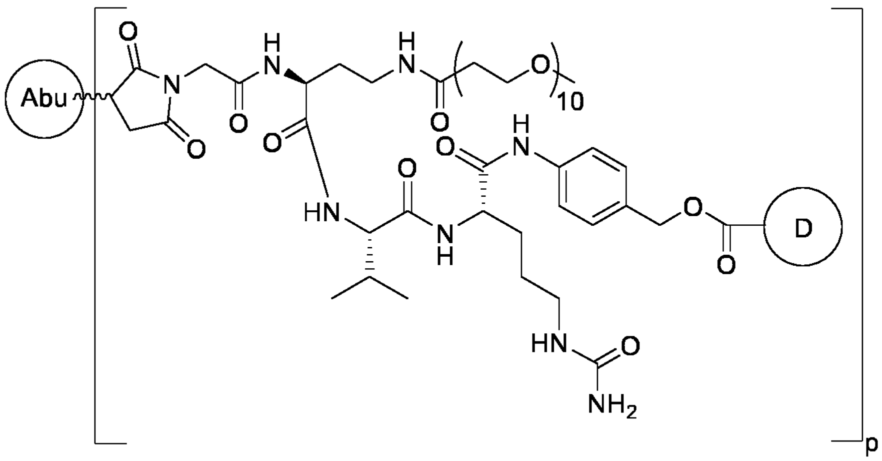

Formula I-7

the Formula I-8 is:

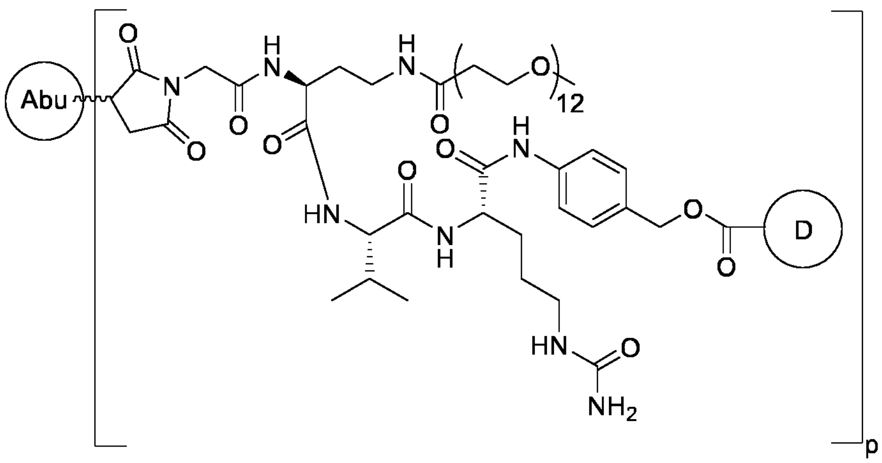

Formula I-8

the Formula I-9 is:

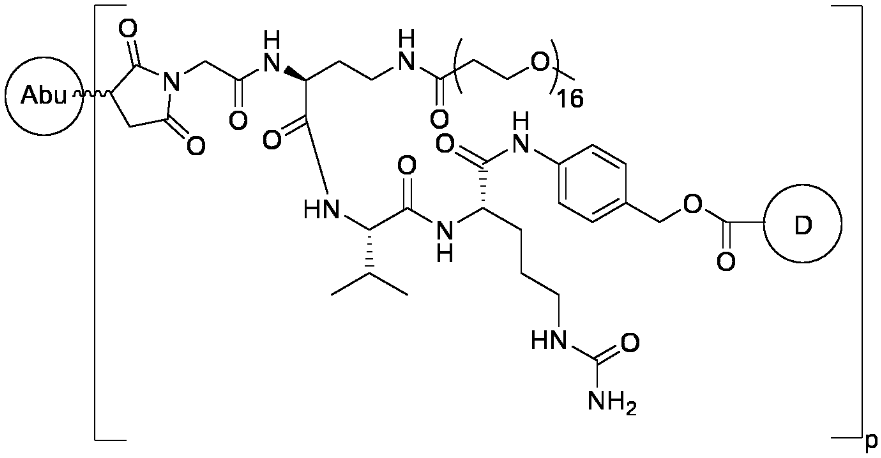

Formula I-9

the Formula I-10 is:

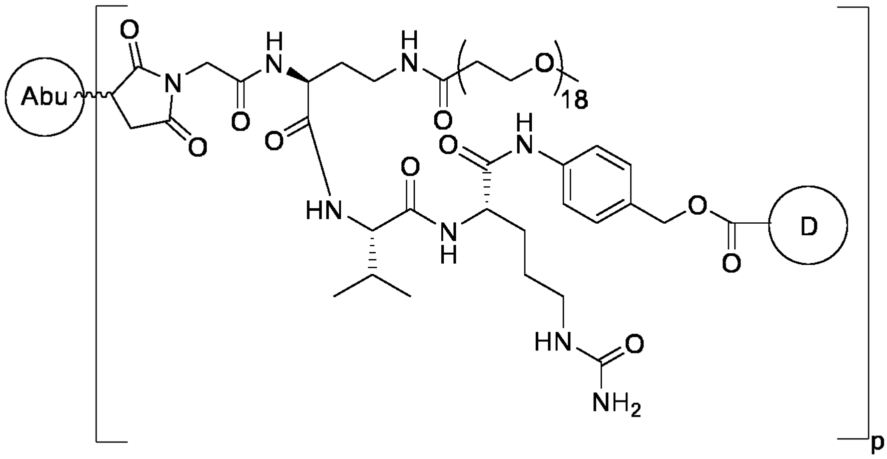

or

Formula I-10

the Formula I-11 is:

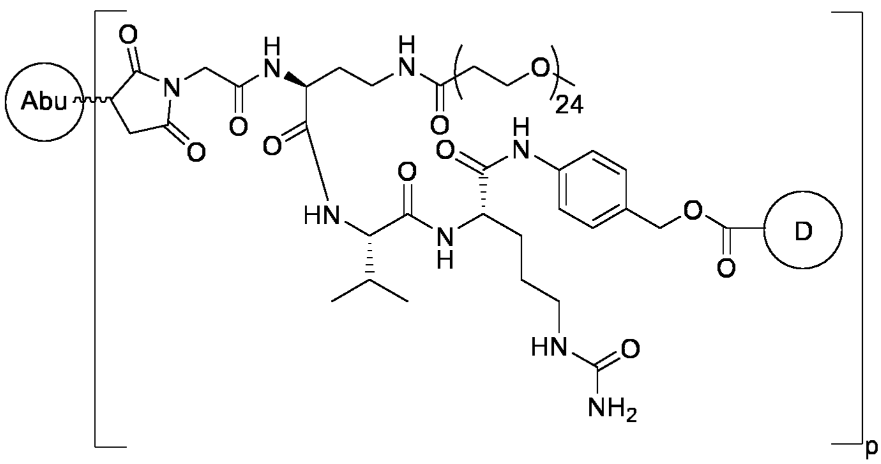

Formula I-11.

wherein

Abu is an antibody or an antigen-binding unit thereof that binds to TROP2;

R is selected from: $-(CH_2)_r-$, $-(CHR^m)_r-$, C3-C8 carbocyclyl, $-O-(CH_2)_r-$, arylene, $-(CH_2)_r$-arylene-, - arylene-$(CH_2)_r$-, $-(CH_2)_r$-(C3-C8 carbocyclyl)-, -(C3-C8 carbocyclyl)-$(CH_2)_r$-, C3-C8 heterocyclyl, - $(CH_2)_r$-(C3-C8 heterocyclyl)-, -(C3-C8 heterocyclyl)-$(CH_2)_r$-, $-(CH_2)_rC(O)NR^m(CH_2)_r-$, $-(CH_2CH_2O)_r-$, $-(CH_2CH_2O)_r-CH_2-$, $-(CH_2)_rC(O)NR^m(CH_2CH_2O)_r-$, $-(CH_2)_rC(O)NR^m(CH_2CH_2O)_r-CH_2-$, - $(CH_2CH_2O)_rC(O)NR^m(CH_2CH_2O)_r-$, $-(CH_2CH_2O)_r-C(O)NR^m(CH_2CH_2O)_r-CH_2-$ and - $(CH_2CH_2O)_rC(O)NR^m(CH_2)_r-$; wherein each $R^m$ is independently H, C1-C6 alkyl, C3-C8 carbocyclyl, phenyl or benzyl; and each r is independently 1, 2, 3, 4, 5, 6, 7, 8, 9 or 10;

D is drug;

n is an integer from 1-24;

p is 1-10.

**11.** The method according to any one of claims 1-10, the characteristic is that, .R is $-(CH_2)_r-$, or r is 1 or 5.

**12.** The method according to any one of claims 1-11, the characteristic is that, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases; or the drug is an anti-cancer drug; or the drug is a tubulin inhibitor, a DNA damaging agent, or a DNA topoisomerase inhibitor; or the tubulin inhibitor is selected from dolastatin, auristatins and maytansinoids; or the drug is an auristatin, selected from MMAE, MMAF, or AF; or the drug is a DNA damaging agent, selected from calicheamicin, duocarmycin, the anthramycin derivative PBD; or the drug is DNA topoisomerase inhibitor or a salt thereof, selected from irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, or *N*-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine, or exatecan; or a pharmaceutically acceptable salt or solvate thereof.

**13.** The method according to any one of claims 1-11, the characteristic is that, the drug is

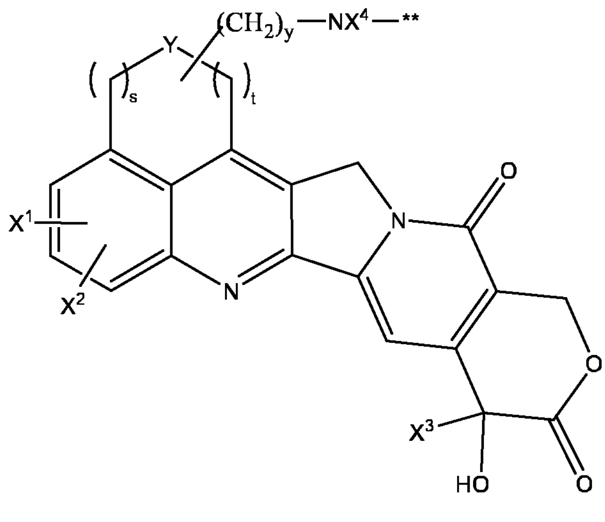

,

wherein

$X^1$ and $X^2$ are each independently:

H,

hydroxy,

C1-C6 alkyl,

C1-C6 alkyl substituted with one or more hydroxy, halogen, nitro or cyano groups,

C2-C6 alkenyl,

C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,
amino-substituted heterocyclyl, which is optionally substituted at a nitrogen atom of the heterocyclyl moiety or at the amino moiety with a protecting group or one or more C1-C6 alkyl groups,
heterocyclylamino, which is optionally substituted at a nitrogen atom of the heterocyclic moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
$X^3$ is C1-C6 alky;
$X^4$ is H, $-(CH_2)_q-CH_3$, $-(CHR^n)_q-CH_3$, C3-C8 carbocyclyl, $-O-(CH_2)_q-CH_3$, arylene-$CH_3$, $-(CH_2)_q$-arylene-$CH_3$, -arylene-$(CH_2)_q-CH_3$, $-(CH_2)_q$-(C3-C8 carbocyclyl)-$CH_3$, -(C3-C8 carbocyclyl)-$(CH_2)_q-CH_3$, C3-C8 heterocyclyl, $-(CH_2)_q$-(C3-C8 heterocyclyl)-$CH_3$, -(C3-C8 heterocyclyl)-$(CH_2)_q-CH_3$, - $(CH_2)_qC(O)NR^n(CH_2)_q-CH_3$, $-(CH_2CH_2O)_q-CH_3$, $-(CH_2CH_2O)_q-CH_2-CH_3$, - $(CH_2)_qC(O)NR^n(CH_2CH_2O)_q-CH_3$, $-(CH_2)_qC(O)NR^n(CH_2CH_2O)_q-CH_2-CH_3$, - $(CH_2CH_2O)_qC(O)NR^n(CH_2CH_2O)_q-CH_3$, $-(CH_2CH_2O)_qC(O)NR^n(CH_2CH_2O)_q-CH_2-CH_3$ or - $(CH_2CH_2O)_qC(O)NR^n(CH_2)_q-CH_3$; wherein each $R^n$ is independently H, C1-C6 alkyl, C3-C8 carbocyclyl, phenyl or benzyl, and each q is independently 1, 2, 3, 4, 5, 6, 7, 8, 9 or 10; or ,$X^4$ is H or C1-C6 alkyl;
** is point of connection;
y is 0, 1 or 2;
Y is O, S or $CR^1R^2$, wherein $R^1$ and $R^2$ are each independently H or C1-C6 alkyl;
s and t are each independently 0, 1 or 2, but not both 0;
or the drug is

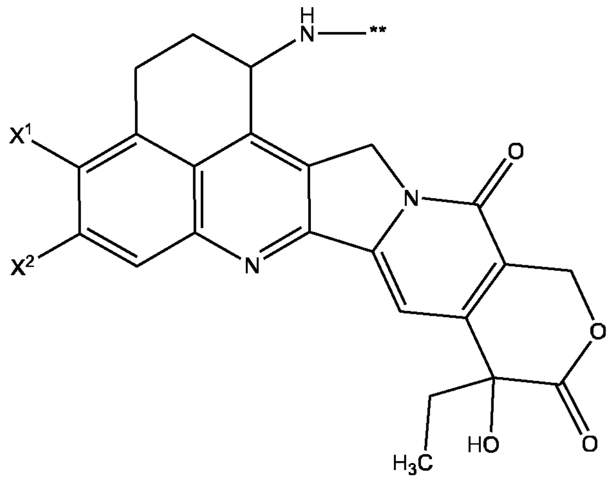

,

wherein $X^1$ and $X^2$ are each independently C1-C6 alkyl, halogen, or -OH; or C1-C6 alkyl is $-CH_3$; or halogen is F; ** is point of connection;

or the drug is

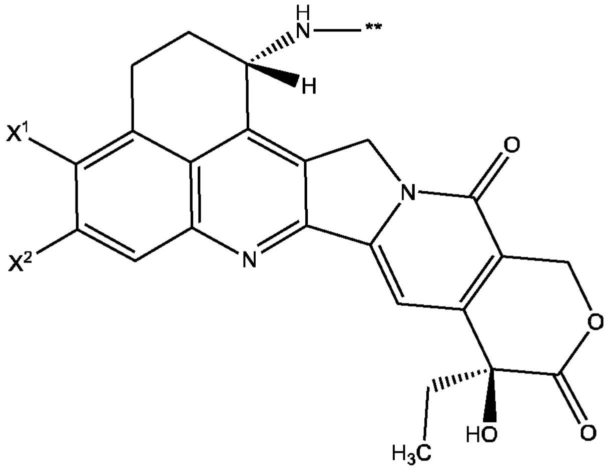

,

wherein $X^1$ and $X^2$ are each independently C1-C6 alkyl, halogen, or -OH; or C1-C6 alkyl is $-CH_3$; or halogen is F; ** is point of connection.

**14.** The method according to any one of claims 1-13, the characteristic is that, n is 4-12; or n is 4-8; or n is 4 or 8.

**15.** A method for treating a solid tumor, being **characterized in that** comprises administering to a patient with a solid tumor an effective amount of an antibody-drug conjugate, wherein, the effective amount of the antibody-drug conjugate is administered once every 1-8 weeks at 0.1-30 mg/kg per dose, the antibody-drug conjugate has a structure shown as Formula I-12, I-13, I-14, I-15, I-16, I-17, I-18, I-19, I-20, I-21, I-22, I-23, I-24, or I-25, or a stereoisomer thereof, or a pharmaceutically acceptable salt or solvate thereof, wherein the Formula I-12, I-13, I-14, I-15, I-16, I-17, I-18, I-19, I-20, I-21, I-22, I-23, I-24, or I-25 is:

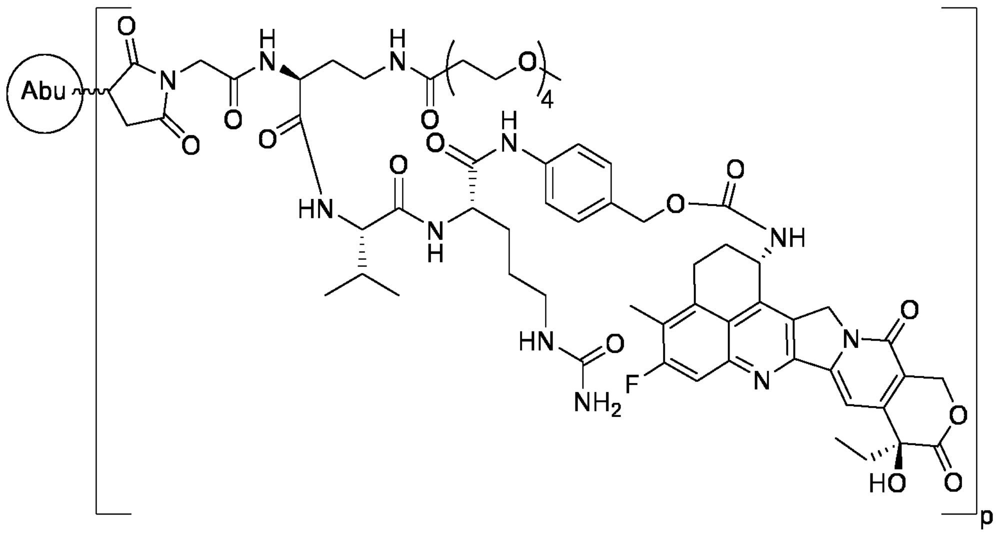

Formula I-12

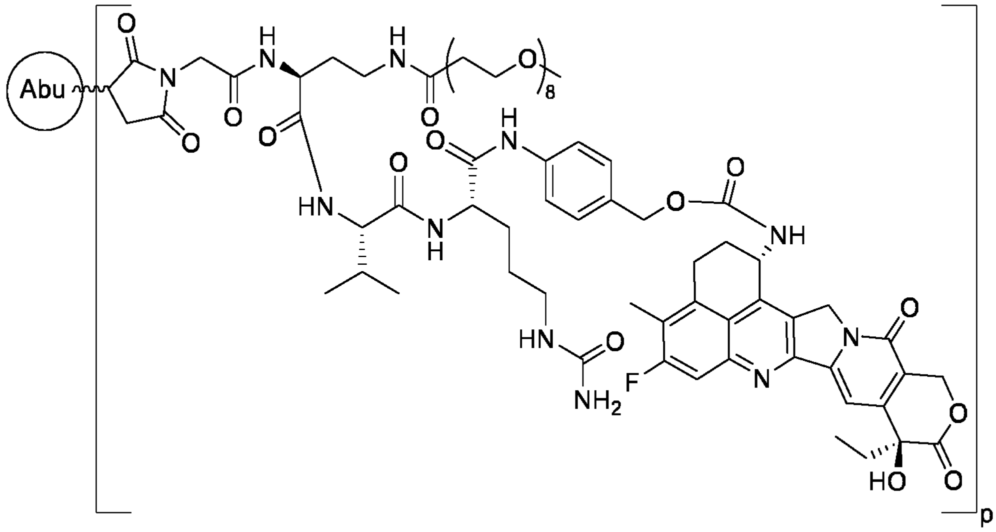

Formula I-13

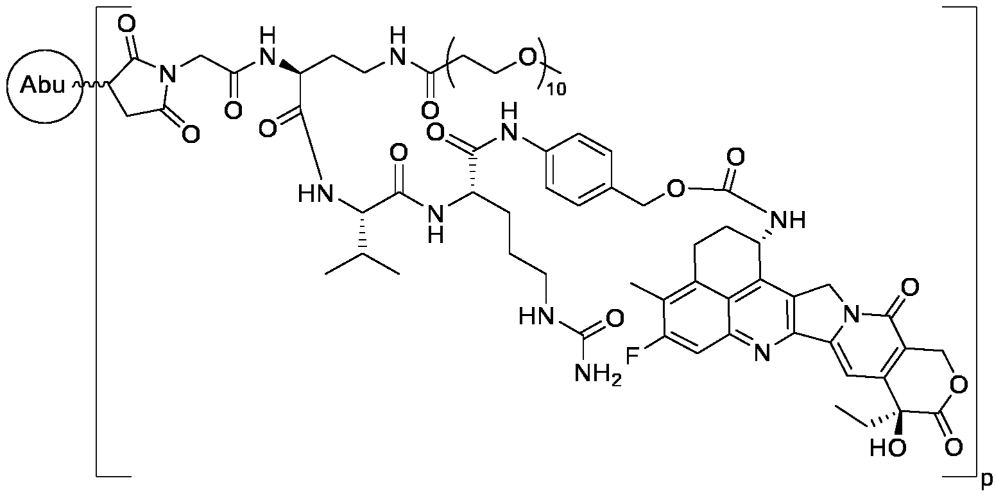

Formula I-14

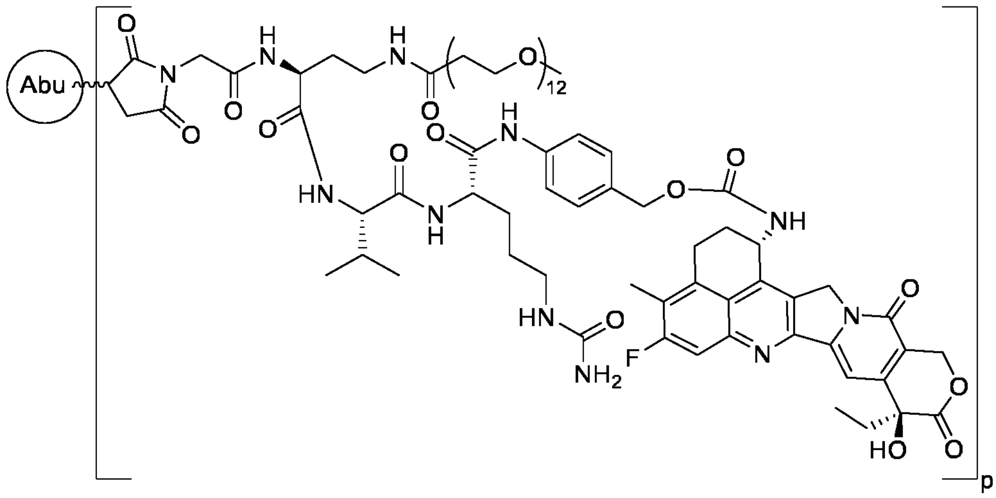

Formula I-15

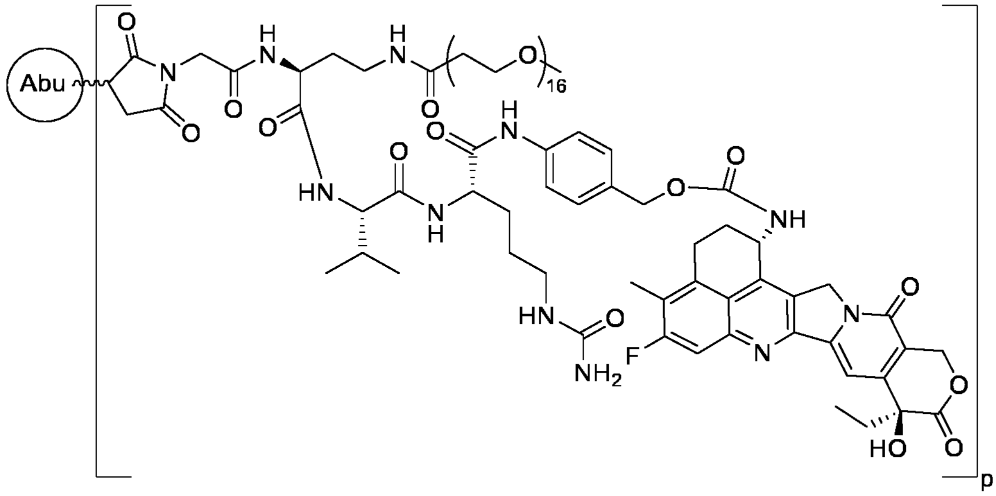

Formula I-16

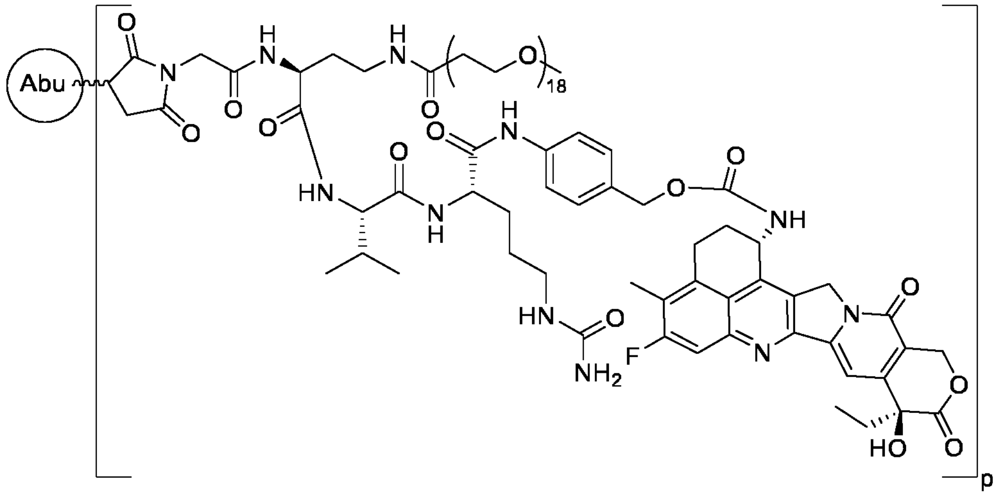

Formula I-17

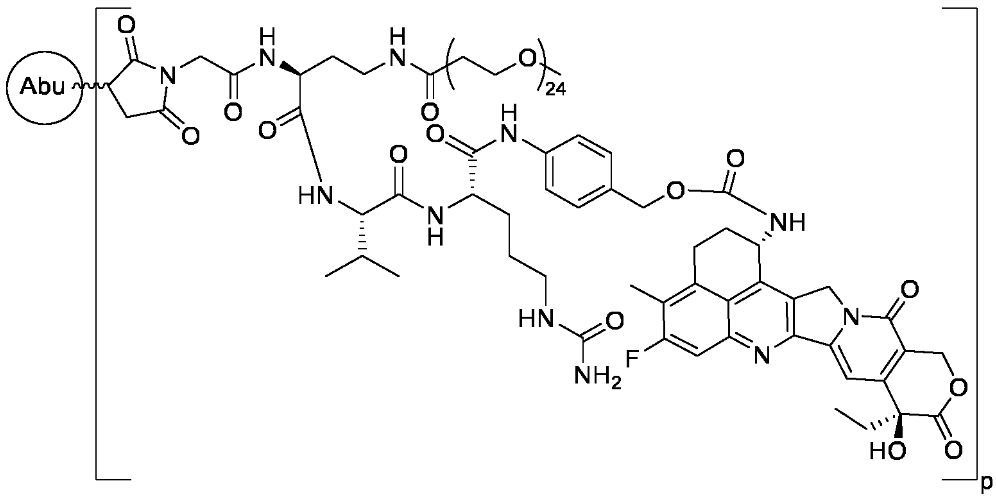

Formula I-18

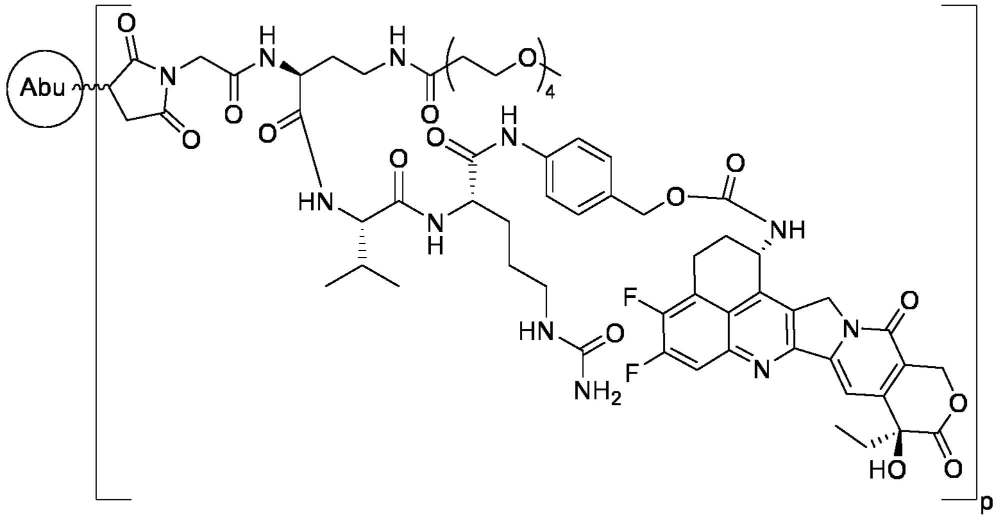

Formula I-19

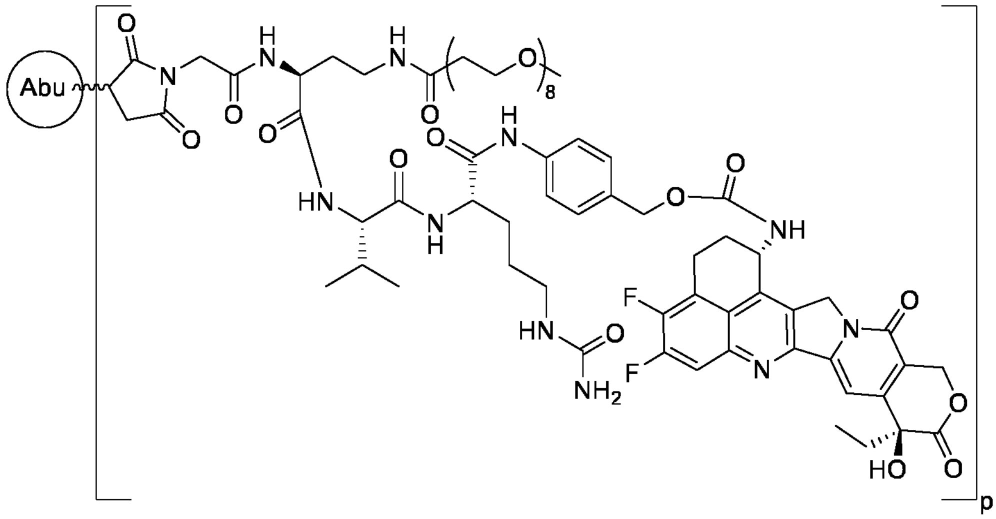

Formula I-20

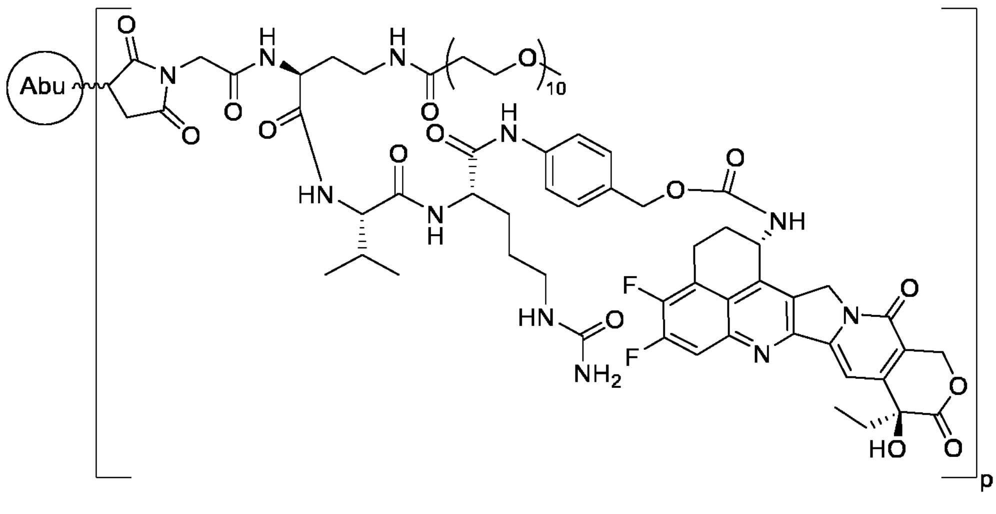

Formula I-21

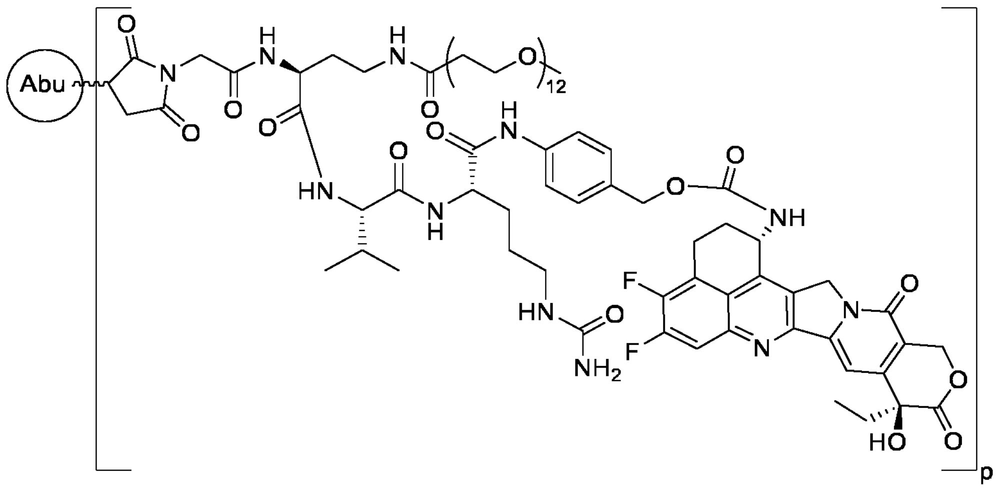

Formula I-22

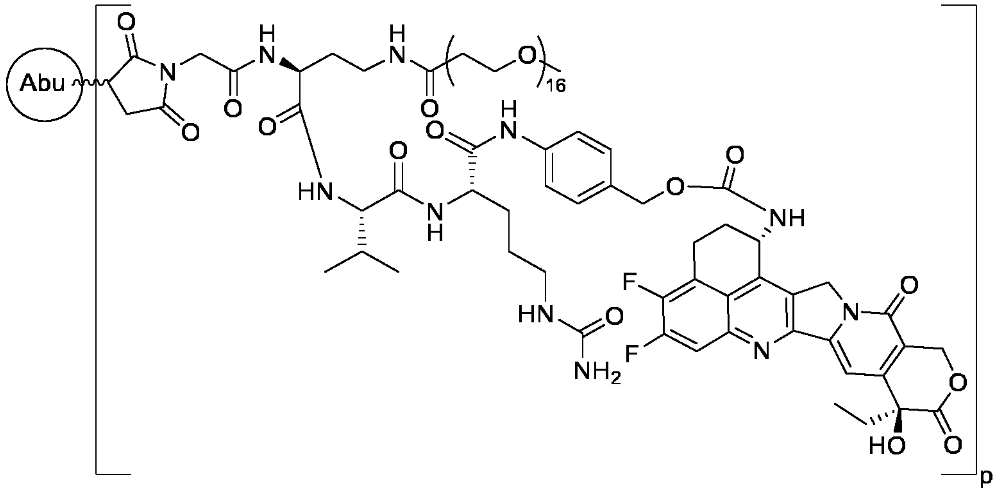

Formula I-23

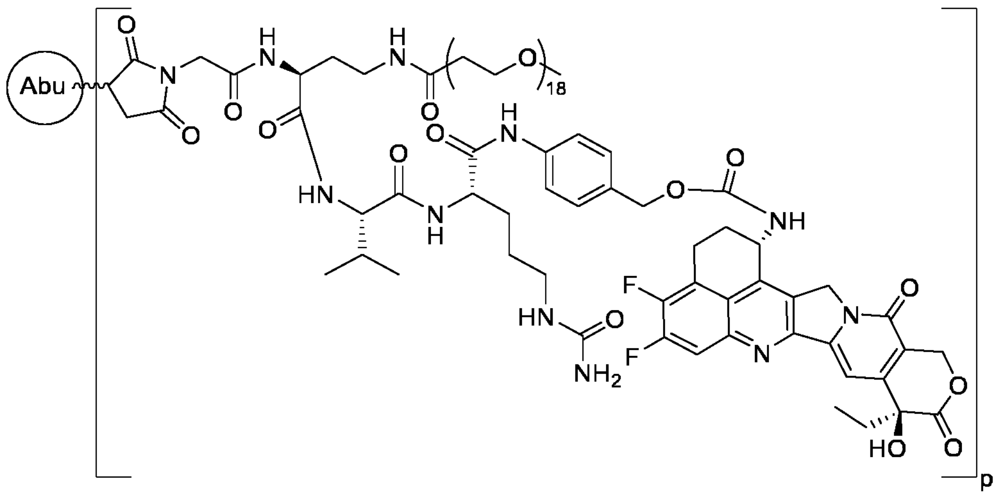

Formula I-24

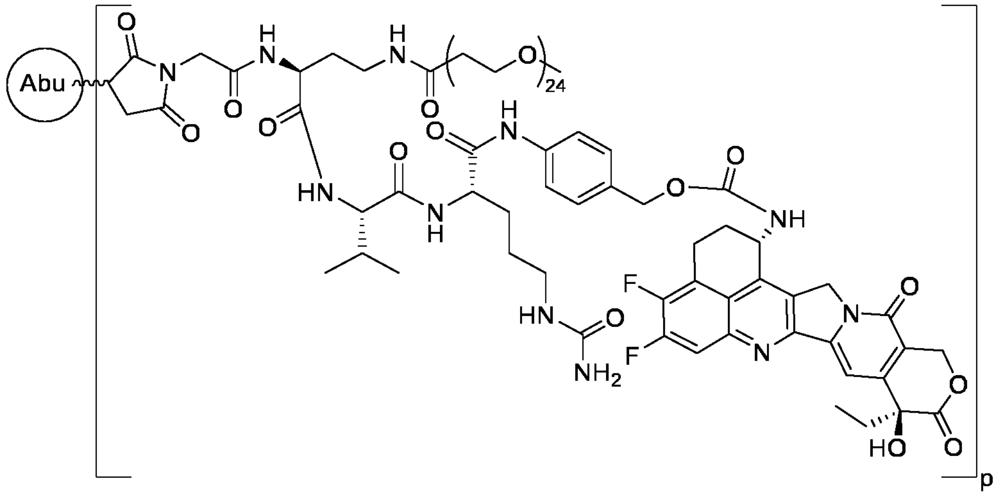

Formula I-25

wherein
Abu is an antibody or an antigen-binding unit thereof that binds to TROP2;
p is 1-10.

**16.** The method according to any one of claims 1-15, the characteristic is that, p is 2-8; or p is 4-8; or p is 6-8; or p is 7-8.

**17.** The method according to any one of claims 1-16, the characteristic is that, Abu is an hRS9 antibody.

**18.** The method according to any one of claims 1-16, the characteristic is that, Abu is an antibody or an antigen-binding unit thereof that binds to TROP2, comprising a heavy chain set forth in SEQ ID NO: 1 and a light chain set forth in SEQ ID NO: 2.

**19.** The method according to any one of claims 1-18, the characteristic is that, the effective amount of the antibody-drug conjugate is administered once every 1, 2, 3, 4, 5, 6, or 7 weeks at 0.1-30 mg/kg per dose.

**20.** The method according to any one of claims 1-19, the characteristic is that, the effective amount of the antibody-drug conjugate is administered once every 1, 2, 3, 4, 5, 6, or 7 weeks at about 0.8 mg/kg to about 3.6 mg/kg per dose.

**21.** The method according to any one of claims 1-19, the characteristic is that, the effective amount of the antibody-drug conjugate is administered once every 1, 2, 3, 4, 5, 6, or 7 weeks at about 2.4 mg/kg to about 3.6 mg/kg per dose.

**22.** The method according to any one of claims 1-19, the characteristic is that, the effective amount of the antibody-drug conjugate is administered once every 1, 2, 3, 4, 5, 6, or 7 weeks at about 2.7 mg/kg to about 3.3 mg/kg per dose.

**23.** The method according to any one of claims 1-19, the characteristic is that, the effective amount of the antibody-drug conjugate is administered once every 1, 2, 3, 4, 5, 6, or 7 weeks at about 0.8 mg/kg, about 1.2 mg/kg, about 2.4 mg/kg, about 2.7 mg/kg, about 3.0 mg/kg, about 3.3 mg/kg, or about 3.6 mg/kg per dose.

**24.** The method according to any one of claims 1-19, the characteristic is that, the effective amount of the antibody-drug conjugate is administered once every 1, 2, 3, 4, 5, 6, or 7 weeks at about 2.4 mg/kg, about 2.7 mg/kg, about 3.0 mg/kg, about 3.3 mg/kg, or about 3.6 mg/kg per dose.

**25.** The method according to any one of claims 1-19, the characteristic is that, the effective amount of the antibody-drug conjugate is administered once every 1, 2, 3, 4, 5, 6, or 7 weeks at about 3.0 mg/kg per dose.

**26.** The method according to any one of claims 1-19, the characteristic is that, the effective amount of the antibody-drug conjugate is administered once every 1, 2, 3, 4, 5, 6, or 7 weeks at 5-3000 mg per dose.

**27.** The method according to any one of claims 1-19, the characteristic is that, the effective amount of the antibody-drug

conjugate is administered once every 1, 2, 3, 4, 5, 6, or 7 weeks at 5-2000 mg per dose.

**28.** The method according to any one of claims 1-19, the characteristic is that, the effective amount of the antibody-drug conjugate is administered once every 1, 2, 3, 4, 5, 6, or 7 weeks at 5-1500 mg per dose.

**29.** The method according to any one of claims 1-19, the characteristic is that, the effective amount of the antibody-drug conjugate is administered once every 1, 2, 3, 4, 5, 6, or 7 weeks at 5-1000 mg per dose.

**30.** The method according to any one of claims 1-19, the characteristic is that, the effective amount of the antibody-drug conjugate is administered once every 1, 2, 3, 4, 5, 6, or 7 weeks at 5-800 mg per dose.

**31.** The method according to any one of claims 1-19, the characteristic is that, the effective amount of the antibody-drug conjugate is administered once every 1, 2, 3, 4, 5, 6, or 7 weeks at 5-600 mg per dose.

**32.** The method according to any one of claims 1-19, the characteristic is that, the effective amount of the antibody-drug conjugate is administered once every 2, 3, or 4 weeks at about 2.4 mg/kg to about 3.6 mg/kg per dose.

**33.** The method according to any one of claims 1-32, the characteristic is that, the effective amount of the antibody-drug conjugate is administered once every 2 weeks.

**34.** The method according to any one of claims 1-32, the characteristic is that, the effective amount of the antibody-drug conjugate is administered once every 3 weeks.

**35.** The method according to any one of claims 1-19, the characteristic is that, the effective amount of the antibody-drug conjugate is administered once every 2 weeks at about 2.4 mg/kg to about 3.6 mg/kg per dose.

**36.** The method according to any one of claims 1-19, the characteristic is that, the effective amount of the antibody-drug conjugate is administered once every 3 weeks at about 2.4 mg/kg to about 3.6 mg/kg per dose.

**37.** The method according to any one of claims 1-19, the characteristic is that, the effective amount of the antibody-drug conjugate is administered once every 2 weeks at about 2.7 mg/kg to about 3.3 mg/kg per dose.

**38.** The method according to any one of claims 1-19, the characteristic is that, the effective amount of the antibody-drug conjugate is administered once every 3 weeks at about 2.7 mg/kg to about 3.3 mg/kg per dose.

**39.** The method according to any one of claims 1-38, the characteristic is that, the antibody-drug conjugate is administered by injection; or administered by intravenous injection, subcutaneous injection or intraperitoneal injection; or administered by intravenous infusion.

**40.** The method according to claim 39, the characteristic is that, the intravenous infusion rate of the antibody-drug conjugate is ≤1000 mg/hr; or the rate of the first intravenous infusion of the antibody-drug conjugate is ≤500 mg/hr, and the rate of each subsequent intravenous infusion is ≤1000 mg/hr; or when the antibody-drug conjugate is administered by intravenous infusion, the rate is ≤200 mg/hr for 0 to 15 min and can be increased by 50-200 mg/hr every 15 to 30 min since 15 min; or when the antibody-drug conjugate is administered at the first intravenous infusion dose, the rate is ≤50 mg/hr for 0 to 15 min and can be increased by 50 mg/hr every 15 to 30 min since 15 min, and for each subsequent dose, the rate is 100-200 mg/hr for 0 to 15 min and can be increased by 100-200 mg/hr every 15 to 30 min since 15 min.

**41.** The method according to any one of claims 1-40, the characteristic is that, the solid tumor is a TROP2-positive solid tumor or an advanced solid tumor; or the solid tumor includes but is not limited to urothelial carcinoma, triple-negative breast cancer, non-small cell lung cancer, small cell lung cancer, pancreatic cancer, glioblastoma, medulloblastoma, breast cancer, head and neck cancer, kidney cancer, ovarian cancer, gastric cancer, Kaposi sarcoma, lung cancer, cervical cancer, carcinoma of colon and rectum, esophageal cancer, oral squamous cell cancer, prostate cancer, thyroid cancer, bladder cancer, neuroglioma, hepatobiliary cancer, carcinoma of colon and rectum, T-cell lymphoma, colorectal cancer, prostate cancer, melanoma, liver cancer.

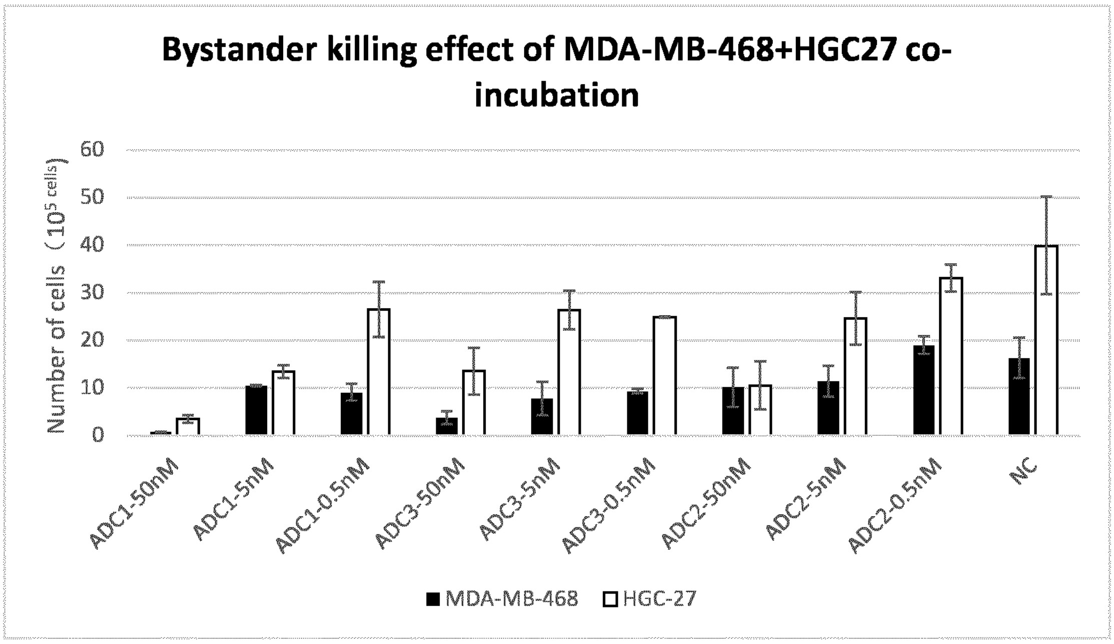
Bystander killing effect of MDA-MB-468+HGC27 co-incubation
Number of cells（10^5 cells）
60
50
40
30
20
10
0
ADC1-50nM
ADC1-5nM
ADC1-0.5nM
ADC3-50nM
ADC3-5nM
ADC3-0.5nM
ADC2-50nM
ADC2-5nM
ADC2-0.5nM
NC
MDA-MB-468
HGC-27

FIG. 1

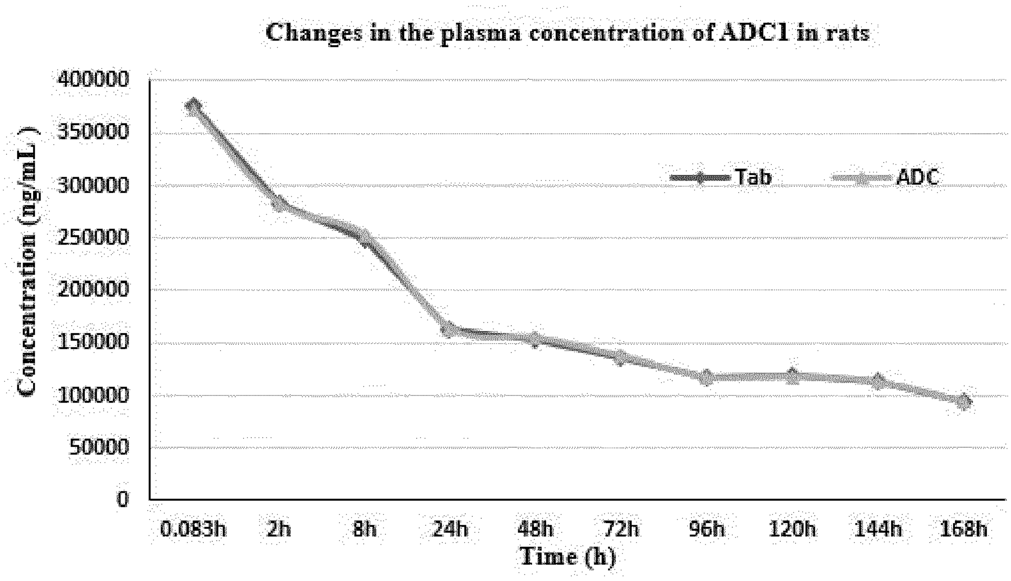
Changes in the plasma concentration of ADC1 in rats
Concentration (ng/mL)
400000
350000
300000
250000
200000
150000
100000
50000
0
Tab
ADC
0.083h
2h
8h
24h
48h
72h
96h
120h
144h
168h
Time (h)

FIG.2

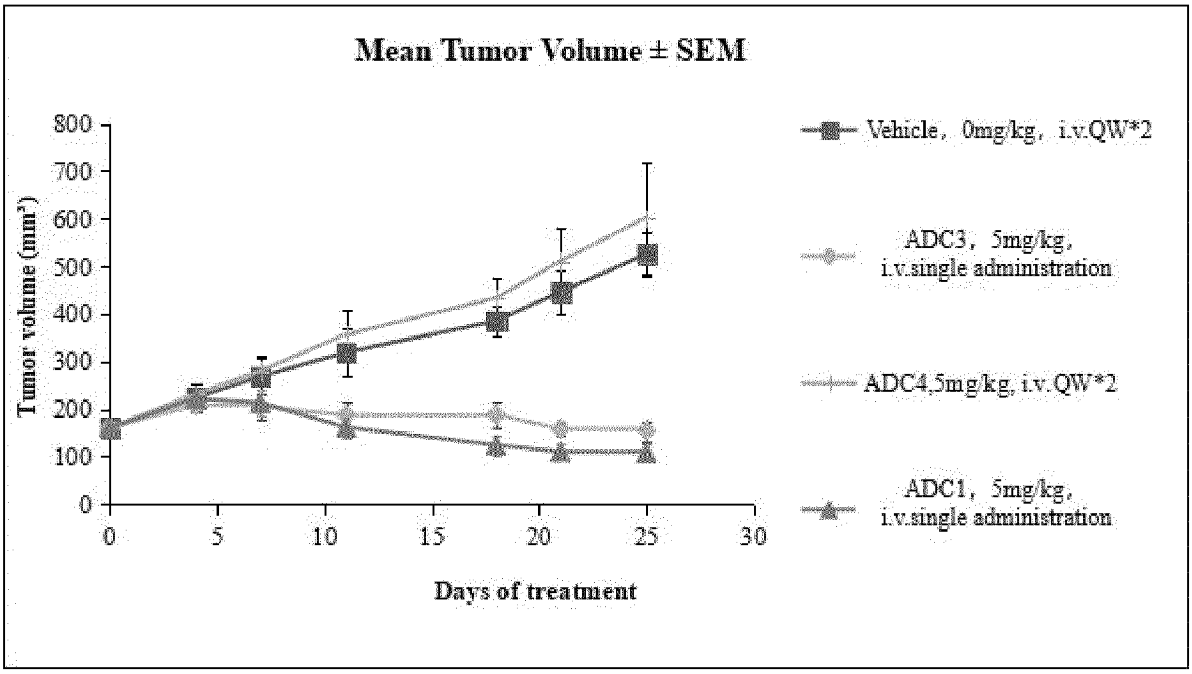
Mean Tumor Volume ± SEM
Tumor volume (mm³)
800
700
600
500
400
300
200
100
0
0
5
10
15
20
25
30
Days of treatment
Vehicle, 0mg/kg, i.v.QW*2
ADC3, 5mg/kg, i.v.single administration
ADC4,5mg/kg, i.v.QW*2
ADC1, 5mg/kg, i.v.single administration

FIG.3

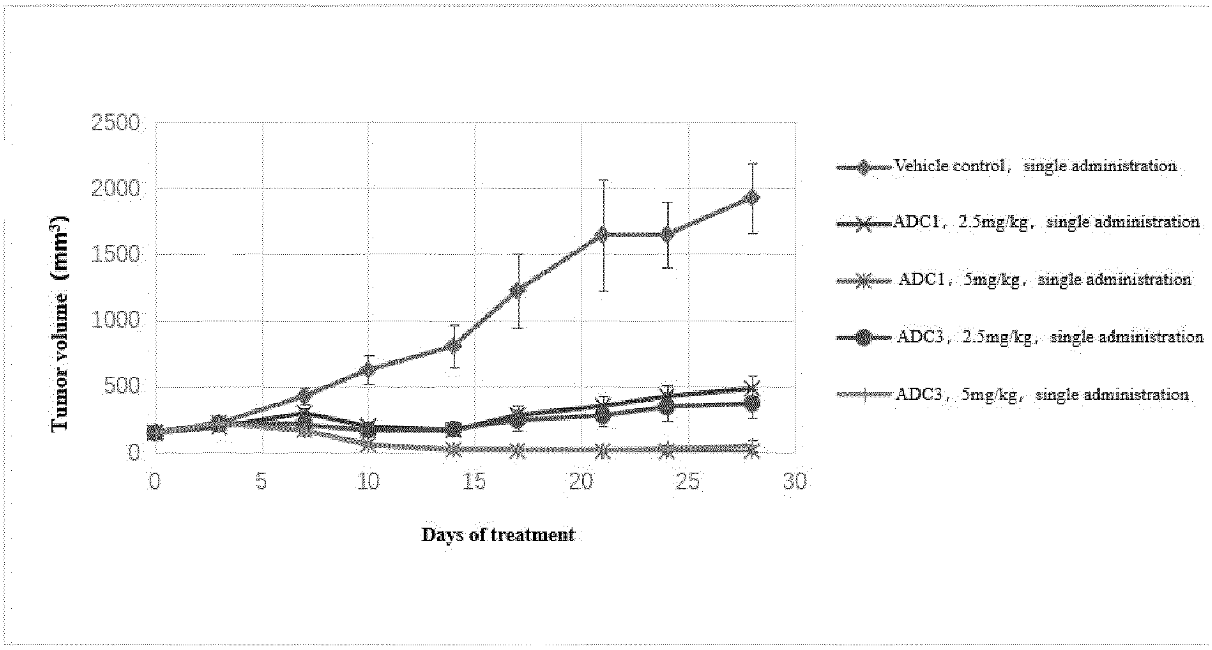
2500
2000
1500
1000
500
0
Tumor volume (mm³)
0
5
10
15
20
25
30
Days of treatment
Vehicle control, single administration
ADC1, 2.5mg/kg, single administration
ADC1, 5mg/kg, single administration
ADC3, 2.5mg/kg, single administration
ADC3, 5mg/kg, single administration

FIG.4

**INTERNATIONAL SEARCH REPORT**

International application No.
**PCT/CN2023/102590**

**A. CLASSIFICATION OF SUBJECT MATTER**

A61K47/68(2017.01)i; A61K47/65(2017.01)i; A61K31/4745(2006.01)i; C07K16/00(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, VEN, WPABS, WPABSC, STN, CNKI, WEB OF SCIENCE, PUBMED, 超星独秀, Chaoxing Duxiu, 百度学术, Baidu Scholar, 谷歌学术, Google Scholar, 万方数据库, Wanfang Database: 百奥泰, 梅星星, 李骁, 宋述强, 汤伟佳, 俞金泉, 李胜峰, 抗体, 抗原, 偶联, 缀合, 连接子, 癌, 肿瘤, ADC, antibody, linker, hRS9, TROP2, bio, conjugate, stn中权利要求1, 10, 15结构式检索, search for structural formula in claims 1, 10 and 15 in stn

**C. DOCUMENTS CONSIDERED TO BE RELEVANT**

| Category* | Citation of document, with indication, where appropriate, of the relevant passages | Relevant to claim No. |
|---|---|---|
| X | US 2021093733 A1 (OBI PHARMA, INC.) 01 April 2021 (2021-04-01)<br>claims 48-76, and description, paragraphs 185-189 and 307 | 1-41 |
| A | WO 2019243825 A1 (CURADEV PHARMA LTD.) 26 December 2019 (2019-12-26)<br>claims 1-37 | 1-41 |
| A | CN 113698414 A (SICHUAN KELUN-BIOTECH BIOPHARMACEUTICAL CO., LTD.) 26 November 2021 (2021-11-26)<br>claims 1-55 | 1-41 |
| A | WO 2020249063 A1 (BIO-THERA SOLUTIONS, LTD.) 17 December 2020 (2020-12-17)<br>claims 1-28 | 1-41 |
| A | WO 2021225892 A1 (LEVENA (SUZHOU) BIOPHARMA CO., LTD. et al.) 11 November 2021 (2021-11-11)<br>claims 1-44 | 1-41 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 September 2023** | **09 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/102590**

**C. DOCUMENTS CONSIDERED TO BE RELEVANT**

| Category* | Citation of document, with indication, where appropriate, of the relevant passages | Relevant to claim No. |
|---|---|---|
| A | CN 113816969 A (LEVENA (SUZHOU) BIOPHARMA CO., LTD.) 21 December 2021 (2021-12-21)<br>claims 1-13 | 1-41 |
| A | 武刚等 (WU, Gang et al.). "抗体偶联药物研发进展 (Progresses in Research and Development of Antibody-Drug Conjugate)"<br>*生物医学转化 (Biomedical Transformation),*<br>Vol. 2, No. (4), 30 December 2021 (2021-12-30),<br>ISSN: 2096-8965,<br>pp. 1-11 | 1-41 |

**INTERNATIONAL SEARCH REPORT**

International application No.
**PCT/CN2023/102590**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/102590**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **1-41**
because they relate to subject matter not required to be searched by this Authority, namely:

Claims 1-41 relate to a diagnosis or treatment method, which falls within subject matter for which a search is not required (PCT Rule 39(iv)). However, it is expected that the applicant may amend the subject matter of claims 1-41 to a corresponding pharmaceutical use; thus, the international search report is provided on this basis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/102590**

| Patent document cited in search report | | | Publication date (day/month/year) | Patent family member(s) | | | Publication date (day/month/year) |
|---|---|---|---|---|---|---|---|
| US | 2021093733 | A1 | 01 April 2021 | None | | | |
| WO | 2019243825 | A1 | 26 December 2019 | TW | 202016081 | A | 01 May 2020 |
| CN | 113698414 | A | 26 November 2021 | WO | 2019114666 | A1 | 20 June 2019 |
| | | | | EP | 3725798 | A1 | 21 October 2020 |
| | | | | EP | 3725798 | A4 | 10 November 2021 |
| | | | | US | 2020347075 | A1 | 05 November 2020 |
| | | | | US | 2021101906 | A2 | 08 April 2021 |
| | | | | CA | 3080236 | A1 | 20 June 2019 |
| | | | | KR | 20200099123 | A | 21 August 2020 |
| | | | | JP | 2021506743 | A | 22 February 2021 |
| WO | 2020249063 | A1 | 17 December 2020 | None | | | |
| WO | 2021225892 | A1 | 11 November 2021 | JP | 2023527962 | A | 03 July 2023 |
| | | | | IL | 297830 | A | 01 January 2023 |
| | | | | EP | 4146283 | A1 | 15 March 2023 |
| | | | | KR | 20230087414 | A | 16 June 2023 |
| | | | | CA | 3177562 | A1 | 11 November 2021 |
| | | | | TW | 202200212 | A | 01 January 2022 |
| | | | | AU | 2021267995 | A1 | 08 December 2022 |
| CN | 113816969 | A | 21 December 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021147993 A1
- CN 101264325 B
- CN 105849126 B
- CN 110903395 A
- CN 113896796 A
- US 20130089872 A
- US 5840854 A
- US 20130122020 A
- US 20120237518 A
- US 2018161440 A1


### Non-patent literature cited in the description


- **ALLEN, T.M.** ; **CULLIS, P.R.** *Science*, 2004, vol. 303 (5665), 1818-22
- **HU, Q.Y. et al.** *Chem Soc Rev*, 2016, vol. 45 (6), 1691-1719
- **THOMAS, A. et al.** *Lancet Oncol*, 2016, vol. 17 (6), e254-e262
- **CHARI, R.V.** *Acc Chem Res*, 2008, vol. 41 (1), 98-107
- **SINGH, S.K. et al.** *Pharm Res*, 2015, vol. 32 (11), 3541-3571
- **HAMILTON, G.S.** *Biologicals*, 2015, vol. 43 (5), 318-332
- **LIPINSKI et al.** *Proc Natl. Acad Sci USA*, 1981, vol. 78, 5147-50
- **IKEDA et al.** *Biochem Biophys Res Comm*, 2015, vol. 458, 877-82
- **OUYANG**. *J. Methods Mol Biol*, 2013, vol. 1045, 275-83
- McGraw-Hill Dictionary of Chemical Terms. McGraw-Hill, 1985
- **WOOD et al.** *J Immunol.*, 1990, vol. 145, 3011
- *CHEMICAL ABSTRACTS*, 143655-49-6